(19)

**Europäisches Patentamt**
**European Patent Office**
**Office européen des brevets**

(11) **EP 4 628 095 A1**

(12) **EUROPEAN PATENT APPLICATION**
published in accordance with Art. 153(4) EPC

(43) Date of publication:
08.10.2025 Bulletin 2025/41

(21) Application number: 23887963.9

(22) Date of filing: 06.11.2023

(51) International Patent Classification (IPC):
*A61K 39/12* (2006.01)     *A61K 39/29* (2006.01)
*A61P 31/00* (2006.01)     *C12N 15/86* (2006.01)

(52) Cooperative Patent Classification (CPC):
A61K 39/12; A61K 39/29; A61P 31/00; C12N 15/86

(86) International application number:
PCT/CN2023/129973

(87) International publication number:
WO 2024/099273 (16.05.2024 Gazette 2024/20)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 07.11.2022 CN 202211384764

(71) Applicants:
• Xiamen University
Xiamen, Fujian 361005 (CN)
• Xiamen Innovax Biotech Co., Ltd.
Xiamen, Fujian 361022 (CN)

(72) Inventors:
• LI, Shaowei
Xiamen, Fujian 361005 (CN)
• LI, Tingting
Xiamen, Fujian 361005 (CN)

• XUE, Wenhui
Xiamen, Fujian 361005 (CN)
• DENG, Tingting
Xiamen, Fujian 361005 (CN)
• FANG, Qianjiao
Xiamen, Fujian 361005 (CN)
• GU, Ying
Xiamen, Fujian 361005 (CN)
• ZHANG, Jun
Xiamen, Fujian 361005 (CN)
• XIA, Ningshao
Xiamen, Fujian 361005 (CN)

(74) Representative: Lavoix
Bayerstraße 83
80335 München (DE)

Remarks:
The complete document including Reference
Table(s) and the Sequence Listing(s) can be
downloaded from the EPO website

(54) **FUSION PROTEIN AND GRANULATED ANTIGEN COMPRISING SAME**

(57) Provided are a fusion protein and a composition, a kit, a granulated antigen, a vaccine, and a pharmaceutical composition comprising same. The present invention also relates to use of the fusion protein and the composition, the kit, and the granulated antigen compris- ing same in the preparation of a pharmaceutical compo- sition or a vaccine. The granulated antigen is particularly suitable for the production and vaccination of a vaccine and has advantages in the prevention and/or treatment of viral infections.

EP 4 628 095 A1

**Description**

**Technical Field**

The present application relates to the field of biomedicine, and specifically, the present application relates to a fusion protein and a composition, kit, particulate antigen, vaccine and pharmaceutical composition containing the same. The present application also relates to the use of the fusion protein and the composition, kit and particulate antigen containing the same in the manufacture of a pharmaceutical composition or vaccine.

**Background**

It is well known that vaccines are the most economical and effective method for preventing and controlling infectious diseases, and vaccination can save nearly 6 million lives each year. At present, genetic engineering vaccines mostly use proteins/polysaccharides critical to pathogen infection as antigens. Although the development of such vaccine antigens has the advantages of high safety and mature technology, and can produce immune protection against specific pathogens, they often face problems such as poor humoral immunity, difficulty in achieving long-term protection, and difficulty in activating efficient cellular immunity. Therefore, the development of safe, efficient and broad-spectrum new immunogens is the key to improving vaccine efficacy.

Particulate antigen-based vaccines are an important vaccine platform for resisting pathogen threats. Currently, there are three main particulate vaccines approved for marketing, including hepatitis E virus (HEV) vaccine, human papillomavirus vaccine (HPV) and hepatitis B virus (HBV) vaccine. HEV vaccine is a virus-like particle (VLP) vaccine. The HEV antigen fragment, p239 protein (aa368-606), expressed by the *Escherichia coli* expression system can be assembled into a particle morphology with a diameter of 25 nm after renaturation, has a structural conformation similar to that of natural virus particle, and can well mimic natural virus antigen epitopes. At present, it has been successfully developed into a vaccine antigen, and became the world's first HEV vaccine, Hecolin. It was launched in mainland China in 2012, and clinical results have confirmed that it has a good preventive protective effect. Currently available HPV vaccines include 2-valent vaccines, 4-valent vaccines and 9-valent vaccines. HPV vaccines are assembled from the main capsid protein L1 of the virus to form virus-like particles, can be produced and prepared by expression systems such as insect cells, yeast cells and *Escherichia coli,* have confirmed by clinical and post-marketing studies that HPV vaccines have good safety and protective effects. The research and development of HBV vaccines have a long history. Currently, there are many HBV vaccines available on the international market. HBV vaccines are mainly composed of S proteins of different forms of surface antigen HBsAg (SHBs, MHBs or LHBs) that spontaneously assemble to form particles, and they are currently mainly produced by yeast systems and CHO systems. Clinical and real-world studies have shown that HBV vaccines can produce high titers of protective antibodies and have good efficacy and safety. In summary, the production of particulate antigens in HEV vaccines, HPV vaccines and HBV vaccines has the advantages of economic efficiency, stable production process, controllable antigen quality, industrial mass production, good safety and strong immunogenicity, which is a good foundation and important reference for further development of other vaccines.

However, many viral proteins lack the ability to assemble into particles, making it impossible to develop particle-based vaccines. Moreover, isolated viral antigens or oligomeric antigens often exhibit insufficient immunogenicity, posing significant challenges for vaccine development.. How to display the target protein on the surface of the particle is a key issue and main goal in the design of recombinant vaccines and drug delivery carriers. Currently, the multivalent antigen display on particulate carriers is predominantly achieved through fusion expression techniques or by employing SpyTag/SpyCatcher-based chemical conjugation strategies. However, these methods have great limitations. First, a major limitation of the fusion expression method lies in the requirement that the particulate carrier must possess exposed N- or C-terminal. For particulate carriers lacking exposed terminal, it is necessary to explore other suitable insertion sites based on structural biology and bioinformatics analyses. Second, fusion expression must take into account the native conformation of the target molecule, as well as a range of factors such as steric hindrance, all of which require comprehensive consideration. The limitation of the technology based on SpyTag/SpyCatcher is that this connection method is limited by the structural characteristics of the particulate carriers. Therefore, it is an important goal in this field to find an efficient method to display target antigens on the surface of particulate carriers, and it faces various difficult problems.

The membrane proteins of enveloped viruses are the main immunogenic targets for vaccine development, but these proteins are usually anchored to the membrane structure on the viral surface, cannot form particulate antigens by themselves. Moreover, they are often highly hydrophobic, making it challenging to directly express the full-length proteins in a membraneassociated particulate form. The severe acute respiratory syndrome coronavirus 2 (SARS-CoV-2) is an RNA virus with an envelope, in which Spike protein is an important molecule on the envelope, and mainly involved in receptor recognition and cell membrane fusion. Among its domains, the receptor-binding domain (RBD) is directly responsible for binding to the host receptor angiotensin-converting enzyme 2 (ACE2), and it serves as the primary target of neutralizing antibodies. Therefore, the RBD is considered as a key immunogen. However, due to the small molecular weight of the RBD and insufficient immunogenicity, how to improve the immunogenicity of RBD is a key issue. Similar to RBD, gE protein is the most abundant glycoprotein on the surface of varicella-zoster virus (VZV) and is also an important target for vaccine development. Compared with the SARS-CoV-2 and varicella-zoster virus, human immunodeficiency virus I (HIV-1) that causes acquired immune deficiency syndrome (AIDS) has a higher genetic mutation rate, which makes the development of HIV vaccines face greater challenges. Env is the primary antigen on the surface of the HIV-1 virus and a key molecule in the development of HIV vaccines. The full-length Env molecule is a transmembrane protein known as gp160. By deleting the cytoplasmic tail, the solubility of Env expression can be significantly improved, resulting in a molecule referred to as gp140. This protein retains a native-like trimeric structure and exhibits clear advantages in the exposure of neutralizing epitopes and the elicitation of antibody responses. Nevertheless, the development of Env-based HIV vaccines has yet to achieve success, partly due to the fact that over 50% of the Env surface is covered by glycan molecules, which significantly diminishes its immunogenicity..

Therefore, enhancing the immunogenicity of antigens is of great significance for the development and application of vaccines.

## Contents of the present invention

This invention leverages the ability of assembly polypeptides-such as the ORF2 protein of HEV or its fragments or variants, the L1 protein of HPV or its fragments or variants, and the surface antigen of HBV or its fragments or variants -to form virus-like particles (VLPs). By fusing nanobodies that specifically bind to these assembly polypeptides with immunogenic polypeptides and utilizing the targeted binding characteristics of the nanobodies to the assembly polypeptides, multiple immunogenic polypeptides are displayed on the surface of VLPs formed by the assembly polypeptides, resulting in the particulate presentation of immunogenic polypeptides. Accordingly, this application provides a system and method for the particulate presentation of immunogenic polypeptides. Furthermore, it has been demonstrated that the particulate immunogenic polypeptides are capable of inducing robust humoral and cellular immune responses. Therefore, the particulate immunogenic polypeptides of the present invention hold potential as vaccine candidate molecules and offer advantages of universality and translatability in vaccine design and production.

Therefore, in the first aspect, the present application provides a fusion protein comprising an immunogenic polypeptide and a nanobody capable of specifically binding to a virus-like particle (VLP);

In certain embodiments, the VLP is a VLP assembled from assembly polypeptides.

In certain embodiments, the assembly polypeptide is a polypeptide capable of assembling into a VLP.

In certain embodiments, the assembly polypeptide is a capsid protein of a natural virus or a viroid. In such embodiments, the VLP assembled by the assembly polypeptide is similar in structure to the natural virus or viroid, except that it does not contain the genome of the natural virus or viroid.

In certain embodiments, the assembly polypeptide is an artificially prepared and/or screened polypeptide capable of assembling into a VLP. In such embodiments, the VLP assembled by the assembly polypeptide is similar or dissimilar in structure to the natural virus or viroid.

In certain embodiments, the assembly polypeptide is selected from the group consisting of protein of hepatitis E virus (HEV) or fragment thereof or variant thereof, protein of hepatitis B virus (HBV) or fragment thereof or variant thereof, protein of human papillomavirus (HPV) or fragment thereof or variant thereof, and any combination thereof; wherein the fragment or variant retains the ability to assemble into a VLP.

In some embodiments, the variant has a substitution, deletion or addition of one or more amino acids (e.g., a substitution, deletion or addition of 1, 2, 3, 4, 5 amino acids) as compared to the sequence of the polypeptide from which it is derived. In some embodiments, the substitution is a conservative substitution. In some embodiments, the polypeptide and variant thereof have the same or similar biological activity. In some preferred embodiments, the biological activity is the ability to assemble into a VLP.

In some embodiments, the assembly polypeptide is an ORF2 protein of HEV or fragment thereof or variant thereof.

In the present invention, the term "ORF2 protein" refers to a protein encoded by the second open reading frame in the genome of HEV virus, and the ORF2 protein or fragment thereof has the ability to self-assemble into a VLP.

In some embodiments, the fragment of ORF2 protein is selected from the group consisting of p239 protein and p495 protein.

In some embodiments, the assembly polypeptide is selected from the group consisting of a p239 protein or fragment thereof or variant thereof, and a p495 protein or fragment thereof or variant thereof.

In some embodiments, the p239 protein has a sequence corresponding to the amino acids at positions 368 to 606 of the amino acid sequence of ORF2 protein. In some embodiments, the p495 protein has a sequence corresponding to the amino acids at positions 112 to 606 of the amino acid sequence of ORF2 protein.

In some embodiments, the ORF2 protein has an amino acid sequence as set forth in SEQ ID NO: 9. In some embodiments, the p239 protein has an amino acid sequence as set forth in SEQ ID NO: 40. In some embodiments, the p495 protein has an amino acid sequence as set forth in SEQ ID NO: 41.

In some embodiments, the assembly polypeptide is a capsid protein L1 of HPV or fragment thereof or variant thereof.

HPV consists of a protein capsid and core DNA, with the capsid composed of the major capsid protein (L1) and a minor capsid protein (L2). In the present invention, the term "capsid protein L1" or "HPV L1 protein" or "L1 protein" refers to a L1 protein that constitutes HPV capsid.

In certain embodiments, the assembly polypeptide has an amino acid sequence as set forth in SEQ ID NO: 74.

In certain embodiments, the assembly polypeptide is a surface protein of HBV (e.g., a hepatitis B virus surface antigen) or fragment thereof or variant thereof. In certain embodiments, the assembly polypeptide is selected from a LHBs protein or fragment thereof or variant thereof, a MHBs protein or fragment thereof or variant thereof, a SHBs protein or fragment thereof or variant thereof of a hepatitis B virus surface antigen (HBsAg). In certain embodiments, the assembly polypeptide is selected from a SHBs protein or fragment thereof or variant thereof of hepatitis B virus surface antigen (HBsAg).

The Hepatitis B virus surface antigen (HBsAg) is composed of a large surface protein (LHBs), a middle surface protein (MHBs) and a small surface protein (SHBs). In the present invention, the term "SHBs protein" refers to a small surface protein constituting HBsAg.

In certain embodiments, the assembly polypeptide has an amino acid sequence as set forth in SEQ ID NO: 73.

In certain embodiments, the immunogenic polypeptide is a polypeptide or immunogenic variant thereof obtained from an organism or a non-organism (e.g., artificially synthesized).

In certain embodiments, the variant has a substitution, deletion or addition of one or more amino acids (e.g., a substitution, deletion or addition of 1, 2, 3, 4, 5 amino acids) as compared to the sequence of the polypeptide from which it is derived. In certain embodiments, the substitution is a conservative substitution. In certain embodiments, the polypeptide and variant thereof have the same or similar biological activity. In certain preferred embodiments, the biological activity is the ability to induce an immune response.

In certain embodiments, the organism is a pathogen (e.g., a virus, a bacterium, a fungus, a parasite) or a non-pathogen.

In certain embodiments, the immunogenic polypeptide is obtained from a non-tumor cell of a mammal (e.g., a human). In certain embodiments, the immunogenic polypeptide is a proprotein convertase subtilisin/kexin type 9 (PCSK9).

In certain embodiments, the immunogenic polypeptide is obtained from a tumor cell (e.g., expressed or overexpressed on the surface of a tumor cell) of a mammal (e.g., a human). In certain embodiments, the immunogenic polypeptide is selected from the group consisting of carcinoembryonic antigen (CEA), alpha-fetoprotein (AFP), and cancer antigen 125 (CA125).

In certain embodiments, the immunogenic polypeptide is obtained from a virus, a bacterium (e.g., *Mycobacterium tuberculosis*), a fungus (e.g., *Nostoc*), a parasite (e.g., *Plasmodium falciparum*).

In certain embodiments, the virus is selected from the group consisting of varicella zoster virus (VZV), severe acute respiratory syndrome coronavirus 2 (SARS-CoV-2), severe acute respiratory syndrome coronavirus (SARS-CoV), human immunodeficiency virus type I (HIV-1), human papillomavirus, hepatitis B virus, hepatitis A virus, hepatitis C virus, hepatitis E virus, measles virus, mumps virus, influenza virus, and encephalitis B virus.

In certain embodiments, the assembly polypeptide has a sequence derived from a viral pathogen, and the immunogenic polypeptide has a sequence derived from a non-viral pathogen (e.g., a bacterium, a fungus, a parasite).

In certain embodiments, the assembly polypeptide has a sequence derived from a viral pathogen, and the immunogenic polypeptide has a sequence derived from another viral pathogen.

The immunogenic polypeptide of the present invention may be a polypeptide derived from the surface or core of a virus. Generally, the immunogenic polypeptide may be a polypeptide of any structure or function comprising at least 6 amino acid residues. In some embodiments, the immunogenic polypeptide has a length of 6 to 10, 000 amino acid residues. In some embodiments, the immunogenic polypeptide has a length of 25 to 2, 000 amino acid residues. In some embodiments, the immunogenic polypeptide has a length of 50 to 500 amino acid residues.

In certain embodiments, the immunogenic polypeptide is selected from the group consisting of a SARS-CoV-2 RBD protein or immunogenic fragment thereof or variant thereof, an HIV-1 Env protein or immunogenic fragment thereof (e.g., gp140, gp160) or variant thereof, and a VZV gE protein or immunogenic fragment thereof or variant thereof.

In certain embodiments, the fragment of the Env protein is selected from the group consisting of gp160 protein, gp120 protein, and gp41 protein.

In certain embodiments, the Env protein has an amino acid sequence as set forth in SEQ ID NO: 34 or 35. In certain embodiments, the gp160 protein has a sequence as shown in GenBank with accession numbers AAB05604 and AAD12142. In certain embodiments, the gp41 has an amino acid sequence as shown in GenBank with accession number CAD20975.

In certain embodiments, the RBD protein has an amino acid sequence as set forth in any one of SEQ ID NOs: 1-8.

In certain embodiments, the gE protein has an amino acid sequence as set forth in SEQ ID NO: 30.

Those skilled in the art Experts in the field are able to prepare nanobodies capable of specifically binding to specific antigens by various methods known in the art, for instance, by immunizing alpacas or sharks with specific antigens, positive clones can be screened and the sequences of the antibody heavy chains obtained through sequencing. Subsequently, vectors containing the heavy chain sequences are constructed and transfected into host cells under defined conditions to express and produce nanobodies.

Therefore, when assembly polypeptides are determined, those skilled in the art are able to prepare and obtain nanobodies capable of specifically binding to the assembly polypeptides. Therefore, the nanobodies of the present application are not limited to the specific forms (e.g., nanobodies) and specific sequences used in the examples.

In certain embodiments, the nanobody is a camel-derived (e.g., alpaca-derived) antibody or a fish-derived (e.g., shark-derived) antibody.

In certain embodiments, the nanobody is a chimeric antibody, a humanized antibody, or a fully human antibody.

In certain embodiments, the fusion protein comprises 2, 3, or more kinds of immunogenic polypeptides.

In certain embodiments, each kind of immunogenic polypeptide is independently obtained from the same or different pathogens (e.g., viruses).

In certain embodiments, each kind of immunogenic polypeptide is a different polypeptide obtained from the same pathogen (e.g., virus).

In certain exemplary embodiments, the fusion protein comprises a first immunogenic polypeptide and a second immunogenic polypeptide, and the first immunogenic polypeptide is a RBD protein of SARS-CoV-2, and the second immunogenic polypeptide is a gE protein of VZV. In certain exemplary embodiments, the nanobody specifically binds to the RBD protein and the gE protein.

In certain exemplary embodiments, the fusion protein comprises a first immunogenic polypeptide, a second immunogenic polypeptide and a third immunogenic polypeptide, and the first immunogenic polypeptide is a RBD protein as set forth in SEQ ID NO: 1, the second immunogenic polypeptide is a RBD protein as set forth in SEQ ID NO: 2, and the third immunogenic polypeptide is a RBD protein as set forth in SEQ ID NO: 3. In certain embodiments, the nanobody specifically binds to the RBD proteins as set forth in SEQ ID NO: 1-3.

In certain embodiments, the fusion protein comprises one kind of immunogenic polypeptide. In certain embodiments, the nanobody is a nanobody that specifically binds to a polypeptide of HEV, HBV and/or HPV.

In certain embodiments, the nanobody comprises CDR-H1, CDR-H2 and CDR-H3 as contained in a heavy chain variable region (VHH) as set forth in any one of SEQ ID NOs: 10-29, 68, 69. In certain embodiments, the CDRs are defined according to the IMGT, Kabat or Chothia numbering system.

In certain embodiments, the nanobody comprises:

(a) a heavy chain variable region (VHH) comprising the following 3 complementary determining regions (CDRs): a VH CDR1 as set forth in SEQ ID NO: 90, a VH CDR2 as set forth in SEQ ID NO: 91, and a VH CDR3 as set forth in SEQ ID NO: 92;

(b) a heavy chain variable region (VHH) comprising the following 3 complementary determining regions (CDRs): a VH CDR1 as set forth in SEQ ID NO: 93, a VH CDR2 as set forth in SEQ ID NO: 94, and a VH CDR3 as set forth in SEQ ID NO: 95;

(c) a heavy chain variable region (VH) comprising the following 3 complementary determining regions (CDRs): a VH CDR1 as set forth in SEQ ID NO: 96, a VH CDR2 as set forth in SEQ ID NO: 97, and a VH CDR3 as set forth in SEQ ID NO: 98;

(d) a heavy chain variable region (VH) comprising the following 3 complementary determining regions (CDRs): a VH CDR1 as set forth in SEQ ID NO: 99, a VH CDR2 as set forth in SEQ ID NO: 100, and a VH CDR3 as set forth in SEQ ID NO: 101;

(e) a heavy chain variable region (VH) comprising the following 3 complementary determining regions (CDRs): a VH CDR1 as set forth in SEQ ID NO: 102, a VH CDR2 as set forth in SEQ ID NO: 103, and a VH CDR3 as set forth in SEQ ID NO: 104;

(f) a heavy chain variable region (VH) comprising the following 3 complementary determining regions (CDRs): a VH CDR1 as set forth in SEQ ID NO: 105, a VH CDR2 as set forth in SEQ ID NO: 106, and a VH CDR3 as set forth in SEQ ID NO: 107;

(g) a heavy chain variable region (VH) comprising the following 3 complementary determining regions (CDRs): a VH CDR1 as set forth in SEQ ID NO: 108, a VH CDR2 as set forth in SEQ ID NO: 109, and a VH CDR3 as set forth in SEQ ID NO: 110;

(h) a heavy chain variable region (VH) comprising the following 3 complementary determining regions (CDRs): a VH CDR1 as set forth in SEQ ID NO: 111, a VH CDR2 as set forth in SEQ ID NO: 112, and a VH CDR3 as set forth in SEQ ID NO: 113;

(i) a heavy chain variable region (VH) comprising the following 3 complementary determining regions (CDRs): a VH CDR1 as set forth in SEQ ID NO: 114, a VH CDR2 as set forth in SEQ ID NO: 115, and a VH CDR3 as set forth in SEQ ID NO: 116;

(j) a heavy chain variable region (VH) comprising the following 3 complementary determining regions (CDRs): a VH CDR1 as set forth in SEQ ID NO: 117, a VH CDR2 as set forth in SEQ ID NO: 118, and a VH CDR3 as set forth in SEQ ID NO: 119;

(k) a heavy chain variable region (VH) comprising the following 3 complementary determining regions (CDRs): a VH CDR1 as set forth in SEQ ID NO: 120, a VH CDR2 as set forth in SEQ ID NO: 121, and a VH CDR3 as set forth in SEQ ID NO: 122;

(l) a heavy chain variable region (VH) comprising the following 3 complementary determining regions (CDRs): a VH CDR1 as set forth in SEQ ID NO: 123, a VH CDR2 as set forth in SEQ ID NO: 124, and a VH CDR3 as set forth in SEQ ID NO: 125;

(m) a heavy chain variable region (VH) comprising the following 3 complementary determining regions (CDRs): a VH CDR1 as set forth in SEQ ID NO: 126, a VH CDR2 as set forth in SEQ ID NO: 127, and a VH CDR3 as set forth in SEQ ID NO: 128;

(n) a heavy chain variable region (VH) comprising the following 3 complementary determining regions (CDRs): a VH CDR1 as set forth in SEQ ID NO: 129, a VH CDR2 as set forth in SEQ ID NO: 130, and a VH CDR3 as set forth in SEQ ID NO: 131;

(o) a heavy chain variable region (VH) comprising the following 3 complementary determining regions (CDRs): a VH CDR1 as set forth in SEQ ID NO: 132, a VH CDR2 as set forth in SEQ ID NO: 133, and a VH CDR3 as set forth in SEQ ID NO: 134;

(p) a heavy chain variable region (VH) comprising the following 3 complementary determining regions (CDRs): a VH CDR1 as set forth in SEQ ID NO: 135, a VH CDR2 as set forth in SEQ ID NO: 136, and a VH CDR3 as set forth in SEQ ID NO: 137;

(q) a heavy chain variable region (VH) comprising the following 3 complementary determining regions (CDRs): a VH CDR1 as set forth in SEQ ID NO: 138, a VH CDR2 as set forth in SEQ ID NO: 139, and a VH CDR3 as set forth in SEQ ID NO: 140;

(r) a heavy chain variable region (VH) comprising the following 3 complementary determining regions (CDRs): a VH CDR1 as set forth in SEQ ID NO: 141, a VH CDR2 as set forth in SEQ ID NO: 142, and a VH CDR3 as set forth in SEQ ID NO: 143;

(s) a heavy chain variable region (VH) comprising the following 3 complementary determining regions (CDRs): a VH CDR1 as set forth in SEQ ID NO: 57, a VH CDR2 as set forth in SEQ ID NO: 58, and a VH CDR3 as set forth in SEQ ID NO: 59;

(t) a heavy chain variable region (VH) comprising the following 3 complementary determining regions (CDRs): a VH CDR1 as set forth in SEQ ID NO: 60, a VH CDR2 as set forth in SEQ ID NO: 61, and a VH CDR3 as set forth in SEQ ID NO: 62; or

(u) a heavy chain variable region (VH) comprising the following 3 complementary determining regions (CDRs): a VH CDR1 as set forth in SEQ ID NO: 65, a VH CDR2 as set forth in SEQ ID NO: 66, and a VH CDR3 as set forth in SEQ ID NO: 67.

In certain embodiments, the nanobody comprises a sequence as set forth in any one of SEQ ID NOs: 10-29, 68, 69 or a variant thereof; wherein the variant has a substitution, deletion or addition of one or several amino acids (e.g., a substitution, deletion or addition of 1, 2 or 3 amino acids) as compared to the sequence from which it is derived. In certain embodiments, the substitution is a conservative substitution.

In certain embodiments, the fusion protein further comprises a linker.

In certain embodiments, the linker is a polypeptide, such as a flexible peptide or a rigid peptide.

In certain embodiments, the linker comprises one or several (e.g., 1, 2 or 3) sequences represented by $(G_mS)_n$, wherein m is an integer selected from 1 to 6, and n is an integer selected from 1 to 6. In certain embodiments, m is 3, 4, or 5. In certain embodiments, n is 2, 3 or 4.

In certain embodiments, the linker has an amino acid sequence as set forth in SEQ ID NO: 39.

In certain embodiments, the immunogenic polypeptide and the nanobody of the fusion protein are directly connected or connected through the linker.

In some embodiments, the immunogenic polypeptide is located at the N-terminal or C-terminal of the fusion protein.

In some embodiments, the fusion protein comprises, from the N-terminal to the C-terminal, the immunogenic polypeptide and the nanobody; or, the nanobody and the immunogenic polypeptide; or, the immunogenic polypeptide, the linker and the nanobody; or, the nanobody, the linker and the immunogenic polypeptide.

In some embodiments, the fusion protein further comprises a signal peptide and/or a tag.

In some embodiments, the signal peptide is selected from the group consisting of tPA signal peptide and bee venom signal peptide. In some embodiments, the signal peptide has an amino acid sequence as set forth in SEQ ID NO: 31, 37 or 38.

In some embodiments, the tag is a tag for purification, for example, selected from a His tag or a GST tag.

In some embodiments, the signal peptide is located at the N-terminal of the fusion protein.

In some embodiments, the tag is located at the C-terminal of the fusion protein.

In certain embodiments, the fusion protein has an amino acid sequence as set forth in any one of SEQ ID NOs: 42-49, 50-56, 72-79, 80-87, 63, 64, 89.

In a second aspect, the present application provides an isolated nucleic acid molecule, comprising a nucleotide sequence encoding the fusion protein as described in the first aspect.

In certain embodiments, the nucleotide sequence is codon-optimized or not optimized according to the codon preference of a host cell.

As known to those skilled in the art, codons have degeneracy. That is, during the translation of a protein, each amino acid may correspond to one or more codons, for example, up to 6 codons. Different species (e.g., host cells) have great differences in the use of degenerate codons encoding a certain amino acid and have different preferences. This preference phenomenon is called "codon preference". Therefore, as used herein, the term "codon preference" refers to a situation where a species prefers to use certain specific codons to encode amino acids. Optimizing the sequence of a nucleic acid molecule according to codon preference is particularly advantageous in some cases, for example, it may help to increase the expression level of the protein encoded by the nucleic acid molecule.

In a third aspect, the present application provides a vector, comprising the isolated nucleic acid molecule as described in the second aspect. In certain embodiments, the vector is used to express (e.g., in vitro in a cell) a protein encoded by the isolated nucleic acid molecule.

In a fourth aspect, the present application provides a host cell, comprising the nucleic acid molecule as described in the second aspect or the vector as described in the third aspect.

In certain embodiments, the host cell is selected from the group consisting of prokaryotic cell and eukaryotic cell.

In certain embodiments, the prokaryotic cell is selected from the group consisting of *Escherichia coli* cell and *Bacillus subtilis* cell.

In certain embodiments, the eukaryotic cell is selected from the group consisting of yeast cell, insect cell, plant cell and animal cell.

In certain embodiments, the animal cell is a mammalian cell (e.g., a mouse cell, a human cell).

In a fifth aspect, the present application provides a method for expressing or producing the fusion protein as described in the first aspect, the method comprising culturing the host cell as described in the fourth aspect under a condition that allows protein expression, and optionally, recovering or purifying the expressed fusion protein.

In a sixth aspect, the present application provides a composition, comprising at least one of the fusion protein as described in the first aspect.

In certain embodiments, the composition further comprises an assembly polypeptide.

In certain embodiments, the assembly polypeptide is present in the form of VLP.

In some embodiments, the fusion protein is attached to the VLP.

In the seventh aspect, the present application provides a kit, comprising: the fusion protein as described in the first aspect or a first nucleic acid molecule containing a nucleotide sequence encoding the fusion protein, and an assembly polypeptide or a second nucleic acid molecule containing a nucleotide sequence encoding the assembly polypeptide.

In some embodiments, the fusion protein or the first nucleic acid molecule and the assembly polypeptide or the second nucleic acid molecule are provided separately or in the form of a composition.

In some embodiments, the kit further comprises a vector (e.g., an expression vector).

In some embodiments, the first nucleic acid molecule and the second nucleic acid molecule are contained in the same or different vectors.

In some embodiments, the kit further comprises a buffer.

In some embodiments, the buffer is selected from the group consisting of phosphate buffer, citric acid buffer, carbonate buffer, acetate buffer, barbituric acid buffer, Tris buffer, and any combination thereof.

In some embodiments, the buffer is a PBS buffer.

In some embodiments, the buffer further comprises a salt.

In some embodiments, the salt is selected from the group consisting of NaCl, $(NH_4)SO_4$, $NaSO_4$, $NH_4Cl$, and any combination thereof.

In the eighth aspect, the present application provides a particulate antigen, comprising an assembly polypeptide in the form of a VLP, and the fusion protein as described in the first aspect attached to the assembly polypeptide.

In certain embodiments, the fusion protein is attached to the VLP through the interaction between the nanobody and the assembly polypeptide.

In certain embodiments, the VLP is attached to at least one of the fusion protein as described in the first aspect.

In certain embodiments, the VLP is also attached to an additional polypeptide or fusion protein (e.g., a T cell epitope).

In the ninth aspect, the present application provides a method for preparing the particulate antigen as described in the eighth aspect, the method comprising: using the kit as described in the seventh aspect.

In certain embodiments, the method comprises: contacting the assembly polypeptide with the fusion protein under a condition that allows VLP assembly.

In certain embodiments, the condition that allows VLP assembly comprise placing the assembly polypeptide in a solution. In certain embodiments, the solution is a buffer comprising a salt. In certain embodiments, the salt is selected from the group consisting of NaCl, $(NH_4)SO_4$, $NaSO_4$, $NH_4Cl$, and any combination thereof.

In some embodiments, the method comprises: (i) adding the assembly polypeptide to the buffer and then contacting it with the fusion protein; or (ii) adding the assembly polypeptide and the fusion protein to the buffer together; or (iii) adding the fusion protein to the buffer and then adding the assembly polypeptide to the buffer;
optionally, recovering or purifying the particulate antigen in the buffer.

In the tenth aspect, the present application provides a vaccine, comprising the fusion protein as described in the first aspect, or the composition as described in the sixth aspect, or the particulate antigen as described in the eighth aspect, and an adjuvant.

In some embodiments, the adjuvant is selected from the group consisting of aluminum salt adjuvant, zinc/aluminum mixed adjuvant (e.g., FH002C), Freund's adjuvant, oil emulsion adjuvant, cytokine, TLR agonist, CpG adjuvant, liposome, AS01B adjuvant, and any combination thereof.

In the eleventh aspect, the present application provides a pharmaceutical composition, comprising any one or more of items (1) to (6):

(1) the fusion protein as described in the first aspect;

(2) the nucleic acid molecule as described in the second aspect;

(3) the vector as described in the third aspect;

(4) the host cell as described in the fourth aspect;

(5) the composition as described in the sixth aspect;

(6) the particulate antigen as described in the eighth aspect;

optionally, the pharmaceutical composition further comprises a pharmaceutically acceptable carrier and/or excipient.

In certain embodiments, the pharmaceutically acceptable carrier and/or excipient is selected from the group consisting of pH regulator (e.g., phosphate buffer), surfactant (e.g., cationic, anionic or non-ionic surfactant, such as Tween-80), adjuvant, ionic strength enhancer (e.g., sodium chloride), diluent, excipient, medium for containing or administering therapeutic agent, and any combination thereof.

In the twelfth aspect, the present application provides the use of the fusion protein as described in the first aspect, or the nucleic acid molecule as described in the second aspect, or the vector as described in the third aspect, or the host cell as described in the fourth aspect, or the composition as described in the sixth aspect, or the kit as described in the seventh aspect, or the particulate antigen as described in the eighth aspect, in the manufacture of a pharmaceutical composition or vaccine, wherein the pharmaceutical composition or vaccine is used to induce an immune response in a subject.

In certain embodiments, the immune response is a response to the immunogenic polypeptide and/or assembly polypeptide. In certain embodiments, the immune response is a T cell response (e.g., a CD4+ response or a CD8+ response). In certain embodiments, the immune response is a B cell response.

In certain embodiments, the subject is a mammal, such as a human, a monkey, or a mouse.

In the thirteenth aspect, the present application provides the use of the fusion protein described in the first aspect, or the nucleic acid molecule described in the second aspect, or the vector described in the third aspect, or the host cell described in the fourth aspect, or the composition described in the sixth aspect, or the kit described in the seventh aspect, or the particulate antigen described in the eighth aspect, in the manufacture of a pharmaceutical composition or vaccine, wherein the pharmaceutical composition or vaccine is used to for preventing and/or treating a disease and/or symptom in a subject that benefits or is prevented by an immune response to an immunogenic polypeptide.

In certain embodiments, the disease and/or symptom is caused by a tumor cell from which the immunogenic polypeptide is derived.

In certain embodiments, the disease and/or symptom is caused by a pathogen (e.g., a virus, a bacterium, a fungus, a parasite) from which the immunogenic polypeptide is derived.

In certain embodiments, the disease and/or symptom is caused by a virus from which the immunogenic polypeptide is derived, for example, chickenpox, COVID-19, AIDS, condyloma acuminatum, viral hepatitis (e.g., viral hepatitis B, viral hepatitis A, viral hepatitis C, viral hepatitis E), measles, mumps.

In certain embodiments, the immunogenic polypeptide may be a RBD protein of SARS-CoV-2 or fragment thereof. In such embodiments, the disease may be COVID-19.

In certain embodiments, the immunogenic polypeptide may be a gE protein of VZV or fragment thereof. In such embodiments, the disease may be a varicella.

In certain embodiments, the immunogenic polypeptide may be a protein of HPV or fragment thereof. In such embodiments, the disease may be a condyloma acuminatum.

In certain embodiments, the immunogenic polypeptide may be a protein or fragment thereof derived from hepatitis B virus, hepatitis A virus, hepatitis C virus, hepatitis E virus. In such embodiments, the disease may be a viral hepatitis (e.g., a viral hepatitis B, a viral hepatitis A, a viral hepatitis C, a viral hepatitis E).

In certain embodiments, the immunogenic polypeptide may be an envelope glycoprotein or fragment thereof derived from measles virus. In such embodiments, the disease may be a measles.

In certain embodiments, the immunogenic polypeptide may be a protein or fragment thereof derived from mumps virus. In such embodiments, the disease may be a mumps.

In certain embodiments, the subject is a mammal, such as a human, monkey or mouse.

In the fourteenth aspect, the present application provides a method for inducing an immune response in a subject, comprising administering to the subject an effective amount of the fusion protein as described in the first aspect, or the nucleic acid molecule as described in the second aspect, or the vector as described in the third aspect, or the host cell as described in the fourth aspect, or the composition as described in the sixth aspect, or the kit as described in the seventh aspect, or the particulate antigen as described in the eighth aspect, or the vaccine as described in the tenth aspect, or the pharmaceutical composition as described in the eleventh aspect.

In certain embodiments, the immune response is a response to the immunogenic polypeptide and/or assembly polypeptide. In certain embodiments, the immune response is a T cell response (e.g., a CD4+ response or a CD8+ response). In certain embodiments, the immune response is a B cell response.

In certain embodiments, the subject is a mammal, such as a human, a monkey or a mouse.

In the fifteenth aspect, the present application provides a method for preventing and/or treating a disease and/or symptom in a subject that benefits or is prevented by an immune response to the immunogenic polypeptide, comprising: administering to the subject an effective amount of the fusion protein as described in the first aspect, or the nucleic acid molecule as described in the second aspect, or the vector as described in the third aspect, or the host cell as described in the fourth aspect, or the composition as described in the sixth aspect, or the kit as described in the seventh aspect, or the particulate antigen as described in the eighth aspect, or the vaccine as described in the tenth aspect, or the pharmaceutical composition as described in the eleventh aspect.

In certain embodiments, the disease and/or symptom is caused by a tumor cell from which the immunogenic polypeptide is derived.

In certain embodiments, the disease and/or symptom is caused by a pathogen (e.g., virus, bacteria, fungus, parasite) from which the immunogenic polypeptide is derived.

In certain embodiments, the disease and/or symptom is caused by a virus from which the immunogenic polypeptide is derived, for example, chickenpox, COVID-19, AIDS, condyloma acuminatum, viral hepatitis (e.g., viral hepatitis B, viral hepatitis A, viral hepatitis C, viral hepatitis E), measles, mumps.

In certain embodiments, the immunogenic polypeptide may be an Env protein of HIV or fragment thereof (e.g., gp160 protein, gp120 protein, gp41 protein). In such embodiments, the disease may be AIDS.

In certain embodiments, the immunogenic polypeptide may be a RBD protein or fragment thereof derived from SARS-CoV-2. In such embodiments, the disease may be COVID-19.

In certain embodiments, the immunogenic polypeptide may be a gE protein of VZV or fragment thereof. In such embodiments, the disease may be a chickenpox.

In certain embodiments, the immunogenic polypeptide may be a protein of HPV or fragment thereof. In such embodiments, the disease may be a condyloma acuminatum.

In certain embodiments, the immunogenic polypeptide may be a protein or fragment thereof derived from hepatitis B virus, hepatitis A virus, hepatitis C virus, hepatitis E virus. In such embodiments, the disease may be a viral hepatitis (e.g., viral hepatitis B, viral hepatitis A, viral hepatitis C, viral hepatitis E).

In certain embodiments, the immunogenic polypeptide may be an envelope glycoprotein or fragment thereof derived from a measles virus. In such embodiments, the disease may be a measles.

In certain embodiments, the immunogenic polypeptide may be a protein or fragment thereof derived from a mumps virus. In such embodiments, the disease may be a mumps.

In certain embodiments, the subject is a mammal, such as a human, a monkey, or a mouse.

In the sixteenth aspect, the present application provides a system for preparing a particulate immunogenic polypeptide, which comprises a first vector and a second vector, wherein the first vector comprises a nucleotide sequence encoding a fusion protein, the fusion protein comprises an immunogenic polypeptide and an assembly polypeptide, and the second vector comprises a nucleotide sequence encoding a nanobody; and the nanobody can specifically bind to the assembly polypeptide, and the assembly polypeptide can be assembled into a VLP;

in certain embodiments, the nucleotide sequence is codon-optimized or not optimized according to the codon preference of a host cell.

In certain embodiments, the assembly polypeptide is selected from a protein of hepatitis E virus (HEV) or fragment thereof or variant thereof. In certain embodiments, the assembly polypeptide is as defined in the first aspect.

In certain embodiments, the fusion protein is as defined in the first aspect.

In the seventeenth aspect, the present application provides a method for enhancing the immunogenicity of an immunogenic polypeptide, comprising preparing or obtaining a fusion protein comprising the immunogenic polypeptide and a nanobody capable of specifically binding to an assembly polypeptide; and contacting the fusion protein with a VLP comprising the assembly polypeptide, thereby obtaining a particulate antigen comprising the immunogenic polypeptide attached to the VLP.

In some embodiments, the method comprises: using the system as described in the sixteenth aspect. In some embodiments, the method comprises: (1) expressing or producing the fusion protein through a first vector, and expressing or producing the assembly polypeptide through a second vector; (2) contacting the fusion protein and the assembly polypeptide under a condition that allows VLP assembly.

In some embodiments, the nanobody is as defined in the first aspect.

In some embodiments, the fusion protein is as defined in the first aspect.

In some embodiments, the assembly polypeptide is assembled into a VLP. In some embodiments, the fusion protein is attached to the VLP through the interaction between the nanobody and the assembly polypeptide.

Definition of terms

In the present invention, unless otherwise specified, the scientific and technical terms used herein have the meanings commonly understood by those skilled in the art. In addition, the virology, biochemistry, and immunology laboratory operation steps used herein are all routine steps widely used in the corresponding fields. At the same time, in order to better understand the present invention, the definitions and explanations of relevant terms are provided below.

As used herein, the term "immunogenic polypeptide" refers to a protein or polypeptide that can induce an immune response. In certain embodiments, the immunogenic polypeptide is administered (directly or indirectly) to a subject, which can induce an immune response in the subject. It is understood by those skilled in the art that the immunogenic polypeptide may be naturally occurring, or may be a protein or polypeptide with a mutation or variation (including but not limited to, substitution, deletion and/or addition) that is naturally produced or artificially introduced but does not affect its biological activity (as used herein, the biological activity is the ability to induce an immune response). Therefore, as used herein, the immunogenic polypeptide may be a polypeptide or variant thereof derived from a non-pathogen (e.g., a tumor cell) or a pathogen (e.g., a virus, bacteria, fungus, parasite or other pathogen).

As used herein, the term "immune response" refers to a response of cells of the immune system (e.g., B cells, T cells or monocytes) to stimulation. In some embodiments, the immune response is a response specific to a particular antigen (i.e., an antigen-specific response). In some embodiments, the immune response is a T cell response, such as a CD4+ response or a CD8+ response. In other embodiments, the immune response is a B cell response, which leads to the production of a specific antibody.

As used herein, the term "subvirus" refers to a microorganism that does not have a complete virus structure, mainly including viroid, mimic and prion.

As used herein, the term "RBD protein of SARS-Cov-2" or "RBD of SARS-Cov-2" has the same meaning and can be used interchangeably. It refers to the receptor binding domain (RBD) on the spike protein (S protein) of SARS-Cov-2. Its main function is to recognize a host cell surface receptor, and mediate the fusion with host cells. It is understood by those skilled in the art that different SARS-CoV-2 virus strains may contain RBD proteins of different sequences, but these RBD proteins have the same or similar biological properties. Therefore, in the present invention, RBD proteins include not only the proteins as set forth in SEQ ID NOs: 1-8, but also the RBD proteins of various SARS-CoV-2 virus strains. The amino acid sequences of these RBD proteins can be obtained from public databases (e.g., GenBank databases), for example, the amino acid sequences shown in GenBank with accession numbers OP077006.1, OP077005.1, and OP077003.1.

As used herein, the terms "gE protein of VZV", "gE of VZV", and "glycoprotein gE of VZV" refer to a kind of envelope glycoprotein of VZV, which have the same meaning and can be used interchangeably. It is understood by those skilled in the art that different VZV virus strains may contain gE proteins of different sequences, but these gE proteins have the same or similar biological properties. Therefore, in the present invention, the gE protein includes not only the protein as set forth in SEQ ID NO: 30, but also the gE proteins of various VZV strains. The amino acid sequences of these gE proteins can be obtained from public databases (e.g., GenBank database).

As used herein, the term "Env of HIV-1" refers to the envelope protein on the surface of HIV-1 virus, also referred to as "BGTSTIP" herein. It is understood by those skilled in the art that different HIV-1 strains may contain Env proteins with different sequences, but these Env proteins have the same or similar biological properties. Therefore, in the present invention, the Env protein includes not only the proteins as set forth in SEQ ID NOs: 34-35, but also the Env proteins of various HIV-1 strains. The amino acid sequences of these Env proteins can be obtained from public databases (e.g., GenBank database).

The capsid protein (also called ORF2 protein) encoded by the ORF2 gene in HEV and its fragments (e.g., p239 protein, p495 protein) have been confirmed to have the ability to assemble into VLPs. The sequence of ORF2 protein is well known in the art, see, for example, DDBJ database accession number: D11092. The p239 protein has a sequence corresponding to the amino acids at positions 368 to 606 of ORF2 protein. In certain embodiments, the p495 protein has a sequence corresponding to the amino acids at positions 112 to 606 of ORF2 protein.

As used herein, the term "assembly polypeptide" refers to a protein or polypeptide that can be assembled into a virus-like particle (VLP). In certain embodiments, the assembly polypeptide is a shell protein of a natural virus or viroid. In such embodiments, the VLP assembled by the assembly polypeptide is similar in structure to the natural virus or viroid, except that it does not contain the genome of the natural virus or viroid. In certain embodiments, the assembly polypeptide is an artificially prepared and/or screened polypeptide. In such embodiments, the VLP assembled by the assembly polypeptide is similar or dissimilar in structure to the natural virus or viroid. The term "virus-like particle (VLP)" is a multimeric particle which structure is similar or dissimilar to that of a natural virus or subvirus. It has been confirmed that proteins (e.g., capsid protein, surface protein, envelope protein) of some viruses (e.g., HBV, HEV, HPV) can spontaneously form VLPs after recombinant expression in an appropriate expression system.

Those skilled in the art can identify proteins or polypeptides that have the ability to assemble into VLPs by publicly disclosed methods, for example, placing the protein to be tested in a buffer solution (e.g., PBS solution) at room temperature and then detecting the presence of VLP. The presence of VLP can be detected using conventional techniques known in the art, such as electron microscopy, biophysical characterization, etc. Specific detection methods can be found in, for example, Baker et al. (1991) Biophys. J. 60: 1445-1456; and Hagensee et al. (1994) J. Virol. 68: 4503-4505. For example, the capsid protein (also called ORF2 protein) encoded by the ORF2 gene in HEV and its fragments (e.g., p239 protein, p495 protein) have been confirmed to have the ability to assemble into a VLP.

Those skilled in the art will understand that, in the present application, the assembly polypeptide includes not only natural proteins or polypeptides that can be assembled into VLPs, but also proteins or polypeptides with mutations or variations (including but not limited to, substitutions, deletions and/or additions) that are naturally generated or artificially introduced based on the natural proteins but do not affect their biological functions (as used herein, the biological function is the ability to assemble into VLP). Therefore, in some embodiments, the assembly polypeptide is selected from an ORF2 protein of hepatitis E virus (HEV) or fragment thereof or variant thereof. In some embodiments, the assembly polypeptide is selected from a p239 protein or fragment thereof or variant thereof, a p495 protein or fragment thereof or variant thereof.

According to the present invention, when used in the context of a protein/polypeptide, the term "variant" refers to a protein/polypeptide that has one or more (e.g., 1-10 or 1-5 or 1-3) amino acid differences (e.g., substitutions, deletions or additions) as compared to the sequence of the protein/polypeptide from which it is derived, and it retains the biological activity of the protein/polypeptide from which it is derived.

As used herein, "the immunogenic polypeptide is a polypeptide or an immunogenic variant thereof derived from an organism or non-organism" means that the sequence of the polypeptide is derived from a sequence in the organism or non-organism, but the method of obtaining the polypeptide is not limited to a specific production method. In certain embodiments, the polypeptide may be naturally isolated, artificially synthesized, or obtained by genetic engineering recombination.

As used herein, the term "particulate immunogenic polypeptide" refers to a collection of immunogenic polypeptides in the form of particle. In certain embodiments, the particulate immunogenic polypeptide refers to an immunogenic polypeptide attached to a virus-like particle.

As used herein, the term "organism" refers to a living individual or object. Except for a few species such as viruses, organisms are composed of cells. In certain embodiments, organisms include pathogens and non-pathogens.

As used herein, the term "pathogens" refers to microorganisms (e.g., bacteria, viruses, rickettsia, fungi), parasites or other vectors (e.g., recombinant microorganisms) that can cause infection in humans, animals and plants.

As used herein, the term "fusion protein" refers to a recombinant protein formed by connecting the amino acid sequences of at least two independent proteins or polypeptides. The amino acid sequences of the two independent proteins or polypeptides may be directly connected or connected via a linker.

As used herein, the term "conservative substitution" refers to an amino acid substitution that does not adversely affect or change the expected properties of the protein/polypeptide containing the amino acid sequence. For example, conservative substitutions can be introduced by standard techniques known in the art, such as site-directed mutagenesis and PCR-mediated mutagenesis. Conservative amino acid substitutions include substitutions of amino acid residues with amino acid residues having similar side chains, such as substitutions with residues that are physically or functionally similar to the corresponding amino acid residues (e.g., having similar size, shape, charge, chemical properties, including the ability to form covalent bonds or hydrogen bonds, etc.). Families of amino acid residues with similar side chains have been defined in the art. These families include amino acids with basic side chains (e.g., lysine, arginine, and histidine), acidic side chains (e.g., aspartic acid, glutamic acid), uncharged polar side chains (e.g., glycine, asparagine, glutamine, serine, threonine, tyrosine, cysteine, tryptophan), nonpolar side chains (e.g., alanine, valine, leucine, isoleucine, proline, phenylalanine, methionine), $\beta$-branched side chains (e.g., threonine, valine, isoleucine), and aromatic side chains (e.g., tyrosine, phenylalanine, tryptophan, histidine). Thus, it is preferred to replace a corresponding amino acid residue with another amino acid residue from the same side chain family. Methods for identifying conservative amino acid substitutions are well known in the art (see, for example, Brummell et al., Biochem. 32:1180-1187 (1993); Kobayashi et al. Protein Eng. 12(10):879-884 (1999); and Burks et al. Proc. Natl Acad. Set USA 94:412-417 (1997), which are incorporated herein by reference).

As used herein, the term "vector" refers to a nucleic acid delivery vehicle into which a polynucleotide can be inserted. When a vector is capable of expressing a protein encoded by the inserted polynucleotide, the vector is referred to as an expression vector. The vector can be introduced into a host cell by transformation, transduction or transfection so that the genetic material elements it carries are expressed in the host cell. Vectors are well known to those skilled in the art, including but not limited to: plasmid (e.g. naked plasmid); phagemid; cosmid; artificial chromosome, such as yeast artificial chromosome (YAC), bacterial artificial chromosome (BAC) or P1-derived artificial chromosome (PAC); bacteriophage such as $\lambda$ phage or M13 phage, and viral vector, etc.

As used herein, the term "host cell" refers to a cell that can be used to amplify or express an exogenous gene, including but not limited to prokaryotic cell such as *Escherichia coli* or *Bacillus subtilis,* fungal cell such as yeast cell or *Aspergillus,* insect cell such as *S2 Drosophila* cell or Sf9, or animal cell such as fibroblast, CHO cell, COS cell, NSO cell, HeLa cell, BHK cell, HEK 293 cell, 293T cell or human cell, etc.

As used herein, the term "antibody" refers to an immunoglobulin molecule capable of specifically binding to a target (e.g., carbohydrate, polynucleotide, lipid, polypeptide, etc.) through at least one antigen recognition site located in the variable region of the immunoglobulin molecule. The antibody includes antibody of any type, such as IgG, IgA, or IgM (or subtypes thereof), and the antibody do not need to belong to any particular type. Immunoglobulins can be assigned to different classes depending on the antibody heavy chain constant region amino acid sequences. There are five major types of immunoglobulins: IgA, IgD, IgE, IgG, and IgM, and several of which can be further divided into subtypes (isotypes), such as IgG1, IgG2, IgG3, IgG4, IgA1, and IgA2. The heavy chain constant regions corresponding to the different types of immunoglobulins are called $\alpha$, $\delta$, $\varepsilon$, $\gamma$, and $\mu$, respectively. Antibody light chains can be classified as $\kappa$ (kappa) and $\lambda$ (lambda) light chains. The subunit structures and three-dimensional configurations of different types of immunoglobulins are well known. The heavy chain constant region consists of 4 domains (CH1, hinge region, CH2, and CH3). Each light chain consists of a light chain variable region (VL) and a light chain constant region (CL). The light chain constant region consists of one domain, CL. The constant domain is not directly involved in the binding of antibody to antigen, but exhibits a variety of effector functions, such as mediating the binding of immunoglobulin to host tissues or factors, including various cells of the immune system (e.g., effector cells) and the first component (C1q) of the classical complement system.

The VH and VL regions of antibodies can also be subdivided into highly variable regions (called complementary determining regions (CDRs)), interspersed with more conservative regions called framework regions (FRs). Each VH and VL consists of three CDRs and four FRs arranged from the amino end to the carboxyl end in the following order: FR1, CDR1, FR2, CDR2, FR3, CDR3, FR4. The variable regions (VH and VL) of each heavy chain/light chain pair form the antigen binding site, respectively. The allocation of amino acids to various regions or domains can follow the definitions of Kabat (Sequences of Proteins of Immunological Interest (National Institutes of Health, Bethesda, Md. (1987 and 1991)), or Chothia & Lesk (1987) J. Mol. Biol. 196:901-917; Chothia et al. (1989) Nature 342:878-883), Chothia (the immunoglobulin numbering system proposed by Chothia et al., which is a classical rule for identifying CDR region boundaries based on the position of structural loop regions (see, e.g., Chothia & Lesk (1987) J. Mol. Biol. 196:901-917; Chothia et al. (1989) Nature 342:878-883)) and/or AbM (the AbM CDR definition method is derived from the relevant studies of Martin (Martin ACR, Cheetham JC, Rees AR (1989) Modelling antibody hypervariable loops: A combined algorithm. Proc Natl Acad Sci USA 86:9268-9272)).

As used herein, the term "complementarity determining region" or "CDR" refers to the amino acid residues in the variable region of an antibody that are responsible for antigen binding. There are three CDRs in each of the heavy and light chain variable regions, designated CDR1, CDR2, and CDR3. The precise boundaries of these CDRs can be defined according to various numbering systems known in the art, such as the Kabat numbering system (Kabat et al., Sequences of Proteins of Immunological Interest, 5th Ed. Public Health Service, National Institutes of Health, Bethesda, Md., 1991), the Chothia numbering system (Chothia & Lesk (1987) J. Mol. Biol. 196:901-917; Chothia et al. (1989) Nature 342:878-883), or the IMGT numbering system (Lefranc et al., Dev. Comparat. Immunol. 27:55-77, 2003). For a given antibody, one skilled in the art will readily identify the CDRs defined by each numbering system. Moreover, the correspondence between different numbering systems is well known to those skilled in the art (for example, see Lefranc et al., Dev. Comparat. Immunol. 27:55-77, 2003). In the present invention, the CDRs contained in the nanobody can be determined according to various numbering systems known in the art.

As used herein, the term "framework region" or "FR" residues refers to those amino acid residues in the antibody variable region other than the CDR residues defined above.

As used herein, the term "single-domain antibody (sdAb)" is also called nanobody, and the two can be used interchangeably. It has the meaning commonly understood by those skilled in the art, which refers to an antibody fragment composed of a single monomer variable antibody domain (e.g., a single heavy chain variable region, also called VHH), which retains the ability to specifically bind to the same antigen bound by the full-length antibody (Holt, L. et al., Trends in Biotechnology, 21(11):484-490, 2003). Single-domain antibody or nanobody can be an alpaca antibody derived from camels or a shark antibody derived from sharks.

Nanobodies can be screened for specificity in the same manner as for intact antibodies using conventional techniques known to those skilled in the art.

As used herein, the expression "specific binding" refers to a non-random binding reaction between two molecules, such as a reaction between an antibody and an antigen to which it is directed. The strength or affinity of a specific binding interaction can be represented by the equilibrium dissociation constant (KD) of the interaction. In the present invention, the term "KD" refers to the dissociation equilibrium constant of a specific antibody-antigen interaction, which is used to describe the binding affinity between an antibody and an antigen. The smaller the equilibrium dissociation constant, the tighter the antibody-antigen binding and the higher the affinity between the antibody and the antigen.

According to the present invention, the term "adjuvant" refers to an immunopotentiator that, when delivered into the body with an antigen together or in advance, can enhance the body's immune response to the antigen or change the type of immune response. There are many kinds of adjuvants, including but not limited to aluminum salt adjuvant, zinc-aluminum mixed adjuvant (e.g., FH002C), Freund's adjuvant, oil emulsion adjuvant, cytokine, TLR agonist, CpG adjuvant, liposome, AS01B adjuvant, or a combination thereof.

As used herein, the term "pharmaceutically acceptable" means that it is recognized in the pharmaceutical field that it can be used in animals, especially in humans. As used herein, the term "pharmaceutically acceptable carrier and/or excipient" refers to a carrier and/or excipient that is pharmacologically and/or physiologically compatible with a subject and an active ingredient, and that is well known in the art (see, for examples, Remington's Pharmaceutical Sciences. Edited by Gennaro AR, 19th ed. Pennsylvania: Mack Publishing Company, 1995), and includes, but is not limited to: pH adjuster (including, but not limited to, phosphate buffer), surfactant (including, but not limited to, cationic, anionic or nonionic surfactant, such as Tween-80), adjuvant, ionic strength enhancer (including, but not limited to, sodium chloride), diluent, excipient, medium for containing or administering therapeutic agent, and any combination thereof.

As used herein, the term "subject" refers to a mammal, including, but not limited to, human, rodent (mouse, rat, guinea pig), dog, horse, cow, cat, pig, monkey, chimpanzee, etc. Preferably, the subject is a human.

As used herein, the term "effective amount" refers to an amount sufficient to obtain or at least partially obtain the desired effect. For example, a disease prevention effective amount refers to an amount sufficient to prevent, arrest, or delay the occurrence of a disease; a disease treatment effective amount refers to an amount sufficient to cure or at least partially prevent the disease and its complications in a patient who already has the disease. Determining such an effective amount is well within the capabilities of those skilled in the art. For example, the amount effective for therapeutic use will depend on the severity of the disease to be treated, the overall state of the patient's own immune system, the patient's general condition such as age, weight and gender, the administration mode of drug, and other treatments administered simultaneously, etc.

As used herein, the term "a condition allowing VLP assembly" refers to a condition under which the assembly polypeptide can be assembled into VLPs. Typically, when a polypeptide or protein with assembly ability is placed in a liquid, it can self-assemble into VLPs. In certain embodiments, the conditions allowing VLP assembly are to place an assembly polypeptide in a solution. In such embodiments, the solution will not contain a component that is not conducive to VLP assembly. In certain embodiments, the solution contains a component that is conducive to VLP assembly, for example, the solution is a buffer containing salt.

Beneficial effects of the invention

In the present invention, the ability of assembly polypeptides (e.g., ORF2 protein of HEV or fragments thereof or their variants, L1 protein of HPV or fragments thereof or their variants, surface antigen of HBV or fragments thereof or their variants) to assemble into VLPs is utilized for fusion expression of nanobodies that specifically bind the assembly polypeptides with immunogenic polypeptides, and the targeted binding property of the nanobodies to the assembly polypeptides is utilized to display a variety of immunogenic polypeptides on the surface of the VLPs formed from the assembly polypeptides, thereby obtaining particulate immunogenic polypeptides. Thus, the present application provides a system and method for granulating immunogenic polypeptides.

Moreover, compared with ordinary immunogenic polypeptides, such particulate immunogenic polypeptides have higher immunogenicity and can stimulate high-level immune responses, including B cell and T cell responses. Therefore, the particulate immunogenic polypeptides of the present invention are particularly suitable for vaccine production and vaccination, and have advantages in preventing and/or treating viral infections.

The system and method for granulating immunogenic polypeptide of the present invention use a nanobody. Compared with the prior art, the use of nanobody has at least the following advantages: ① The display of immunogenic polypeptide mediated by the nanobody is not affected by the N/C terminal structure of the assembly polypeptide, which allows more flexible construction strategy. ② The display of immunogenic polypeptide mediated by the nanobody will not affect the assembly and conformation of the assembly polypeptide, which brings about broader applicability. ③ The fusion of the nanobody and the immunogenic polypeptide is conducive to maintaining the conformation of the immunogenic polypeptide. ④ The nanobodies with different epitopes can be combined together, which is conducive to increasing the display proportion of the immunogenic polypeptide. ⑤ The nanobody can be reasonably designed based on the symmetry of the assembly polypeptide and the oligomeric state of the immunogenic polypeptide. ⑥ The display of immunogenic polypeptide mediated by the nanobody can reduce the potential steric hindrance of the immunogenic polypeptide. ⑦ The system and method can directly use a unmodified or modified VLP, and if the VLP that has been marketed has good safety itself, it needs no consideration on its own drugability issues, and has good convertibility. ⑧ The nanobody as linker can minimize its immunogenicity through a humanization strategy well known in the art, thereby reducing the body's unnecessary immune response, and achieving the immunogenic polypeptide's immune focusing. ⑨ Any immunogenic polypeptide can be particulate by the method of the present invention through the fusion expression with nanobodies, which has universality and versatility. ⑩ Due to the small size of nanobodies, by screening and identifying the binding position of nanobodies, the exposure of the original main immune epitopes of particulate carriers such as HEV ORF2, HPV L1 and HBV S can be retained, the carrier after granulation of immunogenic polypeptide still has good immunogenicity and can be used as a combined vaccine, and this strategy can also be used for the development of other combined vaccines, thus having great application prospects.

The embodiments of the present invention will be described in detail in conjunction with the accompanying drawings and examples, but those skilled in the art will understand that the following drawings and examples are only used to illustrate the present invention, not to limit the scope of the present invention. According to the following detailed description of the drawings and preferred embodiments, the various objects and advantages of the present invention will become apparent to those skilled in the art.

**Brief Description of the Drawings**

Fig. 1 shows the screening process of nanobodies in the example of the present invention. Fig. 1A shows the results of the colony PCR agarose gel electrophoresis for the bacterial library. Fig. 1B shows the results of evaluating the diversity of the bacterial library after sequencing.

Fig. 2 shows the results of SDS-polyacrylamide gel electrophoresis (SDS-PAGE) of various VHH-RBD proteins in the example of the present invention. Fig. 2A shows the electrophoresis results of various VHH-RBD fusion proteins produced and purified by the insect cell expression system. Fig. 2B shows the electrophoresis results of various VHH-RBD fusion proteins produced and purified by the mammalian cell expression system. M represents molecular weight marker.

Fig. 3 shows the results of SDS-polyacrylamide gel electrophoresis (SDS-PAGE) of various VHH-gE fusion proteins in the example of the present invention. M represents molecular weight marker, and lanes 1 to 7 show different VHH-gE fusion proteins, respectively.

Fig. 4 shows the results of SDS-polyacrylamide gel electrophoresis (SDS-PAGE) of various VHH-BGTSTIP proteins in the example of the present invention. "+" indicates reducing conditions, and "-" indicates non-reducing conditions.

Fig. 5 shows the results of Western Blot of various VHH-RBD fusion proteins in the example of the present invention. Fig. 5A shows a variety of VHH-RBD fusion proteins produced and purified by the insect cell expression system, M represents molecular weight marker, and lanes 1 to 8 show fusion proteins of different nanobodies with RBD, respectively. Fig. 5B shows a variety of VHH-RBD fusion proteins produced and purified by the mammalian cell expression system 293F cells.

Fig. 6 shows the results of Western Blot of various candidate VHH-gE molecules in the example of the present invention. M represents molecular weight marker, and lanes 1 to 7 show fusion proteins of different nanobodies with gE, respectively.

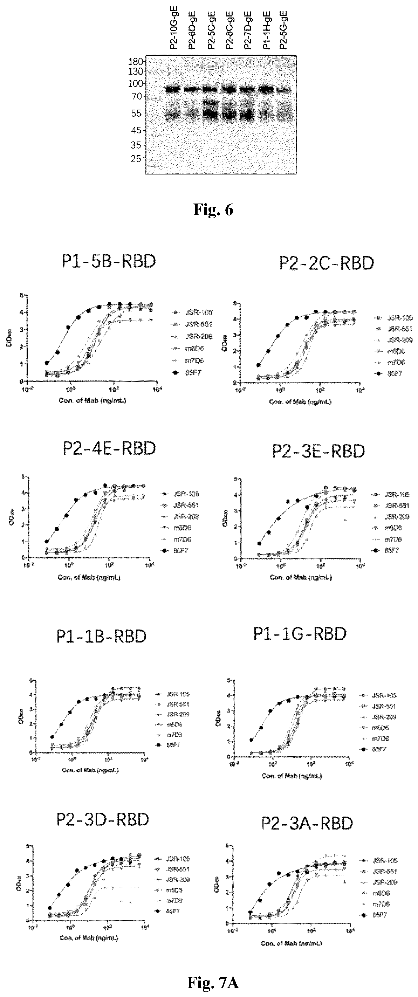

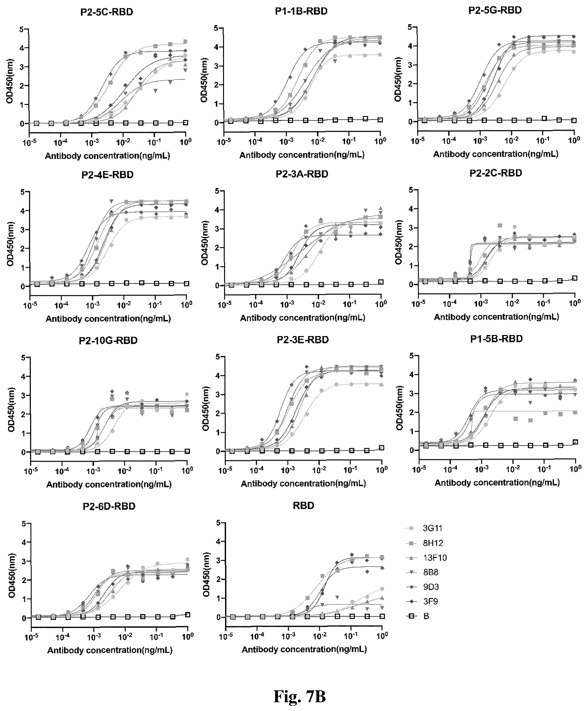

Fig. 7 shows the results of ELISA of various VHH-RBDs in the examples and various reported specific monoclonal antibodies. Fig. 7A shows the activity identification results of various VHH-RBD proteins produced and purified by the insect cell expression system, and the antibodies include broad-spectrum neutralizing antibodies JSR-105, JSR-551, JSR-209, m6D6, m7D6, and non-broad-spectrum neutralizing antibody 85F7. Fig. 7B shows the activity identification results of various VHH-RBD proteins produced and purified by the mammalian cell expression system 293F cells, and the antibodies include 3G11, 8H12, 13F10, 8B8, 9D3, and 3F9.

Fig. 8 shows the results of ELISA of various VHH-gEs in the examples and various specific monoclonal antibodies. The specific monoclonal antibodies include 3H7, 4G4, 6B7, 11B11, 11B12, 13B6, 14G1, and 17B7.

Fig. 9 shows the results of ELISA of various VHH-BGTSTIPs in the examples and various reported HIV-1 neutralizing antibodies or non-neutralizing antibodies.

Fig. 10 shows the results of affinity analysis of various VHH-RBDs in the examples with HEV-p239 protein. Fig. 10A shows the affinity results of VHH-RBD produced and purified by the insect cell expression system with HEV-p239 protein, and Fig. 10B shows the affinity results of VHH-RBD produced and purified by the mammalian cell expression system 293F cells with HEV-p239 protein.

Fig. 11 shows the results of affinity analysis of various VHH-gEs in the examples with HEV-p239 protein.

Fig. 12 shows the results of affinity analysis of various VHH-BGTSTIPs in the examples with HEV-p239 protein.

Fig. 13A shows the results of superdex 200 increase (high performance liquid chromatography molecular sieve) purification of P1-5B-RBD produced and purified by the insect cell expression system in the example of the present invention. Fig. 13B shows the results of superdex 200 increase purification of P2-6D-RBD, P2-3E-RBD, and P2-10G-RBD produced and purified by the mammalian cell expression system in the example of the present invention.

Fig. 14A shows the purification profile and SDS-PAGE identification results of the complex formed by the P1-5B-RBD fusion protein purified by the insect cell expression system and HEV-p239. Fig. 14B shows the purification profile and SDS-PAGE identification results of the complexes formed by the P2-3E-RBD, P2-10G-RBD, P2-6D-RBD fusion proteins purified by the mammalian cell expression system and HEV-p239, respectively.

Fig. 15 shows the purification profile and SDS-PAGE identification results of the complex formed by the candidate fusion protein P2-8C-gE and HEV-p239 in the example of the present invention. Fig. 15A shows the purification results of the complex. Fig. 15B shows the SDS-PAGE identification results of the complex formed by the P2-8C-gE and HEV-p239.

Fig. 16 shows the purification profile and SDS-PAGE identification results of the complex formed by the candidate fusion protein P2-5C-BGTSTIP and HEV-p239 in the example of the present invention.

Fig. 17 shows the results of HPSEC of P2-8C-gE in the example of the present invention.

Fig. 18A shows the molecular size detection results of HEV-RBD complex, which is formed by the VHH-RBD produced and purified by the insect cell expression system and HEV-p239 protein, and HEV-p239 particles in the example of the present invention. Fig. 18B shows the molecular size detection results of three HEV-RBD complexes, which are formed by VHH-RBDs produced and purified by mammalian cell expression system and HEV-p239 protein, and HEV-p239 particles.

Fig. 19 shows the molecular size detection results of HEV-gE complex samples of the present invention. Fig. 19A shows the molecular size of the HEV-p239 particles, and Fig. 19B shows the molecular size of the HEV-gE complexes.

Fig. 20 shows the molecular size detection results of HEV-BGTSTIP complexes and HEV-p239 particles in the example of the present invention.

Fig. 21 shows the analytical ultracentrifugation results of the HEV-p239 and the RBD complex constructed based on HEV-p239 particles in the example of the present invention. Fig. 21A shows the analytical ultracentrifugation results of the complex particles prepared by the VHH-RBD produced and purified by the insect cell expression system and HEV-p239 (right figure), and HEV-p239 (left figure). Fig. 21B shows the analytical ultracentrifugation results of three complex particles prepared by the VHH-RBD produced and purified by the mammalian cell expression system 293F cells, and HEV-p239.

Fig. 22 shows the analytical ultracentrifugation results of the HEV-p239 and the gE complex constructed based on HEV-p239 particles in the example of the present invention. Fig. 22A shows that HEV-p239 presents a single component, and has a sedimentation coefficient of 22S. Fig. 22B shows that the gE complex presents a single component, and has a sedimentation coefficient of 31S.

Fig. 23 shows the analytical ultracentrifugation results of the HEV-p239 and the Env (BGTSTIP) complex constructed based on HEV-p239 particles in the example of the present invention.

Fig. 24A shows the transmission electron microscopy negative staining results of the HEV-p239 and the complex sample prepared by the VHH-RBD produced and purified by the insect cell expression system in the example of the present invention. Fig. 24B shows the transmission electron microscopy negative staining results of the HEV-p239 and the complex samples prepared by the VHH-RBDs produced and purified by the mammalian expression system 293F cells in the example of the present invention.

Fig. 25 shows the transmission electron microscopy negative staining results of the HEV-p239 and the complex sample prepared by the VHH-gE fusion protein in the present invention.

Fig. 26 shows the transmission electron microscopy negative staining results of the HEV-p239 and the Env (BGTSTIP) complex constructed based on the HEV-p239 particles in the example of the present invention.

Fig. 27 shows the immunogenicity detection results of the RBD particulate antigens in the example of the present invention. Fig. 27A shows the detection results of serum binding and neutralization activity after the mice were immunized with the RBD particulate antigens obtained by the insect cell expression system. Fig. 27B and Fig. 27C show the detection results of serum binding activity and wild-type SARS-CoV-2 pseudovirus neutralization activity after the mice were immunized with the RBD particulate antigens obtained by the mammalian cell expression system.

Fig. 28 shows the neutralization detection results of the antibodies induced by the RBD particulate antigens and aluminum adjuvant in the example of the present invention for the wild-type (WT) strain, gamma strain, and BA.2 strain of SARS-CoV-2.

Fig. 29 shows the immunogenicity detection results of the gE particulate antigens in the example of the present invention.

Fig. 30 shows the mouse immune serum live virus neutralization detection results of the gE particulate antigens in the example of the present invention.

Fig. 31 shows the flow cytometry immunoassay results of the monomeric gE antigen, gE particulate antigen, and control adjuvant in the example of the present invention.

Fig. 32 shows the enzyme-linked immunospot assay results of detecting cytokines in the immunization groups using the monomeric gE antigen, gE particulate antigen, and control adjuvant in the example of the present invention.

Fig. 33 shows the results of humanization transformation of nanobody P1-5B in the example of the present invention.

Fig. 34 shows the polyacrylamide electrophoresis results of the single-domain antibody fusion protein P1F8-BGTSTIP in the example of the present invention; wherein, M represents molecular weight marker; "+" indicates reducing conditions, and "-" indicates non-reducing conditions.

Fig. 35 shows the results of ELISA of the single-domain antibody fusion protein P1F8-BGTSTIP in the example of the present invention and a variety of reported broad-spectrum neutralizing antibodies and non-neutralizing antibodies against HIV-1, in which 2G12, VRC01, PGT121, PGT121, SF12, and B12 are broad-spectrum neutralizing antibodies; and 17b, F105, and F240 are non-neutralizing antibodies.

Fig. 36 shows the results of affinity analysis of the single-domain antibody fusion protein P1F8-BGTSTIP in the examples with HPV 58VLP.

Fig. 37 shows the high-performance size exclusion chromatography (HPSEC) and SDS-PAGE identification results of the complex formed by P1F8-BGTSTIP produced and purified by the mammalian expression system in the example of the present invention and 58VLP. The red curve represents the spectrum of the complex formed by P1F8-BGTSTIP and 58VLP, the green curve represents the spectrum of the 58VLP, and the blue curve represents the spectrum of the fusion protein P1F8-BGTSTIP.

Fig. 38 shows the analytical ultracentrifugation results of the complex formed by P1F8-BGTSTIP and 58VLP and the 58VLP in the example of the present invention.

Fig. 39 shows the transmission electron microscopy negative staining results of the complex sample formed by P1F8-BGTSTIP and 58VLP in the example of the present invention.

Fig. 40 shows the titer detection results of the BGTSTIP-specific binding antibody after the mice were immunized with the particulate antigen 58VLP-BGTSTIP in the example of the present invention.

Fig. 41 shows the SDS-PAGE identification diagram of the particulate antigen formed after the HBsAg binds to the nanobody fusion protein S2-gE in the present invention.

Fig. 42 shows the molecular sieve identification diagram of the particulate antigen formed after the HBsAg binds to the nanobody fusion protein S2-gE in the present invention.

Fig. 43 shows the DLS identification diagram of the particulate antigen HBsAg-S2-gE (HBV-gE) formed after the HBsAg binds to the nanobody fusion protein S2-gE in the present invention.

Fig. 44 shows the transmission electron microscopy negative staining photographs of the particulate antigen HBsAg-S2-gE formed after the HBsAg binds to the nanobody fusion protein S2-gE in the present invention.

Fig. 45 shows the specific antibody titer results of the mice immunized with the particulate antigen HBsAg-S2-gE formed after the HBsAg binds to the nanobody fusion protein S2-gE in the present invention and the S2-gE.

Sequence information

The information of some sequences involved in the present invention is provided in Table 1 below.

Table 1

| SEQ ID NO. | Description | Sequence information |
|---|---|---|
| 1 | SARS-CoV-2 wild-type RBD protein amino acid sequence | RVQPTESIVRFPNITNLCPFGEVFNATRFASVYAWNRKRISNCVADYSVLY NSASFSTFKCYGVSPTKLNDLCFTNVYADSFVIRGDEVRQIAPGQTGKIA DYNYKLPDDFTGCVIAWNSNNLDSKVGGNYNYLYRLFRKSNLKPFERDI STEIYQAGSTPCNGVEGFNCYFPLQSYGFQPTNGVGYQPYRVVVLSFELL HAPATVCGPKKSTNLVKNKCVNF |
| 2 | SARS-CoV-2 Alpha strain RBD protein amino acid sequence | RVQPTESIVRFPNITNLCPFGEVFNATRFASVYAWNRKRISNCVADYSVLY NSASFSTFKCYGVSPTKLNDLCFTNVYADSFVIRGDEVRQIAPGQTGKIA DYNYKLPDDFTGCVIAWNSNNLDSKVGGNYNYLYRLFRKSNLKPFERDI STEIYQAGSTPCNGVEGFNCYFPLQSYGFQPTYGVGYQPYRVVVLSFELL HAPATVCGPKKSTNLVKNKCVNF |
| 3 | SARS-CoV-2 Beta strain RBD protein amino acid sequence | RVQPTESIVRFPNITNLCPFGEVFNATRFASVYAWNRKRISNCVADYSVLY NSASFSTFKCYGVSPTKLNDLCFTNVYADSFVIRGDEVRQIAPGQTGNIA DYNYKLPDDFTGCVIAWNSNNLDSKVGGNYNYLYRLFRKSNLKPFERDI STEIYQAGSTPCNGVKGFNCYFPLQSYGFQPTYGVGYQPYRVVVLSFELL HAPATVCGPKKSTNLVKNKCVNF |
| 4 | SARS-CoV-2 Gamma strain RBD protein amino acid sequence | RVQPTESIVRFPNITNLCPFGEVFNATRFASVYAWNRKRISNCVADYSVLY NSASFSTFKCYGVSPTKLNDLCFTNVYADSFVIRGDEVRQIAPGQTGTIAD YNYKLPDDFTGCVIAWNSNNLDSKVGGNYNYLYRLFRKSNLKPFERDIS TEIYQAGSTPCNGVKGFNCYFPLQSYGFQPTYGVGYQPYRVVVLSFELLH APATVCGPKKSTNLVKNKCVNF |
| 5 | SARS-CoV-2 Delta strain RBD protein amino acid sequence | RVQPTESIVRFPNITNLCPFGEVFNATRFASVYAWNRKRISNCVADYSVLY NSASFSTFKCYGVSPTKLNDLCFTNVYADSFVIRGDEVRQIAPGQTGKIA

DYNYKLPDDFTGCVIAWNSNNLDSKVGGNYNYRYRLFRKSNLKPFERDI STEIYQAGSKPCNGVEGFNCYFPLQSYGFQPTNGVGYQPYRVVVLSFELL HAPATVCGPKKSTNLVKNKCVNF |
| 6 | SARS-CoV-2 BA.2 strain RBD protein amino acid sequence | RVQPTESIVRFPNITNLCPFDEVFNATRFASVYAWNRKRISNCVADYSVLY NFAPFFAFKCYGVSPTKLNDLCFTNVYADSFVIRGNEVSQIAPGQTGNIAD YNYKLPDDFTGCVIAWNSNKLDSKVGGNYNYLYRLFRKSNLKPFERDIS TEIYQAGNKPCNGVAGFNCYFPLRSYGFRPTYGVGHQPYRVVVLSFELLH APATVCGPKKSTNLVKNKCVNF |

18

EP 4 628 095 A1

(continued)

| SEQ ID NO. | Description | Sequence information |
|---|---|---|
| 7 | SARS-CoV-2 BA.4 strain RBD protein amino acid sequence | RVQPTESIVRFPNITNLCPFDEVFNATRFASVYAWNRKRISNCVADYSVLY NFAPFFAFKCYGVSPTKLNDLCFTNVYADSFVIRGNEVSQIAPGQTGTIAD YNYKLPDDFTGCVIAWNSNKLDSKVGGNYNYRYRLFRKSNLKPFERDIS TEIYQAGNKPCNGVKGVNCYFPLQSYGFRPTYGVGHQPYRVVVLSFELL HAPATVCGPKKSTNLVKNKCVNF |
| 8 | SARS-CoV-2 BA.5 strain RBD protein amino acid sequence | RVQPTESIVRFPNITNLCPFDEVFNATRFASVYAWNRKRISNCVADYSVLY NFAPFFAFKCYGVSPTKLNDLCFTNVYADSFVIRGNEVSQIAPGQTGTIAD YNYKLPDDFTGCVIAWNSNKLDSKVGGNYNYRYRLFRKSNLKPFERDIS TEIYQAGNKPCNGVKGVNCYFPLQSYGFRPTYGVGHQPYRVVVLSFELL HAPATVCGPKKSTNLVKNKCVNF |
| 9 | HEV ORF2 amino acid sequence | MRPRPILLLLLMFLPMLPAPPPGQPSGRRRGRRSGGSGGGFWGDRVDSQP FAIPYIHPTNPFAPDVTAAAGAGPRVRQPARPLGSAWRDQAQRPAAASRR RPTTAGAAPLTAVAPAHDTPPVPDVDSRGAILRRQYNLSTSPLTSSVATGTN LVLYAAPLSPLLPLQDGTNTHIMATEASNYAQYRVARATIRYRPLVPNAVG GYAISISFWPQTTTTPTSVDMNSITSTDVRILVQPGIASELVIPSERLHYRNQ GWRSVETSGVAEEEATSGLVMLCIHGSPVNSYTNTPYTGALGLLDFALEL EFRNLTPGNTNTRVSRYSSTARHRLRRGADGTAELTTTAATRFMKDLYFTS TNGVGEIGRGIALTLFNLADTLLGGLPTELISSAGGQLFYSRPVVSANGEP TVKLYTSVENAQQDKGIAIPHDIDLGESRVVIQDYDNQHEQDRPTPSPAPS RPFSVLRANDVLWLSLTAAEYDQSTYGSSTGPVYVSDSVTLVNVATGAQ AVARSLDWTKVTLDGRPLSTIQQYSKTFFVLPLRGKLSFWEAGTTKAGYP YNYNTTASDQLLVENAAGHRVAISTYTTSLGAGPVSISAVAVLAPHSVLAL LEDTMDYPARAHTFDDFCPECRPLGLQGCAFQSTVAELQRLKMKVGKTR EL* |
| 10 | P1-1B nanobody amino acid sequence | DVQLQESGGGLVQPGGSLRLSCAASGFTLSRSTMTWGRQAPGKGLEWV SDIYSSGSTTYADSVKGRFTISRDNAKNTVYLQMNSLKPEDTAVYYCAVV LPGREYRGQGTQVTVSS |
| 11 | P1-1G nanobody amino acid sequence | DVQLQESGGGLVQPGGSLRLSCAASGFSLDYYAIGWFRQAPGKEREGVS CVSGREGSTIYTNSVKGRFTISRDNAKNTVHLQMNSLKPEDTAVYYCAA AGWNGLGCPPSLYEYDYWGQGTQVTVSS |

EP 4 628 095 A1

19

| SEQ ID NO. | Description | Sequence information |
|---|---|---|
| 12 | P1-5B nanobody amino acid sequence | DVQLQESGGGLVQPGGSLRLSCAASTFTLDYYAIGWFRQAPGKEREGVS CISSRGGSTDYADSVKGRFTISRDNAKNTVYLQMSSLQPEDTAVYYCAAA GWAGYGCPPNRYEYDYWGQGTQVTVSS |
| 13 | P1-5-E nanobody amino acid sequence | DVQLQESGGGLVQPGGSLRLSCAASGFTLDYYTIGWFRQAPGKEREGVS CISSSDDSTYYADSVKGRFTISRDNAKNTVFLQMNSLKPEDTAVYSCAAV PRHTGISPVLAMCASSVWYDYWGQGTQVTVSS |
| 14 | P1-11B nanobody amino acid sequence | DVQLQESGGGLVQPGGSLRLSCAASGFTLDYYAVGWFRQAPGKEREGVS CLSGRNGSPYYANSGKGRFTISRDNAENTVYLQMTGLEPEDTAVYYCAA AGWNGLGCPPSLYEYDYWGQGTQVTVSS |
| 15 | P2-2C nanobody amino acid sequence | DVQLQESGGGLVQPGGSLRLSCAASGFTLDYYAIGWFRQAPGKEREGVS CISGRSDSTNYADSVKGRFTISRDNAKNAVYLQMNSLKPEDTAVYYCAVS GWAGNGCPPIYEYDYWGQGTQVTVSS |
| 16 | P2-3A nanobody amino acid sequence | DVQLQESGGGLVQPGGSLRLSCAASGFTLDYYAIGWFRQAPGKEREEVS CISSRGGSTNYPDSVKGRFTISRDNAKNTVYLQMNSLRPEDTAVYYCAAA GWAGFGCPPNRYEYDYWGQGTQVTVSS |
| 17 | P2-3D nanobody amino acid sequence | DVQLQESGGGLVQPGGSLRLSCAGSGFTLDYYAIGWFRQAPGKEREGVS CISGRGGSTNYADSTKGRFTISRDNAKNTVYLQMNSLKPEDTAVYYCAA AGWAGNVCPPNRYEYDYWGQGTQVTVSS |
| 18 | P2-3E nanobody amino acid sequence | DVQLQESGGGLVQPGGSLRLSCAASGFTLDYYAIGWFRQAPGEVREEVS CISGRGDSTNYAHSMKGRFTISRDNAKNTVYLQINSLKPEDTAVYYCAAA GWAGYECPANRYEYDYWGQGTQVTVSS |
| 19 | P2-4E nanobody amino acid sequence | DVQLQESGGGLVQPGGSLRLSCAASGFTLDYYAIGWFRQAPGKEREEVS CISGRGSSTNYADSVKGRFTVSRDNAKNTVYLQINSLKPEDTAVYYCAAA GWAGYGCPANRYEYDYWGQGTQVTVSS |
| 20 | P2-4G nanobody amino acid sequence | DVQLQESGGGLVQPGGSTRLSCAASGFTLDYYAIGWFRQAPGKEREEVS CMSSRGGSTNYVDSVQGRFTLSRDNAKNTVYLQMNSLKPEDTAVYYCA AAGWAGNGCPPNRYEYDYWGQGTQVTVSS |

| SEQ ID NO. | Description | Sequence information |
|---|---|---|
| 21 | P2-5C nanobody amino acid sequence | DVQLQESGGGLVQPGGSLRLSCAASGFTLDYYAIGWFRQAPGKEREGVS CISSRGGSTNTPDSVKGRFTISRDNAKNTVYLQMNSLRPEDTAVYYCAAA GWAGFGCPPNRYEYDYWGQGTQVTVSS |
| 22 | P2-5G nanobody amino acid sequence | DVQLQESGGGLVQPGGSLRLSCAASGFTLSRSTMTWGRQAPGKGLEWV SDIYRSGSTTYADSVKGRFTISRDDAKNTVYLQMNSLKPEDTAVYYCAVV LPGREYRGQGTQVTVSS |
| 23 | P2-6D nanobody amino acid sequence | DVQLQESGGGLVQPGGSLRLSCAASTFTLDYYAIGWFRQAPGKEREEVS CISSRSGSTNYPDSVKGRFTISRDNAKNTVYLQMNSLKPEDTAVYYCAAA GWAGYGCPANRYEYDYWGQGTQVTVSS |
| 24 | P2-7C nanobody amino acid sequence | DVQLQESGGGLVQPGGSLRLSCAASGFTLDHYAIGWFRQAPGKEREGISC ISSRRGSTNYEDSVKGRFTISRDNAKNTVYLQMNSLKPEDTAVYYCAAA GWAGYMCPPNRYEYDYWGQGTQVTVSS |
| 25 | P2-7D nanobody amino acid sequence | DVQLQESGGGLVQPGGSLRLSCAASRFTLDYYAIGWFRQAPGKEREEVS CISSRAGSTNYQDSVKGRFTISRDNAKNTVYLQMNSLKPEDTAVYYCAA AGWAGNACPPNRYEYDYWGQGTQVTVSS |
| 26 | P2-8C nanobody amino acid sequence | DVQLQESGGGLVQPGGSLRLSCAASGFTLDYYAIGWFRQAPGKEREGVS CISGRRGSTIYANSVKGRFTNSRDNAKNTVYLQINSLKPEDTAVYYCAAA GWNGLGCPPSLDEYDYWGQGTQVTVSS |
| 27 | P2-10G nanobody amino acid sequence | DVQLQESGGGLVQPGGSLRLSCAASGFTLDYYAIGWFRQAPGKEREGVS CVSGRHGSTIYANSVKGRFTNSRDNAKNTVYLQINSLKPEDTAVYYCAA AGWNGLGCPPSLDEYDYWGQGTQVTVSS |
| 28 | P2-11H nanobody amino acid sequence | DVQLQESGGGLVQPGGSLRLSCAASGLTLDYYNIGWFRQAPGKEREGVS CISVSGGVTNYADSVMGRFTVSRDNAKNTVYLQMNSLKPEDTAVYYCA AVSLPFVPMLGCPEGYDYWGQGTQVTVSS |
| 29 | P1-1H nanobody amino acid sequence | DVQLQESGGGLVQPGGSLRLSCAASGFTLDEYTIGWFRQAPGKERQGVS CISSRGGRTNYADSVKGRFTISRDNAKNTVYLLMNSLEPEDTAVYYCAAA SYYDDCRVPAWYDYWGQGTQVTVSS |

EP 4 628 095 A1

| SEQ ID NO. | Description | Sequence information |
|---|---|---|
| 30 | gE protein amino acid sequence | MGTVNKPVVGVLMGFGIITGTLRITNPVRASVLRYDDFHIDEDKLDTNSV YEPYYHSDHAESSWVNRGESSRKAYDHNSPYIWPRNDYDGFLENAHEH HGVYNQGRGIDSGERLMQPTQMSAQEDLGDDTGIHVIPTLNGDDRHKIV NVDQRQYGDVFKGDLNPKPQGQRLIEVSVEENHPFTLRAPIQRIYGVRYT ETWSFLPSLTCTGDAAPAIQHICLKHTTCFQDVVVDVDCAENTKEDQLAE ISYRFQGKKEADQPWIVVNTSTLFDELELDPPEIEPGVLKVLRTEKQYLG VYIWNMRGSDGTSTYATFLVTWKGDEKTRNPTPAVTPQPRGAEFHMWN YHSHVFSVGDTFSLAMHLQYKIHEAPFDLLLEWLYVPIDPTCQPMRLYST CLYHPNAPQCLSHMNSGCTFTSPHLAQRVASTVYQNCEHADNYTAYCLG ISHMEPSFGLILHDGGTTLKFVDTPESLSGLYVFVVYFNGHVEAVAYTVVS TVDHFVNAIEERGFPPTAGQPPATTKPKEITPVNPGTSPLIRYAAWTGGLA AVVLLCLVIFLICTAKRMRVKAYRVDKSPYNQSMYYAGLPVDDFEDSEST DTEEEFGNAIGGSHGGSSYTVYIDKTR |
| 31 | Bee venom signal peptide amino acid sequence | KFLVNVALVFMVVYISYIYA |
| 32 | P1-5B-RBD fusion protein amino acid sequence | KFLVNVALVFMVVYISYIYADVQLQESGGGLVQPGGSLRLSCAASTFTLD YYAIGWFRQAPGKEREGVSCISSRGGSTDYADSVKGRFTISRDNAKNTVY LQMSSLQPEDTAVYYCAAAGWAGYGCPPNRYEYDYWGQGTQVTVSSRV QPTESIVRFPNITNLCPFGEVFNATRFASVYAWNRKRISNCVADYSVLYNS ASFSTFKCYGVSPTKLNDLCFTNVYADSFVIRGDEVRQIAPGQTGTIADY NYKLPDDFTGCVIAWNSNNLDSKVGGNYNYLYRLFRKSNLKPFERDISTE IYQAGSTPCNGVKGFNCYFPLQSYGFQPTYGVGYQPYRVVVLSFELLHAP ATVCGPKKSTNLVKNKCVNFHHHHHH |

EP 4 628 095 A1

| SEQ ID NO. | Description | Sequence information |
|---|---|---|
| 33 | P2-8C-gE fusion protein amino acid sequence | KFLVNVALVFMVVYISYIYADVQLQESGGGLVQPGGSLRLSCAASGFTLD YYAIGWFRQAPGKEREGVSCISGRRGSTIYANSVKGRFTNSRDNAKNTVY LQINSLKPEDTAVYYCAAAGWNGLGCPPSLDEYDYWGQGTQVTVSSRIT NPVRASVLRYDDFHIDEDKLDTNSVYEPYYHSDHAESSWVNRGESSRKA YDHNSPYIWPRNDYDGFLENAHEHHGVYNQGRGIDSGERLMQPTQMSA QEDLGDDTGIHVIPTLNGDDRHKIVNVDQRQYGDVFKGDLNPKPQGQRL IEVSVEENHPFTLRAPIQRIYGVRYTETWSFLPSLTCTGDAAPAIQHICLKH TTCFQDVVVDVDCAENTKEDQLAEISYRFQGKKEADQPWIVVNTSTLFD ELELDPPEIEPGVLKVLRTEKQYLGVYIWNMRGSDGTSTYATFLVTWKGD EKTRNPTPAVTPQPRGAEFHMWNYHSHVFSVGDTFSLAMHLQYKIHEAP FDLLLEWLYVPIDPTCQPMRLYSTCLYHPNAPQCLSHMNSGCTFTSPHLA QRVASTVYQNCEHADNYTAYCLGISHMEPSFGLILHDGGTTLKFVDTPES LSGLYVFVVYFNGHVEAVAYTVVSTVDHFVNAIEERGFPPTAGQPPATTK PKEITPVNPGTSPLLRHHHHHHHHHH |
| 34 | Amino acid sequence of full-length Env extracellular segment (BG505 TSTIP) | AENLAVGIGAVFLGFLGAAGSTMGAASMTLTVQARNLLSGIVQQQSNLL RAPEAQQHLLKLTVWGIKQLQARVLAVERYLRDQQLLGIWGCSGKLICT TNVPNLWVTVYYGVPVWKDAETTLFCASDAKAYETEKHNVWATHACV PTDPNPQEIHLENVTEEFNMWKNNMVEQMHTDIISLWDQSLKPCVKLTP LCVTLQCTNVTNNITDDMRGELKNCSFNMTTELRDKKQKVYSLFYRLD VVQINENQGNRSNNSNKEYRLINCNTSAITQACPKVSFEPIPIHYCAPAGF AILKCKDKKFNGTGPCPSVSTVQCTHGIKPVVSTQLLLNGSLAEEEVMIR SENITNNAKNILVQFNTPVQINCTRPNNNTRKSIRIGPGQAFYATGDIIGDIR QAHCNVSKATWNETLGKVVKQLRKHFGNNTIIRFANSSGGDLEVTTHSF NCGGEFFYCNTSGLFNSTWISNTSVQGSNSTGSNDSITLPCRIKQIINMWQ RIGQAMYAPPIQGVIRCVSNITGLILTRDGGSTNSTTETFRPGGGDMRDN WRSELYKYKVVKIEPLGVAPTRRNLSEIWDNMTWLQWDKEISNYTQIIY GLLEESQNQQEKNEQDLLALDHHHHHHHH* |

| SEQ ID NO. | Description | Sequence information |
|---|---|---|
| 35 | Amino acid sequence of full-length Env extracellular segment (BG505 SOSIP) | AENLWVTVYYGVPVWKDAETTLFCASDAKAYETEKHNVWATHACVPT DPNPQEIHLENVTEEFNMWKNNMVEQMHTDIISLWDQSLKPCVKLTPLC VTLQCTNVTNNITDDMRGELKNCSFNMTTELRDKKQKVYSLFYRLDVV QINENQGNRSNNSNKEYRLINCNTSAITQACPKVSFEPIPIHYCAPAGFAIL KCKDKKFNGTGPCPSVSTVQCTHGIKPVVSTQLLLNGSLAEEEVMIRSEN ITNNAKNILVQFNTPVQINCTRPNNNTRKSIRIGPGQAFYATGDIIGDIRQA HCNVSKATWNETLGKVVKQLRKHFGNNTIIRFANSSGGDLEVTTHSFNC GGEFFYCNTSGLFNSTWISNTSVQGSNSTGSNDSITLPCRIKQIINMWQRI GQAMYAPPIQGVIRCVSNITGLILTRDGGSTNSTTETFRPGGGDMRDNWR SELYKYKVVKIEPLGVAPTRCKRRVVGRRRRRAVGIGAVFLGFLGAAGS TMGAASMTLTVQARNLLSGIVQQQSNLLRAPEAQQHLLKLTVWGIKQLQ ARVLAVERYLRDQQLLGIWGCSGKLICCTNVPWNSSWSNRNLSEIWDNM TWLQWDKEISNYTQIIYGLLEESQNQQEKNEQDLLALDHHHHHH |
| 36 | P2-5C BG505 TSTIP amino acid sequence | DVQLQESGGGLVQPGGSLRLSCAASGFTLDYYAIGWFRQAPGKEREGVS CISSRGGSTNTPDSVKGRFTISRDNAKNTVYLQMNSLRPEDTAVYYCAAA GWAGFGCPPNRYEYDYWGQGTQVTVSSGGGGSGGGGSGGGGSAENLA VGIGAVFLGFLGAAGSTMGAASMTLTVQARNLLSGIVQQQSNLLRAPEA QQHLLKLTVWGIKQLQARVLAVERYLRDQQLLGIWGCSGKLICTTNVPN LWVTVYYGVPVWKDAETTLFCASDAKAYETEKHNVWATHACVPTDPNP QEIHLENVTEEFNMWKNNMVEQMHTDIISLWDQSLKPCVKLTPLCVTLQ CTNVTNNITDDMRGELKNCSFNMTTELRDKKQKVYSLFYRLDVVQINEN QGNRSNNSNKEYRLINCNTSAITQACPKVSFEPIPIHYCAPAGFAILKCKD KKFNGTGPCPSVSTVQCTHGIKPVVSTQLLLNGSLAEEEVMIRSENITNN AKNILVQFNTPVQINCTRPNNNTRKSIRIGPGQAFYATGDIIGDIRQAHCNV SKATWNETLGKVVKQLRKHFGNNTIIRFANSSGGDLEVTTHSFNCGGEFF YCNTSGLFNSTWISNTSVQGSNSTGSNDSITLPCRIKQIINMWQRIGQAMY APPIQGVIRCVSNITGLILTRDGGSTNSTTETFRPGGGDMRDNWRSELYKY KVVKIEPLGVAPTRRNLSEIWDNMTWLQWDKEISNYTQIIYGLLEESQNQ QEKNEQDLLALDHHHHHHHH |
| 37 | tPA signal peptide sequence | MDAMKRGLCCVLLLCGAVFVSPSQEIHARFRRGAR |
| 38 | Antibody signal peptide | MGWSCIILFLVATATGVHS |
| 39 | Linker | GGGGSGGGGSGGGGS |

EP 4 628 095 A1

| SEQ ID NO. | Description | Sequence information |
|---|---|---|
| 40 | HEV p239 protein amino acid sequence | IALTLFNLADTLLGGLPTELISSAGGQLFYSRPVVSANGEPTVKLYTSVEN AQQDKGIAIPHDIDLGESRVVIQDYDNQHEQDRPTPSPAPSRPFSVLRAND VLWLSLTAAEYDQSTYGSSTGPVYVSDSVTLVNVATGAQAVARSLDWTK VTLDGRPLSTIQQYSKTFFVLPLRGKLSFWEAGTTKAGYPYNYNTTASDQ LLVENAAGHRVAISTYTTSLGAGPVSISAVAVLAPHSV |
| 41 | HEV p495 protein amino acid sequence | AVAPAHDTPPVPDVDSRGAILRRQYNLSTSPLTSSVATGTNLVLYAAPLSPL LPLQDGTNTHIMATEASNYAQYRVARATIRYRPLVPNAVGGYAISISFWPQ TTTTPTSVDMNSITSTDVRILVQPGIASELVIPSERLHYRNQGWRSVETSG VAEEEATSGLVMLCIHGSPVNSYTNTPYTGALGLLDFALELEFRNLTPGNT NTRVSRYSSTARHRLRRGADGTAELTTTAATRFMKDLYFTSTNGVGEIGR GIALTLFNLADTLLGGLPTELISSAGGQLFYSRPVVSANGEPTVKLYTSVE NAQQDKGIAIPHDIDLGESRVVIQDYDNQHEQDRPTPSPAPSRPFSVLRAN DVLWLSLTAAEYDQSTYGSSTGPVYVSDSVTLVNVATGAQAVARSLDWT KVTLDGRPLSTIQQYSKTFFVLPLRGKLSFWEAGTTKAGYPYNYNTTASD QLLVENAAGHRVAISTYTTSLGAGPVSISAVAVLAPHSV |
| 42 | P1-5B-RBD | DVQLQESGGGLVQPGGSLRLSCAASTFTLDYYAIGWFRQAPGKEREGVS CISSRGGSTDYADSVKGRFTISRDNAKNTVYLQMSSLQPEDTAVYYCAAA GWAGYGCPPNRYEYDYWGQGTQVTVSSRVQPTESIVRFPNITNLCPFGEV FNATRFASVYAWNRKRISNCVADYSVLYNSASFSTFKCYGVSPTKLNDLC FTNVYADSFVIRGDEVRQIAPGQTGKIADYNYKLPDDFTGCVIAWNSNNL DSKVGGNYNYLYRLFRKSNLKPFERDISTEIYQAGSTPCNGVEGFNCYFP LQSYGFQPTNGVGYQPYRVVVLSFELLHAPATVCGPKKSTNLVKNKCVN FHHHHHH |
| 43 | P2-2C-RBD | DVQLQESGGGLVQPGGSLRLSCAASGFTLDYYAIGWFRQAPGKEREGVS CISGRSDSTNYADSVKGRFTISRDNAKNAVYLQMNSLKPEDTAVYYCAVS GWAGNGCPPIYEYDYWGQGTQVTVSSRVQPTESIVRFPNITNLCPFGEVF NATRFASVYAWNRKRISNCVADYSVLYNSASFSTFKCYGVSPTKLNDLCF TNVYADSFVIRGDEVRQIAPGQTGKIADYNYKLPDDFTGCVIAWNSNNL DSKVGGNYNYLYRLFRKSNLKPFERDISTEIYQAGSTPCNGVEGFNCYFP LQSYGFQPTNGVGYQPYRVVVLSFELLHAPATVCGPKKSTNLVKNKCVN FHHHHHH |

| SEQ ID NO. | Description | Sequence information |
|---|---|---|
| 44 | P1-1B-RBD | DVQLQESGGGLVQPGGSLRLSCAASGFTLSRSTMTWGRQAPGKGLEWV SDIYSSGSTTYADSVKGRFTISRDNAKNTVYLQMNSLKPEDTAVYYCAVV LPGREYRGQGTQVTVSSRVQPTESIVRFPNITNLCPFGEVFNATRFASVYA WNRKRISNCVADYSVLYNSASFSTFKCYGVSPTKLNDLCFTNVYADSFVI RGDEVRQIAPGQTGKIADYNYKLPDDFTGCVIAWNSNNLDSKVGGNYN YLYRLFRKSNLKPFERDISTEIYQAGSTPCNGVEGFNCYFPLQSYGFQPTN GVGYQPYRVVVLSFELLHAPATVCGPKKSTNLVKNKCVNFHHHHHH |
| 45 | P1-1G-RBD | DVQLQESGGGLVQPGGSLRLSCAASGFSLDYYAIGWFRQAPGKEREGVS CVSGREGSTIYTNSVKGRFTISRDNAKNTVHLQMNSLKPEDTAVYYCAA AGWNGLGCPPSLYEYDYWGQGTQVTVSSRVQPTESIVRFPNITNLCPFGE VFNATRFASVYAWNRKRISNCVADYSVLYNSASFSTFKCYGVSPTKLNDL CFTNVYADSFVIRGDEVRQIAPGQTGKIADYNYKLPDDFTGCVIAWNSNN LDSKVGGNYNYLYRLFRKSNLKPFERDISTEIYQAGSTPCNGVEGFNCYF PLQSYGFQPTNGVGYQPYRVVVLSFELLHAPATVCGPKKSTNLVKNKCV NFHHHHHH |
| 46 | P2-4E-RBD | DVQLQESGGGLVQPGGSLRLSCAASGFTLDYYAIGWFRQAPGKEREEVS CISGRGSSTNYADSVKGRFTVSRDNAKNTVYLQINSLKPEDTAVYYCAAA GWAGYGCPANRYEYDYWGQGTQVTVSSRVQPTESIVRFPNITNLCPFGE VFNATRFASVYAWNRKRISNCVADYSVLYNSASFSTFKCYGVSPTKLNDL CFTNVYADSFVIRGDEVRQIAPGQTGKIADYNYKLPDDFTGCVIAWNSNN LDSKVGGNYNYLYRLFRKSNLKPFERDISTEIYQAGSTPCNGVEGFNCYF PLQSYGFQPTNGVGYQPYRVVVLSFELLHAPATVCGPKKSTNLVKNKCV NFHHHHHH |
| 47 | P2-3E-RBD | DVQLQESGGGLVQPGGSLRLSCAASGFTLDYYAIGWFRQAPGEVREEVS CISGRGDSTNYAHSMKGRFTISRDNAKNTVYLQINSLKPEDTAVYYCAAA GWAGYECPANRYEYDYWGQGTQVTVSSRVQPTESIVRFPNITNLCPFGEV FNATRFASVYAWNRKRISNCVADYSVLYNSASFSTFKCYGVSPTKLNDLC FTNVYADSFVIRGDEVRQIAPGQTGKIADYNYKLPDDFTGCVIAWNSNNL DSKVGGNYNYLYRLFRKSNLKPFERDISTEIYQAGSTPCNGVEGFNCYFP LQSYGFQPTNGVGYQPYRVVVLSFELLHAPATVCGPKKSTNLVKNKCVN FHHHHHH |

| SEQ ID NO. | Description | Sequence information |
|---|---|---|
| 48 | P2-3D-RBD | DVQLQESGGGLVQPGGSLRLSCAGSGFTLDYYAIGWFRQAPGKEREGVS CISGRGGSTNYADSTKGRFTISRDNAKNTVYLQMNSLKPEDTAVYYCAA AGWAGNVCPPNRYEYDYWGQGTQVTVSSRVQPTESIVRFPNITNLCPFGE VFNATRFASVYAWNRKRISNCVADYSVLYNSASFSTFKCYGVSPTKLNDL CFTNVYADSFVIRGDEVRQIAPGQTGKIADYNYKLPDDFTGCVIAWNSNN LDSKVGGNYNYLYRLFRKSNLKPFERDISTEIYQAGSTPCNGVEGFNCYF PLQSYGFQPTNGVGYQPYRVVVLSFELLHAPATVCGPKKSTNLVKNKCV NFHHHHHH |
| 49 | P2-3A-RBD | DVQLQESGGGLVQPGGSLRLSCAASGFTLDYYAIGWFRQAPGKEREEVS CISSRGGSTNYPDSVKGRFTISRDNAKNTVYLQMNSLRPEDTAVYYCAAA GWAGFGCPPNRYEYDYWGQGTQVTVSSRVQPTESIVRFPNITNLCPFGEV FNATRFASVYAWNRKRISNCVADYSVLYNSASFSTFKCYGVSPTKLNDLC FTNVYADSFVIRGDEVRQIAPGQTGKIADYNYKLPDDFTGCVIAWNSNNL DSKVGGNYNYLYRLFRKSNLKPFERDISTEIYQAGSTPCNGVEGFNCYFP LQSYGFQPTNGVGYQPYRVVVLSFELLHAPATVCGPKKSTNLVKNKCVN FHHHHHH |
| 50 | P2-10G-gE | DVQLQESGGGLVQPGGSLRLSCAASGFTLDYYAIGWFRQAPGKEREGVS CVSGRHGSTIYANSVKGRFTNSRDNAKNTVYLQINSLKPEDTAVYYCAA AGWNGLGCPPSLDEYDYWGQGTQVTVSSMGTVNKPVVGVLMGFGIITG TLRITNPVRASVLRYDDFHIDEDKLDTNSVYEPYYHSDHAESSWVNRGES SRKAYDHNSPYIWPRNDYDGFLENAHEHHGVYNQGRGIDSGERLMQPT QMSAQEDLGDDTGIHVIPTLNGDDRHKIVNVDQRQYGDVFKGDLNPKP QGQRLIEVSVEENHPFTLRAPIQRIYGVRYTETWSFLPSLTCTGDAAPAIQ HICLKHTTCFQDVVVDVDCAENTKEDQLAEISYRFQGKKEADQPWIVVN TSTLFDELELDPPEIEPGVLKVLRTEKQYLGVYIWNMRGSDGTSTYATFLV TWKGDEKTRNPTPAVTPQPRGAEFHMWNYHSHVFSVGDTFSLAMHLQY KIHEAPFDLLLEWLYVPIDPTCQPMRLYSTCLYHPNAPQCLSHMNSGCTF TSPHLAQRVASTVYQNCEHADNYTAYCLGISHMEPSFGLILHDGGTTLKF VDTPESLSGLYVFVVYFNGHVEAVAYTVVSTVDHFVNAIEERGFPPTAGQ PPATTKPKEITPVNPGTSPLIRYAAWTGGLAAVVLLCLVIFLICTAKRMRVK AYRVDKSPYNQSMYYAGLPVDDFEDSESTDTEEEFGNAIGGSHGGSSYT VYIDKTRHHHHHH |

| SEQ ID NO. | Description | Sequence information |
|---|---|---|
| 51 | P2-6D-gE | DVQLQESGGGLVQPGGSLRLSCAASTFTLDYYAIGWFRQAPGKEREEVS CISSRSGSTNYPDSVKGRFTISRDNAKNTVYLQMNSLKPEDTAVYYCAAA GWAGYGCPANRYEYDYWGQGTQVTVSSMGTVNKPVVGVLMGFGIITGT LRITNPVRASVLRYDDFHIDEDKLDTNSVYEPYYHSDHAESSWVNRGESS RKAYDHNSPYIWPRNDYDGFLENAHEHHGVYNQGRGIDSGERLMQPTQ MSAQEDLGDDTGIHVIPTLNGDDRHKIVNVDQRQYGDVFKGDLNPKPQ GQRLIEVSVEENHPFTLRAPIQRIYGVRYTETWSFLPSLTCTGDAAPAIQHI CLKHTTCFQDVVVDVDCAENTKEDQLAEISYRFQGKKEADQPWIVVNTS TLFDELELDPPEIEPGVLKVLRTEKQYLGVYIWNMRGSDGTSTYATFLVT WKGDEKTRNPTPAVTPQPRGAEFHMWNYHSHVFSVGDTFSLAMHLQYK IHEAPFDLLLEWLYVPIDPTCQPMRLYSTCLYHPNAPQCLSHMNSGCTFTS PHLAQRVASTVYQNCEHADNYTAYCLGISHMEPSFGLILHDGGTTLKFVD TPESLSGLYVFVVYFNGHVEAVAYTVVSTVDHFVNAIEERGFPPTAGQPPA TTKPKEITPVNPGTSPLIRYAAWTGGLAAVVLLCLVIFLICTAKRMRVKAY RVDKSPYNQSMYYAGLPVDDFEDSESTDTEEEFGNAIGGSHGGSSYTVYI DKTRHHHHHH |
| 52 | P2-5C-gE | DVQLQESGGGLVQPGGSLRLSCAASGFTLDYYAIGWFRQAPGKEREGVS CISSRGGSTNTPDSVKGRFTISRDNAKNTVYLQMNSLRPEDTAVYYCAAA GWAGFGCPPNRYEYDYWGQGTQVTVSSMGTVNKPVVGVLMGFGIITGT LRITNPVRASVLRYDDFHIDEDKLDTNSVYEPYYHSDHAESSWVNRGESS RKAYDHNSPYIWPRNDYDGFLENAHEHHGVYNQGRGIDSGERLMQPTQ MSAQEDLGDDTGIHVIPTLNGDDRHKIVNVDQRQYGDVFKGDLNPKPQ GQRLIEVSVEENHPFTLRAPIQRIYGVRYTETWSFLPSLTCTGDAAPAIQHI CLKHTTCFQDVVVDVDCAENTKEDQLAEISYRFQGKKEADQPWIVVNTS TLFDELELDPPEIEPGVLKVLRTEKQYLGVYIWNMRGSDGTSTYATFLVT WKGDEKTRNPTPAVTPQPRGAEFHMWNYHSHVFSVGDTFSLAMHLQYK IHEAPFDLLLEWLYVPIDPTCQPMRLYSTCLYHPNAPQCLSHMNSGCTFTS PHLAQRVASTVYQNCEHADNYTAYCLGISHMEPSFGLILHDGGTTLKFVD TPESLSGLYVFVVYFNGHVEAVAYTVVSTVDHFVNAIEERGFPPTAGQPPA TTKPKEITPVNPGTSPLIRYAAWTGGLAAVVLLCLVIFLICTAKRMRVKAY RVDKSPYNQSMYYAGLPVDDFEDSESTDTEEEFGNAIGGSHGGSSYTVYI DKTRHHHHHH |

EP 4 628 095 A1

| SEQ ID NO. | Description | Sequence information |
|---|---|---|
| 53 | P2-8C-gE | DVQLQESGGGLVQPGGSLRLSCAASGFTLDYYAIGWFRQAPGKEREGVS CISGRRGSTIYANSVKGRFTNSRDNAKNTVYLQINSLKPEDTAVYYCAAA GWNGLGCPPSLDEYDYWGQGTQVTVSSMGTVNKPVVGVLMGFGIITGT LRITNPVRASVLRYDDFHIDEDKLDTNSVYEPYYHSDHAESSWVNRGESS RKAYDHNSPYIWPRNDYDGFLENAHEHHGVYNQGRGIDSGERLMQPTQ MSAQEDLGDDTGIHVIPTLNGDDRHKIVNVDQRQYGDVFKGDLNPKPQ GQRLIEVSVEENHPFTLRAPIQRIYGVRYTETWSFLPSLTCTGDAAPAIQHI CLKHTTCFQDVVVDVDCAENTKEDQLAEISYRFQGKKEADQPWIVVNTS TLFDELELDPPEIEPGVLKVLRTEKQYLGVYIWNMRGSDGTSTYATFLVT WKGDEKTRNPTPAVTPQPRGAEFHMWNYHSHVFSVGDTFSLAMHLQYK IHEAPFDLLLEWLYVPIDPTCQPMRLYSTCLYHPNAPQCLSHMNSGCTFTS PHLAQRVASTVYQNCEHADNYTAYCLGISHMEPSFGLILHDGGTTLKFVD TPESLSGLYVFVVYFNGHVEAVAYTVVSTVDHFVNAIEERGFPPTAGQPPA TTKPKEITPVNPGTSPLIRYAAWTGGLAAVVLLCLVIFLICTAKRMRVKAY RVDKSPYNQSMYYAGLPVDDFEDSESTDTEEEFGNAIGGSHGGSSYTVYI DKTRHHHHHH |
| 54 | P2-1H-gE | DVQLQESGGGLVQPGGSLRLSCAASGLTLDYYNIGWFRQAPGKEREGVS CISVSGGVTNYADSVMGRFTVSRDNAKNTVYLQMNSLKPEDTAVYYCA AVSLPFVPMLGCPEGYDYWGQGTQVTVSSMGTVNKPVVGVLMGFGIIT GTLRITNPVRASVLRYDDFHIDEDKLDTNSVYEPYYHSDHAESSWVNRG ESSRKAYDHNSPYIWPRNDYDGFLENAHEHHGVYNQGRGIDSGERLMQP TQMSAQEDLGDDTGIHVIPTLNGDDRHKIVNVDQRQYGDVFKGDLNPKP QGQRLIEVSVEENHPFTLRAPIQRIYGVRYTETWSFLPSLTCTGDAAPAIQ HICLKHTTCFQDVVVDVDCAENTKEDQLAEISYRFQGKKEADQPWIVVN TSTLFDELELDPPEIEPGVLKVLRTEKQYLGVYIWNMRGSDGTSTYATFLV TWKGDEKTRNPTPAVTPQPRGAEFHMWNYHSHVFSVGDTFSLAMHLQY KIHEAPFDLLLEWLYVPIDPTCQPMRLYSTCLYHPNAPQCLSHMNSGCTF TSPHLAQRVASTVYQNCEHADNYTAYCLGISHMEPSFGLILHDGGTTLKF VDTPESLSGLYVFVVYFNGHVEAVAYTVVSTVDHFVNAIEERGFPPTAGQ PPATTKPKEITPVNPGTSPLIRYAAWTGGLAAVVLLCLVIFLICTAKRMRVK AYRVDKSPYNQSMYYAGLPVDDFEDSESTDTEEEFGNAIGGSHGGSSYT VYIDKTRHHHHHH |

| SEQ ID NO. | Description | Sequence information |
|---|---|---|
| 55 | P2-5G-gE | DVQLQESGGGLVQPGGSLRLSCAASGFTLSRSTMTWGRQAPGKGLEWV SDIYRSGSTTYADSVKGRFTISRDDAKNTVYLQMNSLKPEDTAVYYCAVV LPGREYRGQGTQVTVSSMGTVNKPVVGVLMGFGIITGTLRITNPVRASVL RYDDFHIDEDKLDTNSVYEPYYHSDHAESSWVNRGESSRKAYDHNSPYI WPRNDYDGFLENAHEHHGVYNQGRGIDSGERLMQPTQMSAQEDLGDD TGIHVIPTLNGDDRHKIVNVDQRQYGDVFKGDLNPKPQGQRLIEVSVEEN HPFTLRAPIQRIYGVRYTETWSFLPSLTCTGDAAPAIQHICLKHTTCFQDV VVDVDCAENTKEDQLAEISYRFQGKKEADQPWIVVNTSTLFDELELDPP EIEPGVLKVLRTEKQYLGVYIWNMRGSDGTSTYATFLVTWKGDEKTRNP TPAVTPQPRGAEFHMWNYHSHVFSVGDTFSLAMHLQYKIHEAPFDLLLE WLYVPIDPTCQPMRLYSTCLYHPNAPQCLSHMNSGCTFTSPHLAQRVAST VYQNCEHADNYTAYCLGISHMEPSFGLILHDGGTTLKFVDTPESLSGLYV FVVYFNGHVEAVAYTVVSTVDHFVNAIEERGFPPTAGQPPATTKPKEITPV NPGTSPLIRYAAWTGGLAAVVLLCLVIFLICTAKRMRVKAYRVDKSPYNQ SMYYAGLPVDDFEDSESTDTEEEFGNAIGGSHGGSSYTVYIDKTRHHHH HH |
| 56 | P2-7D-gE | DVQLQESGGGLVQPGGSLRLSCAASRFTLDYYAIGWFRQAPGKEREEVS CISSRAGSTNYQDSVKGRFTISRDNAKNTVYLQMNSLKPEDTAVYYCAA AGWAGNACPPNRYEYDYWGQGTQVTVSSMGTVNKPVVGVLMGFGIIT GTLRITNPVRASVLRYDDFHIDEDKLDTNSVYEPYYHSDHAESSWVNRG ESSRKAYDHNSPYIWPRNDYDGFLENAHEHHGVYNQGRGIDSGERLMQP TQMSAQEDLGDDTGIHVIPTLNGDDRHKIVNVDQRQYGDVFKGDLNPKP QGQRLIEVSVEENHPFTLRAPIQRIYGVRYTETWSFLPSLTCTGDAAPAIQ HICLKHTTCFQDVVVDVDCAENTKEDQLAEISYRFQGKKEADQPWIVVN TSTLFDELELDPPEIEPGVLKVLRTEKQYLGVYIWNMRGSDGTSTYATFLV TWKGDEKTRNPTPAVTPQPRGAEFHMWNYHSHVFSVGDTFSLAMHLQY KIHEAPFDLLLEWLYVPIDPTCQPMRLYSTCLYHPNAPQCLSHMNSGCTF TSPHLAQRVASTVYQNCEHADNYTAYCLGISHMEPSFGLILHDGGTTLKF VDTPESLSGLYVFVVYFNGHVEAVAYTVVSTVDHFVNAIEERGFPPTAGQ PPATTKPKEITPVNPGTSPLIRYAAWTGGLAAVVLLCLVIFLICTAKRMRVK AYRVDKSPYNQSMYYAGLPVDDFEDSESTDTEEEFGNAIGGSHGGSSYT VYIDKTRHHHHHH |
| 57 | P1-5B CDR1 | TFTLDYYA |

EP 4 628 095 A1

| SEQ ID NO. | Description | Sequence information |
|---|---|---|
| 58 | P1-5B CDR2 | ISSRGGST |
| 59 | P1-5B CDR3 | AAAGWAGYGCPPNRYEYDY |
| 60 | P2-5C CDR1 | GFTLDYYA |
| 61 | P2-5C CDR2 | ISSRGGST |
| 62 | P2-5C CDR3 | AAAGWAGFGCPPNRYEYDY |
| 63 | H1-RBD | MGWSCIILFLVATATGVHSEVQLVESGGGLVQPGGSLRLSCSASTFTLDYY AIGWVRQAPGKGLEYVSCISSRGGSTDYADSVKGRFTISRDNSKNTLYLQ MSSLRAEDTAVYYCAAAGWAGYGCPPNRYEYDYWGQGTLVTVSSRVQP TESIVRFPNITNLCPFGEVFNATRFASVYAWNRKRISNCVADYSVLYNSAS FSTFKCYGVSPTKLNDLCFTNVYADSFVIRGDEVRQIAPGQTGTIADYNY KLPDDFTGCVIAWNSNNLDSKVGGNYNYLYRLFRKSNLKPFERDISTEIY QAGSTPCNGVKGFNCYFPLQSYGFQPTYGVGYQPYRVVVLSFELLHAPA TVCGPKKSTNLVKNKCVNFHHHHHH* |
| 64 | H2-RBD | MGWSCIILFLVATATGVHSEVQLLESGGGLVQPGGSLRLSCAASTFTLDYY AIGWVRQAPGKGLEWVSCISSRGGSTDYADSVKGRFTISRDNSKNTLYLQ MNSLRAEDTAVYYCAAAGWAGYGCPPNRYEYDYWGQGTLVTVSSRVQP TESIVRFPNITNLCPFGEVFNATRFASVYAWNRKRISNCVADYSVLYNSAS FSTFKCYGVSPTKLNDLCFTNVYADSFVIRGDEVRQIAPGQTGTIADYNY KLPDDFTGCVIAWNSNNLDSKVGGNYNYLYRLFRKSNLKPFERDISTEIY QAGSTPCNGVKGFNCYFPLQSYGFQPTYGVGYQPYRVVVLSFELLHAPA TVCGPKKSTNLVKNKCVNFHHHHHH* |
| 65 | H1-RBD CDR1 | TFTLDYYAIG |
| 66 | H1-RBD CDR2 | CISSRGGSTD |
| 67 | H1-RBD CDR3 | AAAGWAGYGCPPNRYEY |
| 68 | H1 | EVQLVESGGGLVQPGGSLRLSCSASTFTLDYYAIGWVRQAPGKGLEYVS CISSRGGSTDYADSVKGRFTISRDNSKNTLYLQMSSLRAEDTAVYYCAAA GWAGYGCPPNRYEYDYWGQGTLVTVSS |

EP 4 628 095 A1

| SEQ ID NO. | Description | Sequence information |
|---|---|---|
| 69 | H2 | EVQLLESGGGLVQPGGSLRLSCAASTFTLDYYAIGWVRQAPGKGLEWVS CISSRGGSTDYADSVKGRFTISRDNSKNTLYLQMNSLRAEDTAVYYCAAA GWAGYGCPPNRYEYDYWGQGTLVTVSS |
| 70 | HBV-SHBs | MENTTSGFLGPLLGLQAGFFLLTRILTIPQSLDLWWTSLNFLGGAPKCPGL NSQSPTSNHSPTSCPPICPGYRSMCLRRFIIFLFILLLCLIFLLVLLDYQGML PVCPLLPGTPTTSTGPCKTCTSPAQGNSTFPSCCCTKPSDGNCICIPIPSSWA FARFLWEWASVRFSWLSLLVPFVQWFVGLSPIVWLSVIWMMWYWGRSL YNILSPFLPLLPIFFCLWVYI |
| 71 | HPV 58VLP-L1(human papillomavirus 58 type L1 capsid protein sequence) | MSVWRPSEATVYLPPVPVSKVVSTDEYVSRTSIYYYAGSSRLLAVGNPYF SIKSPNNNKKVLVPKVSGLQYRVFRVRLPDPNKFGFPDTSFYNPDTQRLV WACVGLEIGRGQPLGVGVSGHPYLNKFDDTETSNRYPAQPGSDNRECLS MDYKQTQLCLIGCKPPTGEHWGKGVACNNNAAATDCPPLELFNSIIEDG DMVDTGFGCMDFGTLQANKSDVPIDICNSTCKYPDYLKMASEPYGDSLF FFLRREQMFVRHFFNRAGKLGEAVPDDLYIKGSGNTAVIQSSAFFPTPSGSI VTSESQLFNKPYWLQRAQGHNNGICWGNQLFVTVVDTTRSTNMTLCTE VTKEGTYKNDNFKEYVRHVEEYDLQFVFQLCKITLTAEIMTYIHTMDSNI LEDWQFGLTPPPSASLQDTYRFVTSQAITCQKTAPPKEKEDPLNKYTFWE VNLKEKFSADLDQFPLGRKFLLQSGLKAKPRLKRSAPTTRAPSTKRKKV KK |
| 72 | P1-1B-RBD amino acid sequence | DVQLQESGGGLVQPGGSLRLSCAASGFTLSRSTMTWGRQAPGKGLEWV SDIYSSGSTTYADSVKGRFTISRDNAKNTVYLQMNSLKPEDTAVYYCAVV LPGREYRGQGTQVTVSSGGGGSGGGGSGGGGSRVQPTESIVRFPNITNLC PFGEVFNATRFASVYAWNRKRISNCVADYSVLYNSASFSTFKCYGVSPTK LNDLCFTNVYADSFVIRGDEVRQIAPGQTGKIADYNYKLPDDFTGCVIAW NSNNLDSKVGGNYNYLYRLFRKSNLKPFERDISTEIYQAGSTPCNGVEGF NCYFPLQSYGFQPTNGVGYQPYRVVVLSFELLHAPATVCGPKKSTNLVK NKCVNFHHHHHHHH* |

EP 4 628 095 A1

32

| SEQ ID NO. | Description | Sequence information |
|---|---|---|
| 73 | P2-2C-RBD amino acid sequence | DVQLQESGGGLVQPGGSLRLSCAASGFTLDYYAIGWFRQAPGKEREGVS CISGRSDSTNYADSVKGRFTISRDNAKNAVYLQMNSLKPEDTAVYYCAVS GWAGNGCPPIYEYDYWGQGTQVTVSSGGGGSGGGGSGGGGSSRVQPTE SIVRFPNITNLCPFGEVFNATRFASVYAWNRKRISNCVADYSVLYNSASFST FKCYGVSPTKLNDLCFTNVYADSFVIRGDEVRQIAPGQTGKIADYNYKLP DDFTGCVIAWNSNNLDSKVGGNYNYLYRLFRKSNLKPFERDISTEIYQAG STPCNGVEGFNCYFPLQSYGFQPTNGVGYQPYRVVVLSFELLHAPATVCG PKKSTNLVKNKCVNFHHHHHHHH |
| 74 | P1-5B-RBD amino acid sequence | DVQLQESGGGLVQPGGSLRLSCAASTFTLDYYAIGWFRQAPGKEREGVS CISSRGGSTDYADSVKGRFTISRDNAKNTVYLQMSSLQPEDTAVYYCAAA GWAGYGCPPNRYEYDYWGQGTQVTVSSGGGGSGGGGSGGGGSSRVQP TESIVRFPNITNLCPFGEVFNATRFASVYAWNRKRISNCVADYSVLYNSAS FSTFKCYGVSPTKLNDLCFTNVYADSFVIRGDEVRQIAPGQTGKIADYNY KLPDDFTGCVIAWNSNNLDSKVGGNYNYLYRLFRKSNLKPFERDISTEIY QAGSTPCNGVEGFNCYFPLQSYGFQPTNGVGYQPYRVVVLSFELLHAPAT VCGPKKSTNLVKNKCVNFHHHHHHHH |
| 75 | P2-3E-RBD amino acid sequence | DVQLQESGGGLVQPGGSLRLSCAASGFTLDYYAIGWFRQAPGEVREEVS CISGRGDSTNYAHSMKGRFTISRDNAKNTVYLQINSLKPEDTAVYYCAAA GWAGYECPANRYEYDYWGQGTQVTVSSGGGGSGGGGSGGGGSSRVQP TESIVRFPNITNLCPFGEVFNATRFASVYAWNRKRISNCVADYSVLYNSAS FSTFKCYGVSPTKLNDLCFTNVYADSFVIRGDEVRQIAPGQTGKIADYNY KLPDDFTGCVIAWNSNNLDSKVGGNYNYLYRLFRKSNLKPFERDISTEIY QAGSTPCNGVEGFNCYFPLQSYGFQPTNGVGYQPYRVVVLSFELLHAPAT VCGPKKSTNLVKNKCVNFHHHHHHHH |
| 76 | P2-3A-RBD amino acid sequence | DVQLQESGGGLVQPGGSLRLSCAASGFTLDYYAIGWFRQAPGKEREEVS CISSRGGSTNYPDSVKGRFTISRDNAKNTVYLQMNSLRPEDTAVYYCAAA GWAGFGCPPNRYEYDYWGQGTQVTVSSGGGGSGGGGSGGGGSSRVQPT ESIVRFPNITNLCPFGEVFNATRFASVYAWNRKRISNCVADYSVLYNSASFS TFKCYGVSPTKLNDLCFTNVYADSFVIRGDEVRQIAPGQTGKIADYNYKL PDDFTGCVIAWNSNNLDSKVGGNYNYLYRLFRKSNLKPFERDISTEIYQA GSTPCNGVEGFNCYFPLQSYGFQPTNGVGYQPYRVVVLSFELLHAPATVC GPKKSTNLVKNKCVNFHHHHHHHH |

EP 4 628 095 A1

| SEQ ID NO. | Description | Sequence information |
|---|---|---|
| 77 | P2-4E-RBD amino acid sequence | DVQLQESGGGLVQPGGSLRLSCAASGFTLDYYAIGWFRQAPGKEREEVS CISGRGSSTNYADSVKGRFTVSRDNAKNTVYLQINSLKPEDTAVYYCAAA GWAGYGCPANRYEYDYWGQGTQVTVSSGGGGSGGGGSGGGGSSRVQP TESIVRFPNITNLCPFGEVFNATRFASVYAWNRKRISNCVADYSVLYNSAS FSTFKCYGVSPTKLNDLCFTNVYADSFVIRGDEVRQIAPGQTGKIADYNY KLPDDFTGCVIAWNSNNLDSKVGGNYNYLYRLFRKSNLKPFERDISTEIY QAGSTPCNGVEGFNCYFPLQSYGFQPTNGVGYQPYRVVVLSFELLHAPAT VCGPKKSTNLVKNKCVNFHHHHHHHH |
| 78 | P2-5C-RBD amino acid sequence | DVQLQESGGGLVQPGGSLRLSCAASGFTLDYYAIGWFRQAPGKEREGVS CISSRGGSTNTPDSVKGRFTISRDNAKNTVYLQMNSLRPEDTAVYYCAAA GWAGFGCPPNRYEYDYWGQGTQVTVSSGGGGSGGGGSGGGGSSRVQPT ESIVRFPNITNLCPFGEVFNATRFASVYAWNRKRISNCVADYSVLYNSASFS TFKCYGVSPTKLNDLCFTNVYADSFVIRGDEVRQIAPGQTGKIADYNYKL PDDFTGCVIAWNSNNLDSKVGGNYNYLYRLFRKSNLKPFERDISTEIYQA GSTPCNGVEGFNCYFPLQSYGFQPTNGVGYQPYRVVVLSFELLHAPATVC GPKKSTNLVKNKCVNFHHHHHHHH |
| 79 | P2-5G-RBD amino acid sequence | DVQLQESGGGLVQPGGSLRLSCAASGFTLSRSTMTWGRQAPGKGLEWV SDIYRSGSTTYADSVKGRFTISRDDAKNTVYLQMNSLKPEDTAVYYCAVV LPGREYRGQGTQVTVSSGGGGSGGGGSGGGGSSRVQPTESIVRFPNITNL CPFGEVFNATRFASVYAWNRKRISNCVADYSVLYNSASFSTFKCYGVSPT KLNDLCFTNVYADSFVIRGDEVRQIAPGQTGKIADYNYKLPDDFTGCVIA WNSNNLDSKVGGNYNYLYRLFRKSNLKPFERDISTEIYQAGSTPCNGVE GFNCYFPLQSYGFQPTNGVGYQPYRVVVLSFELLHAPATVCGPKKSTNLV KNKCVNFHHHHHHHH |

34

| SEQ ID NO. | Description | Sequence information |
|---|---|---|
| 80 | P1-1B-BGTSTIP amino acid sequence | DVQLQESGGGLVQPGGSLRLSCAASGFTLSRSTMTWGRQAPGKGLEWV SDIYSSGSTTYADSVKGRFTISRDNAKNTVYLQMNSLKPEDTAVYYCAVV LPGREYRGQGTQVTVSSGGGGSGGGGSGGGGSAENLAVGIGAVFLGFLG AAGSTMGAASMTLTVQARNLLSGIVQQQSNLLRAPEAQQHLLKLTVWGI KQLQARVLAVERYLRDQQLLGIWGCSGKLICTTNVPNLWVTVYYGVPV WKDAETTLFCASDAKAYETEKHNVWATHACVPTDPNPQEIHLENVTEEF NMWKNNMVEQMHTDIISLWDQSLKPCVKLTPLCVTLQCTNVTNNITDD MRGELKNCSFNMTTELRDKKQKVYSLFYRLDVVQINENQGNRSNNSNK EYRLINCNTSAITQACPKVSFEPIPIHYCAPAGFAILKCKDKKFNGTGPCPS VSTVQCTHGIKPVVSTQLLLNGSLAEEEVMIRSENITNNAKNILVQFNTPV QINCTRPNNNTRKSIRIGPGQAFYATGDIIGDIRQAHCNVSKATWNETLGK VVKQLRKHFGNNTIIRFANSSGGDLEVTTHSFNCGGEFFYCNTSGLFNST WISNTSVQGSNSTGSNDSITLPCRIKQIINMWQRIGQAMYAPPIQGVIRCV SNITGLILTRDGGSTNSTTETFRPGGGDMRDNWRSELYKYKVVKIEPLGV APTRRNLSEIWDNMTWLQWDKEISNYTQIIYGLLEESQNQQEKNEQDLL ALDHHHHHHHH* |
| 81 | P1-5B-BGTSTIP amino acid sequence | DVQLQESGGGLVQPGGSLRLSCAASTFTLDYYAIGWFRQAPGKEREGVS CISSRGGSTDYADSVKGRFTISRDNAKNTVYLQMSSLQPEDTAVYYCAAA GWAGYGCPPNRYEYDYWGQGTQVTVSSGGGGSGGGGSGGGGSAENLA VGIGAVFLGFLGAAGSTMGAASMTLTVQARNLLSGIVQQQSNLLRAPEA QQHLLKLTVWGIKQLQARVLAVERYLRDQQLLGIWGCSGKLICTTNVPN LWVTVYYGVPVWKDAETTLFCASDAKAYETEKHNVWATHACVPTDPNP QEIHLENVTEEFNMWKNNMVEQMHTDIISLWDQSLKPCVKLTPLCVTLQ CTNVTNNITDDMRGELKNCSFNMTTELRDKKQKVYSLFYRLDVVQINEN QGNRSNNSNKEYRLINCNTSAITQACPKVSFEPIPIHYCAPAGFAILKCKD KKFNGTGPCPSVSTVQCTHGIKPVVSTQLLLNGSLAEEEVMIRSENITNN AKNILVQFNTPVQINCTRPNNNTRKSIRIGPGQAFYATGDIIGDIRQAHCNV SKATWNETLGKVVKQLRKHFGNNTIIRFANSSGGDLEVTTHSFNCGGEFF YCNTSGLFNSTWISNTSVQGSNSTGSNDSITLPCRIKQIINMWQRIGQAMY APPIQGVIRCVSNITGLILTRDGGSTNSTTETFRPGGGDMRDNWRSELYKY KVVKIEPLGVAPTRRNLSEIWDNMTWLQWDKEISNYTQIIYGLLEESQNQ QEKNEQDLLALDHHHHHHHH* |

EP 4 628 095 A1

| SEQ ID NO. | Description | Sequence information |
|---|---|---|
| 82 | P2-2C-BGTSTIP amino acid sequence | DVQLQESGGGLVQPGGSLRLSCAASGFTLDYYAIGWFRQAPGKEREGVS CISGRSDSTNYADSVKGRFTISRDNAKNAVYLQMNSLKPEDTAVYYCAVS GWAGNGCPPIYEYDYWGQGTQVTVSSGGGGSGGGGSGGGGSAENLAVG IGAVFLGFLGAAGSTMGAASMTLTVQARNLLSGIVQQQSNLLRAPEAQQ HLLKLTVWGIKQLQARVLAVERYLRDQQLLGIWGCSGKLICTTNVPNLW VTVYYGVPVWKDAETTLFCASDAKAYETEKHNVWATHACVPTDPNPQE IHLENVTEEFNMWKNNMVEQMHTDIISLWDQSLKPCVKLTPLCVTLQCT NVTNNITDDMRGELKNCSFNMTTELRDKKQKVYSLFYRLDVVQINENQ GNRSNNSNKEYRLINCNTSAITQACPKVSFEPIPIHYCAPAGFAILKCKDK KFNGTGPCPSVSTVQCTHGIKPVVSTQLLLNGSLAEEEVMIRSENITNNA KNILVQFNTPVQINCTRPNNNTRKSIRIGPGQAFYATGDIIGDIRQAHCNVS KATWNETLGKVVKQLRKHFGNNTIIRFANSSGGDLEVTTHSFNCGGEFFY CNTSGLFNSTWISNTSVQGSNSTGSNDSITLPCRIKQIINMWQRIGQAMYA PPIQGVIRCVSNITGLILTRDGGSTNSTTETFRPGGGDMRDNWRSELYKYK VVKIEPLGVAPTRRNLSEIWDNMTWLQWDKEISNYTQIIYGLLEESQNQQ EKNEQDLLALDHHHHHHHH* |
| 83 | P2-5C-BGTSTIP amino acid sequence | DVQLQESGGGLVQPGGSLRLSCAASGFTLDYYAIGWFRQAPGKEREGVS CISSRGGSTNTPDSVKGRFTISRDNAKNTVYLQMNSLRPEDTAVYYCAAA GWAGFGCPPNRYEYDYWGQGTQVTVSSGGGGSGGGGSGGGGSAENLA VGIGAVFLGFLGAAGSTMGAASMTLTVQARNLLSGIVQQQSNLLRAPEA QQHLLKLTVWGIKQLQARVLAVERYLRDQQLLGIWGCSGKLICTTNVPN LWVTVYYGVPVWKDAETTLFCASDAKAYETEKHNVWATHACVPTDPNP QEIHLENVTEEFNMWKNNMVEQMHTDIISLWDQSLKPCVKLTPLCVTLQ CTNVTNNITDDMRGELKNCSFNMTTELRDKKQKVYSLFYRLDVVQINEN QGNRSNNSNKEYRLINCNTSAITQACPKVSFEPIPIHYCAPAGFAILKCKD KKFNGTGPCPSVSTVQCTHGIKPVVSTQLLLNGSLAEEEVMIRSENITNN AKNILVQFNTPVQINCTRPNNNTRKSIRIGPGQAFYATGDIIGDIRQAHCNV SKATWNETLGKVVKQLRKHFGNNTIIRFANSSGGDLEVTTHSFNCGGEFF YCNTSGLFNSTWISNTSVQGSNSTGSNDSITLPCRIKQIINMWQRIGQAMY APPIQGVIRCVSNITGLILTRDGGSTNSTTETFRPGGGDMRDNWRSELYKY KVVKIEPLGVAPTRRNLSEIWDNMTWLQWDKEISNYTQIIYGLLEESQNQ QEKNEQDLLALDHHHHHHHH* |

EP 4 628 095 A1

| SEQ ID NO. | Description | Sequence information |
|---|---|---|
| 84 | P2-3A-BGTSTSIP amino acid sequence | DVQLQESGGGLVQPGGSLRLSCAASGFTLDYYAIGWFRQAPGKEREEVS CISSRGGSTNYPDSVKGRFTISRDNAKNTVYLQMNSLRPEDTAVYYCAAA GWAGFGCPPNRYEYDYWGQGTQVTVSSGGGGSGGGGSGGGGSAENLA VGIGAVFLGFLGAAGSTMGAASMTLTVQARNLLSGIVQQQSNLLRAPEA QQHLLKLTVWGIKQLQARVLAVERYLRDQQLLGIWGCSGKLICTTNVPN LWVTVYYGVPVWKDAETTLFCASDAKAYETEKHNVWATHACVPTDPNP QEIHLENVTEEFNMWKNNMVEQMHTDIISLWDQSLKPCVKLTPLCVTLQ CTNVTNNITDDMRGELKNCSFNMTTELRDKKQKVYSLFYRLDVVQINEN QGNRSNNSNKEYRLINCNTSAITQACPKVSFEPIPIHYCAPAGFAILKCKD KKFNGTGPCPSVSTVQCTHGIKPVVSTQLLLNGSLAEEEVMIRSENITNN AKNILVQFNTPVQINCTRPNNNTRKSIRIGPGQAFYATGDIIGDIRQAHCNV SKATWNETLGKVVKQLRKHFGNNTIIRFANSSGGDLEVTTHSFNCGGEFF YCNTSGLFNSTWISNTSVQGSNSTGSNDSITLPCRIKQIINMWQRIGQAMY APPIQGVIRCVSNITGLILTRDGGSTNSTTETFRPGGGDMRDNWRSELYKY KVVKIEPLGVAPTRRNLSEIWDNMTWLQWDKEISNYTQIIYGLLEESQNQ QEKNEQDLLALDHHHHHHH* |
| 85 | P2-3E-BGTSTIP amino acid sequence | DVQLQESGGGLVQPGGSLRLSCAASGFTLDYYAIGWFRQAPGEVREEVS CISGRGDSTNYAHSMKGRFTISRDNAKNTVYLQINSLKPEDTAVYYCAAA GWAGYECPANRYEYDYWGQGTQVTVSSGGGGSGGGGSGGGGSAENLA VGIGAVFLGFLGAAGSTMGAASMTLTVQARNLLSGIVQQQSNLLRAPEA QQHLLKLTVWGIKQLQARVLAVERYLRDQQLLGIWGCSGKLICTTNVPN LWVTVYYGVPVWKDAETTLFCASDAKAYETEKHNVWATHACVPTDPNP QEIHLENVTEEFNMWKNNMVEQMHTDIISLWDQSLKPCVKLTPLCVTLQ CTNVTNNITDDMRGELKNCSFNMTTELRDKKQKVYSLFYRLDVVQINEN QGNRSNNSNKEYRLINCNTSAITQACPKVSFEPIPIHYCAPAGFAILKCKD KKFNGTGPCPSVSTVQCTHGIKPVVSTQLLLNGSLAEEEVMIRSENITNN AKNILVQFNTPVQINCTRPNNNTRKSIRIGPGQAFYATGDIIGDIRQAHCNV SKATWNETLGKVVKQLRKHFGNNTIIRFANSSGGDLEVTTHSFNCGGEFF YCNTSGLFNSTWISNTSVQGSNSTGSNDSITLPCRIKQIINMWQRIGQAMY APPIQGVIRCVSNITGLILTRDGGSTNSTTETFRPGGGDMRDNWRSELYKY KVVKIEPLGVAPTRRNLSEIWDNMTWLQWDKEISNYTQIIYGLLEESQNQ QEKNEQDLLALDHHHHHHH* |

| SE Q ID NO. | Description | Sequence information |
|---|---|---|
| 86 | P2-4G-BGTSTIP amino acid sequence | DVQLQESGGGLVQPGGSTRLSCAASGFTLDYYAIGWFRQAPGKEREEVS CMSSRGGSTNYVDSVQGRFTLSRDNAKNTVYLQMNSLKPEDTAVYYCA AAGWAGNGCPPNRYEYDYWGQGTQVTVSSGGGGSGGGGSGGGGSAEN LAVGIGAVFLGFLGAAGSTMGAASMTLTVQARNLLSGIVQQQSNLLRAP EAQQHLLKLTVWGIKQLQARVLAVERYLRDQQLLGIWGCSGKLICTTNV PNLWVTVYYGVPVWKDAETTLFCASDAKAYETEKHNVWATHACVPTDP NPQEIHLENVTEEFNMWKNNMVEQMHTDIISLWDQSLKPCVKLTPLCVT LQCTNVTNNITDDMRGELKNCSFNMTTELRDKKQKVYSLFYRLDVVQIN ENQGNRSNNSNKEYRLINCNTSAITQACPKVSFEPIPIHYCAPAGFAILKCK DKKFNGTGPCPSVSTVQCTHGIKPVVSTQLLLNGSLAEEEVMIRSENITN NAKNILVQFNTPVQINCTRPNNNTRKSIRIGPGQAFYATGDIIGDIRQAHCN VSKATWNETLGKVVKQLRKHFGNNTIIRFANSSGGDLEVTTHSFNCGGEF FYCNTSGLFNSTWISNTSVQGSNSTGSNDSITLPCRIKQIINMWQRIGQAM YAPPIQGVIRCVSNITGLILTRDGGSTNSTTETFRPGGGDMRDNWRSELYK YKVVKIEPLGVAPTRRNLSEIWDNMTWLQWDKEISNYTQIIYGLLEESQN QQEKNEQDLLALDHHHHHHHH* |
| 87 | P2-7D-BGTSTIP amino acid sequence | DVQLQESGGGLVQPGGSLRLSCAASRFTLDYYAIGWFRQAPGKEREEVS CISSRAGSTNYQDSVKGRFTISRDNAKNTVYLQMNSLKPEDTAVYYCAA AGWAGNACPPNRYEYDYWGQGTQVTVSSGGGGSGGGGSGGGGSAENL AVGIGAVFLGFLGAAGSTMGAASMTLTVQARNLLSGIVQQQSNLLRAPE AQQHLLKLTVWGIKQLQARVLAVERYLRDQQLLGIWGCSGKLICTTNVP NLWVTVYYGVPVWKDAETTLFCASDAKAYETEKHNVWATHACVPTDP NPQEIHLENVTEEFNMWKNNMVEQMHTDIISLWDQSLKPCVKLTPLCVT LQCTNVTNNITDDMRGELKNCSFNMTTELRDKKQKVYSLFYRLDVVQIN ENQGNRSNNSNKEYRLINCNTSAITQACPKVSFEPIPIHYCAPAGFAILKCK DKKFNGTGPCPSVSTVQCTHGIKPVVSTQLLLNGSLAEEEVMIRSENITN NAKNILVQFNTPVQINCTRPNNNTRKSIRIGPGQAFYATGDIIGDIRQAHCN VSKATWNETLGKVVKQLRKHFGNNTIIRFANSSGGDLEVTTHSFNCGGEF FYCNTSGLFNSTWISNTSVQGSNSTGSNDSITLPCRIKQIINMWQRIGQAM YAPPIQGVIRCVSNITGLILTRDGGSTNSTTETFRPGGGDMRDNWRSELYK YKVVKIEPLGVAPTRRNLSEIWDNMTWLQWDKEISNYTQIIYGLLEESQN QQEKNEQDLLALDHHHHHHHH* |

EP 4 628 095 A1

| SE Q ID NO. | Description | Sequence information |
|---|---|---|
| 88 | P1F8 amino acid sequence | DVQLQESGGGLVKPGGSLTLSCAASRSIFTINTMGWYRQAPGKQRELVAD ITNGGGRNYADSVKGRFTIGRDNAKNTVYLTMNSLKPEDTAVYYCNARV LVWPSPPDDYWGQGTQVTVSSGR |
| 89 | P1F8-BGTSTIP amino acid sequence | MGWSCIILFLVATATGVHSDVQLQESGGGLVKPGGSLTLSCAASRSIFTINT MGWYRQAPGKQRELVADITNGGGRNYADSVKGRFTIGRDNAKNTVYLT MNSLKPEDTAVYYCNARVLVWPSPPDDYWGQGTQVTVSSGRGGGGSGG GGSGGGGSAENLAVGIGAVFLGFLGAAGSTMGAASMTLTVQARNLLSGI VQQQSNLLRAPEAQQHLLKLTVWGIKQLQARVLAVERYLRDQQLLGIWG CSGKLICTTNVPNLWVTVYYGVPVWKDAETTLFCASDAKAYETEKHNV WATHACVPTDPNPQEIHLENVTEEFNMWKNNMVEQMHTDIISLWDQSLK PCVKLTPLCVTLQCTNVTNNITDDMRGELKNCSFNMTTELRDKKQKVYS LFYRLDVVQINENQGNRSNNSNKEYRLINCNTSAITQACPKVSFEPIPIHY CAPAGFAILKCKDKKFNGTGPCPSVSTVQCTHGIKPVVSTQLLLNGSLAE EEVMIRSENITNNAKNILVQFNTPVQINCTRPNNNTRKSIRIGPGQAFYATG DIIGDIRQAHCNVSKATWNETLGKVVKQLRKHFGNNTIIRFANSSGGDLE VTTHSFNCGGEFFYCNTSGLFNSTWISNTSVQGSNSTGSNDSITLPCRIKQI INMWQRIGQAMYAPPIQGVIRCVSNITGLILTRDGGSTNSTTETFRPGGGD MRDNWRSELYKYKVVKIEPLGVAPTRRNLSEIWDNMTWLQWDKEISNY TQIIYGLLEESQNQQEKNEQDLLALDHHHHHHH |
| 90 | P1-1B CDR1 | GFTLSRST |
| 91 | P1-1B CDR2 | IYSSGST |
| 92 | P1-1B CDR3 | AVVLPGREYRGQ |
| 93 | P1-1G CDR1 | GFSLDYYA |
| 94 | P1-1G CDR2 | VSGREGST |
| 95 | P1-1G CDR3 | AAAGWNGLGCPPSLYEYDY |
| 96 | P1-1H CDR1 | GFTLDEYT |
| 97 | P1-1H CDR2 | ISSRGGRT |
| 98 | P1-1H CDR3 | AAASYYDDCRVPAWYDY |
| 99 | P1-5-E CDR1 | GFTLDYYT |

(continued)

| SE Q ID NO. | Description | Sequence information |
|---|---|---|
| 100 | P1-5-E CDR2 | ISSSDDST |
| 101 | P1-5-E CDR3 | AAVPRHTGISPVLAMCASSVWYDY |
| 102 | P1-11B CDR1 | GFTLDYYA |
| 103 | P1-11B CDR2 | LSGRNGSP |
| 104 | P1-11B CDR3 | AAAGWNGLGCPPSLYEYDY |
| 105 | P2-2C CDR1 | GFTLDYYA |
| 106 | P2-2C CDR2 | ISGRSDST |
| 107 | P2-2C CDR3 | AVSGWAGNGCPPIYEYDY |
| 108 | P2-3A CDR1 | GFTLDYYA |
| 109 | P2-3A CDR2 | ISSRGGST |
| 110 | P2-3A CDR3 | AAAGWAGFGCPPNRYEYDY |
| 111 | P2-3D CDR1 | GFTLDYYA |
| 112 | P2-3D CDR2 | ISGRGGST |
| 113 | P2-3D CDR3 | AAAGWAGNVCPPNRYEYDY |
| 114 | P2-3E CDR1 | GFTLDYYA |
| 115 | P2-3E CDR2 | ISGRGDST |
| 116 | P2-3E CDR3 | AAAGWAGNVCPPNRYEYDY |
| 117 | P2-4E CDR1 | GFTLDYYA |
| 118 | P2-4E CDR2 | ISGRGSST |
| 119 | P2-4E CDR3 | AAAGWAGYGCPANRYEYDY |
| 120 | P2-4G CDR1 | GFTLDYYA |
| 121 | P2-4G CDR2 | MSSRGGST |
| 122 | P2-4G CDR3 | AAAGWAGNGCPPNRYEYDY |
| 123 | P2-5G CDR1 | GFTLSRST |
| 124 | P2-5G CDR2 | IYRSGST |
| 125 | P2-5G CDR3 | VVLPGREYRGQ |

| SEQ ID NO. | Description | Sequence information |
|---|---|---|
| 126 | P2-6D CDR1 | TFTLDYYA |
| 127 | P2-6D CDR2 | ISSRSGST |
| 128 | P2-6D CDR3 | AAAGWAGYGCPANRYEYDY |
| 129 | P2-7C CDR1 | GFTLDHYA |
| 130 | P2-7C CDR2 | ISSRRGST |
| 131 | P2-7C CDR3 | AAAGWAGYMCPPNRYEYDY |
| 132 | P2-7D CDR1 | RFTLDYYA |
| 133 | P2-7D CDR2 | ISSRAGST |
| 134 | P2-7D CDR3 | AAAGWAGNACPPNRYEYDY |
| 135 | P2-8C CDR1 | GFTLDYYA |
| 136 | P2-8C CDR2 | ISGRRGST |
| 137 | P2-8C CDR3 | AAAGWNGLGCPPSLDEYDY |
| 138 | P2-10G CDR1 | GFTLDYYA |
| 139 | P2-10G CDR2 | VSGRHGST |
| 140 | P2-10G CDR3 | AAAGWNGLGCPPSLDEYDY |
| 141 | P2-11H CDR1 | GLTLDYYN |
| 142 | P2-11H CDR2 | ISVSGGVT |
| 143 | P2-11H CDR3 | AAVSLPFVPMLGCPEGYDY |

**Specific Models for Carrying Out the present invention**

The present invention is now described with reference to the following examples which are intended to illustrate the present invention (but not to limit the present invention).

Unless otherwise specified, the molecular biology experimental methods and immunoassays used in the present invention are basically carried out in accordance with the methods described in J. Sambrook et al., Molecular Cloning: A Laboratory Manual, 2nd edition, Cold Spring Harbor Laboratory Press, 1989, and F. M. Ausubel et al., Compiled Molecular Biology Laboratory Manual, 3rd edition, John Wiley & Sons, Inc., 1995; the use of restriction endonucleases is in accordance with the conditions recommended by the product manufacturer. Those skilled in the art will appreciate that the examples describe the present invention by way of example and are not intended to limit the scope of protection claimed by the present invention.

In addition, for those in the examples where specific conditions were not indicated, they were carried out under conventional conditions or conditions recommended by the manufacturer. The reagents or instruments used without indicating the manufacturer were all conventional products that can be obtained commercially. Those skilled in the art will appreciate that the examples describe the present invention by way of example and are not intended to limit the scope sought to be protected by the present invention. All the disclosures and other references mentioned herein are incorporated herein by reference in their entirety.

Example 1: Construction and screening process of nanobody phage library

1. RNA extraction (Trizol method)

(1) Peripheral blood lymphocytes (obtained from alpacas) preserved with Trizol were transferred to a 1.5 mL centrifuge tube, added with 1/5 volume of chloroform, and mixed;

(2) After standing at room temperature for 5 min, centrifugation was performed at 4°C, 12000g for 15 min;

(3) The supernatant after centrifugation was carefully transferred to a new centrifuge tube;

(4) An equal volume of isopropanol was added to the new centrifuge tube;

(5) After standing at room temperature for 10 min, centrifugation was performed at 4°C, 12000g for 10 min;

(6) The precipitate in each tube was washed with 1 mL of 75% ethanol, centrifuged at 7500g for 5 min to remove the ethanol, and dried, then the precipitate was dissolved in an appropriate amount of RNase-free water, and all samples were combined to obtain the total RNA extracted.

2. Reverse transcription of cDNA (Takara reverse transcription kit)

3. PCR amplification

Table 2. PCR amplification program

| Cycle | Temperature | Time |
|---|---|---|
| Step 1 | 98°C | 3min |
| Step 2 | 95°C | 50s |
| | 55°C+0.5/cycle | 30s |
| | 72°C | 45s |
| | Go to Step 2 | 22 cycles |
| Step 3 | 72°C | 3min |
| Step 4 | 4°C | forever |

4. Enzyme digestion and ligation

Table 3. Enzyme digestion and ligation procedures

| Reagent | Dosage |
|---|---|
| PCR product | 1.6μg |
| Vector (pComb3XSS) | 4μg |
| T4 ligase | 30μL |
| 10×T4 reaction Buffer | 200μL |
| ddH$_2$O | Up to 2000μL |

5. Construction of bacterial library and phage library

6. Phage screening, purification and amplification

$$V(ul) = \frac{10^{12}}{T_{library}} \times 1000$$

wherein, V was the volume of phage added (unit: μL), $T_{library}$ was the phage titer;

$$T\ (pfu/ml) = N \times D \times 400$$

wherein, T was the phage titer (unit: pfu/mL), D was the dilution factor, and N was the number of single colonies at the corresponding dilution factor.

7. Library quality verification

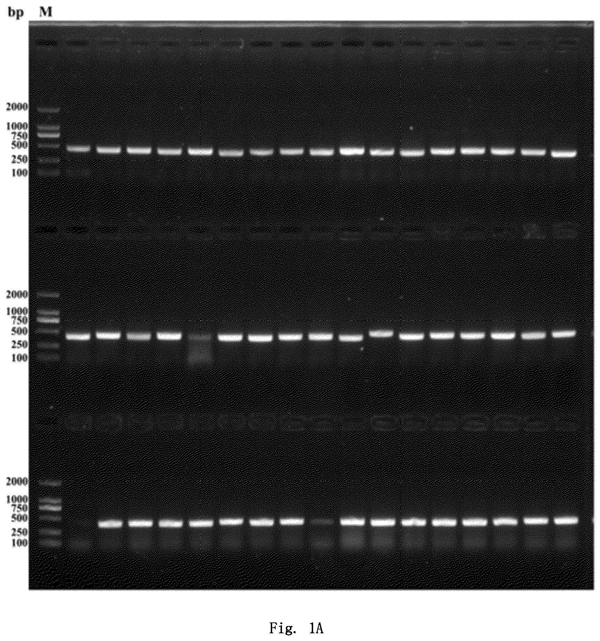

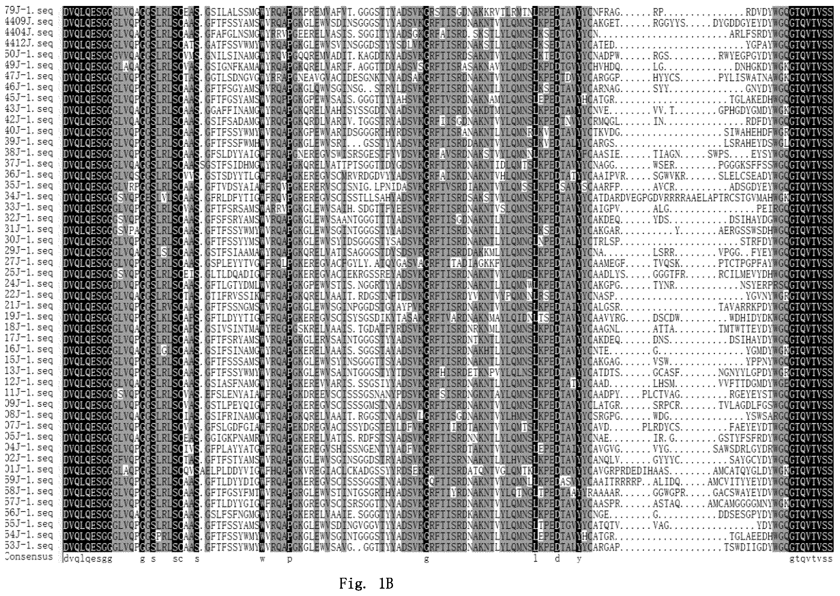

Fig. 1 showed the screening process of the nanobodies of the example of the present invention. Fig. 1A showed the results of colony PCR agarose gel electrophoresis, in which 50 of the 51 randomly selected monoclones were positive clones, the positive rate was 98%, and the positive clone rate of the bacterial library met the requirements. Fig. 1B showed the protein sequences obtained by translation with software after sequencing, in which the sequence diversity comparison showed that all 50 sequences were independent sequences with good diversity, and the diversity of the bacterial library met the requirements.

8. Monoclonal ELISA detection

After calculation according to the following formula, auxiliary phage M13K07 was added to each well so that the ratio of bacteria number: phage number = 1:20:

$$V\ (ml) = \frac{OD600 \times 1.6 \times 10^{11}}{T_{helper-phage}}$$

wherein, V was the volume of the auxiliary phage added (unit: mL), and $T_{helper-phage}$ was the titer of the auxiliary phage used.

9. Performing secondary ELISA verification on the selected positive clones

The finally selected positive clones were sent to a biological company for sequencing and analysis, and a total of 20 nanobody heavy chain variable region (VHH) sequences were obtained. The amino acid sequences of these nanobodies were set forth in SEQ ID NOs: 10-29 in Table 1, and the CDR1-3 sequences were set forth in SEQ ID NOs: 57-62 and 90-144 in Table 1.

Example 2: Preparation and expression of VHH-RBD, VHH-gE and VHH-BGTSTIP fusion proteins

1. Preparation of fusion proteins

The construction method of VHH-RBD sequences in the insect cell expression system comprised: connecting a RBD sequence (SEQ ID NOs: 1-8) to the C-terminal of a VHH sequence (SEQ ID NOs: 10-29), and then connecting the bee venom signal peptide (SEQ ID NO: 31) to the N-terminal of the above sequence to promote protein secretion and expression. The nucleotide sequences encoding the above amino acid sequences were synthesized by Sangon Biotech, and the nucleotide sequence was constructed on the PIEX/bac-1 vector through Nco I and BamH I restriction sites. Finally, 8 kinds of VHH-RBD proteins (P1-5B-RBD, P2-2C-RBD, P1-1B-RBD, P1-1G-RBD, P2-4E-RBD, P2-3E-RBD, P2-3D-RBD, P2-3A-RBD) were obtained through the insect cell expression system, and their sequences were set forth in SEQ ID NOs: 42-49 in Table 1.

The sequences of VHH-gE fusion proteins were constructed using the same method, except that the RBD sequence connected to the C-terminal of the VHH sequence was replaced with the gE sequence (SEQ ID NO: 30). Finally, 7 kinds of VHH-gE proteins (P2-10G-gE, P2-6D-gE, P2-5C-gE, P2-8C-gE, P2-1H-gE, P2-5G-gE, P2-7D-gE) were obtained through the insect cell expression system, and their sequences were set forth in SEQ ID NOs: 50-56 in Table 1.

The construction method of VHH-RBD sequences in the mammalian cell expression system comprised: sequentially connecting the $(G_4S)_3$ flexible protein linker (SEQ ID NO: 39), the RBD sequences (SEQ ID NOs: 1-8), and histidine tag (8-His tag) to the C-terminal of the VHH sequences (SEQ ID NOs: 10-29), and introducing a signal peptide sequence (SEQ ID NO: 38) at the N-terminal to promote protein secretion expression. The nucleotide sequences encoding the above amino acid sequences were codon-optimized, then synthesized by Tongyong Shengwu, and cloned between the ECORI and Xbal restriction sites of the pcDNA3.1 vector. Finally, 8 kinds of VHH-RBD proteins (P1-1B-RBD, P1-5B-RBD, P2-2C-RBD, P2-5C-RBD, P2-3A-RBD, P2-3E-RBD, P2-4E-RBD, P2-5G-RBD) were obtained through the mammalian cell expression system, and their sequences were set forth in SEQ ID NOs: 72 to 89 in Table 1.

The sequences of VHH-BGTSTIP (i.e., VHH-Env) fusion proteins were constructed by the same method, except that the connected RBD sequence was replaced with the full-length Env extracellular segment amino acid sequence (SEQ ID NO: 34 or 35). Finally, 8 kinds of VHH-BGTSTIP proteins (P1-1B-BGTSTIP, P1-5B-BGTSTIP, P2-2C-BGTSTIP, P2-5C-BGTSTIP, P2-3A BGTSTSIP, P2-3E-BGTSTIP, PA-4G-BGTSTIP, P2-7D-BGTSTIP) were obtained through the mammalian cell expression system, and their sequences were set forth in SEO ID NOs: 80 to 87 in Table 1.

2. Expression of VHH-RBD and VHH-gE in insect expression system

Transfection of insect cells

(1) It was confirmed that *sf9* cells (purchased from Invitrogen, 11496-015) or *sf21* cells (purchased from Invitrogen, 11497-013) were in the logarithmic growth phase ($1.5-2.5 \times 10^6$/mL) and the survival rate was maintained above 90%. 200 $\mu$L of ESF 921 medium (purchased from Expression systems, 96-001-01) containing 2% FBS, 0.1 $\mu$g of Baculovirus DNA (purchased from Expression systems, 91-002) and 1 $\mu$g of pAc-S plasmid were added to a 24-well plate, and mixed well. 1 $\mu$L of transfection reagent (purchased from Expression systems, 95-055-075) was added to 50 $\mu$L of ESF921 medium (purchased from Expression systems, 96-001-01) and mixed well. The two were combined in one tube, mixed well, and allowed to stand at room temperature for 30 min. The cells were washed during the standing period (this was performed near the end of the standing), comprising: after the cells were completely attached to the wall, the medium was removed by a pipette, then 300 $\mu$L of ESF921 medium was added in a quick motion to avoid dehydration of the cells; after gently shaking, the medium was removed, and 300 $\mu$L of ESF921 medium was added again. After the time was up, about 100 $\mu$L of the above mixture was evenly added dropwise to each well with the cells. Then, incubation was performed at 27°C for 6 h, the supernatant was discarded, and 500 $\mu$L of complete culture medium (50% CCM3 + 50% TNM-FH (SIGMA-ALDRICH, T1032) + 10% FBS) was supplemented.

(2) The cell supernatant obtained in step (1) was collected, centrifuged at 500 g for 5 min, the cell remains and debris were discarded, and the supernatant was stored at 4°C in the dark, and used as P1 virus seed solution.

(3) Amplification of baculovirus

It was confirmed that sf9 cells or *sf21* cells were in the logarithmic growth phase ($1.5\text{-}2.5\times10^6$/mL) and the survival rate was maintained above 90%. 8 to 10 mL of the cells with a density of $6\times10^5$/mL was coated on a 10cm plate, and allowed to stand for 15 min to allow the cells to adhere the wall. About 600 $\mu$L of the P1 virus solution was evenly added dropwise, and incubated at 27°C for 3 to 4 days. Cytopathic effects were observed. The cell supernatant was collected, centrifuged at 1000rpm for 5min to remove cell remains and debris, and filtered with a 0.22 $\mu$m filter. The supernatant was stored at 4°C in the dark, and used as P2 virus seed solution. The P2 virus titer was about $10^6$ to $10^7$ pfu/mL. P3 virus could be amplified in a manner of scaled-up by volume in a 250 mL shake flask according to this method.

Operation of insect cell protein expression

H5 cells (purchased from Invitrogen, B855-02) with a density of $2\text{x}10^6$/mL and a survival rate of more than 90% were cultured in 250 mL of ESF921 culture medium added in a 1L shake flask. The virus was added in an amount according to the corresponding MOI, the flask mouth was sealed with sealing film, and the cells were cultured in a 27°C shaker at 120 rpm. The cells in the shake flask were taken out every day for observation and counting, and relevant data were recorded. The appropriate MOI could ensure that more than 70% of the cells were diseased on the first day. On the second day, all the cells were diseased, and the survival rate was about 80%. On the third day, the cells ruptured and the survival rate dropped to 30% to 50%. At this time, it could be considered to collect the cells. The cells were collected by centrifugation at 10000 rpm, for 10 min, and then the supernatant was separated and purified.

3. Purification of VHH-RBD and VHH-gE in insect expression system

AKTA system was used for Ni affinity chromatography purification;

Instrument system: AKTA Pure type preparative liquid chromatograph;

Purification medium: Ni Sepharose 6 Fast Flow affinity medium; Buffers: pump A buffer and pump B buffer, in which generally, pump A buffer was $1\times$PBS buffer (160 g/L NaCL, 8.1 mmol/L $Na_2HPO_4$, 1.5 mmol/L $KH_2PO_4$, 2.7 mmol/L KCL, pH7.4), and pump B buffer was $1\times$PBS + 250 mmol/L imidazole buffer;

System flow rate: 5 mL/min; Detection wavelength: UV@280 nm

Elution conditions: 50 mM imidazole buffer (obtained by diluting 250 mmol/L imidazole buffer with $1\times$ PBS buffer) was used to elute the impurities, followed by washing with $1\times$ PBS, and then the target protein (S trimer protein) was eluted with 250 mM imidazole buffer;

The product eluted with 250 mM imidazole was collected to obtain 10 mL of purified sample. 50 $\mu$L of each eluted product was taken, added with 10 $\mu$L of 6X Loading Buffer, mixed well, and underwent a 80°C water bath for 10 min, then 10$\mu$l was taken and electrophoresed in a 10% SDS-polyacrylamide gel at 80 V for 120 min. Subsequently, Coomassie Brilliant Blue staining was performed to display the electrophoresis bands.

4. Transfection expression of VHH-RBD and VHH-BGTSTIP in mammalian expression system

Frozen 293F cells were taken from a -80°C refrigerator, thawed at 37°C, centrifuged at 1300rpm for 4min, the supernatant was discarded in clean bench, the cells were flicked and resuspended with 293 freestyle medium incubated at 37°C in advance, transferred to a flask containing 50 mL of incubation medium, cultured at 37°C, 5% $CO_2$ and 120 rpm, and subcultured when cell density reached $2.0\times10^6$, and the culture system was gradually expanded. When the cells were sufficient, PEI (MW 25, 000) was used to transiently transfect 293F cells, the cells were collected in a sterile 50 mL tube, and centrifuged at 1300 rpm for 4 min, the cells were flicked and then resuspended in 37°C incubation medium, transferred to a conical flask containing 450 mL of 37°C incubation medium, and placed in a 37°C shaker for later use.
The extracted VHH-RBD and VHH-BGTSTIP plasmids and PEI (MW 25, 000) at a ratio of 1:2 were were placed in 50 ml of culture medium, mixed thoroughly, then allowed to stand for 18 min, transferred to the above 450 ml of culture medium, suspended and cultured at 37°C, 5% $CO_2$ and 120 rpm for 6 days to express VHH-RBD and VHH-BGTSTIP proteins. During the transfection process, attention was paid to the light-proof operation of PEI.

5. Purification of VHH-RBD and VHH-BGTSTIP in mammalian expression system

After 6 days of transient transfection, the cell culture medium was collected, centrifuged at 7000g for 10 min with a JA-14 rotor, and the cell supernatant was taken. After centrifugation at 20000 g for 10 min, the supernatant was taken and filtered twice using a filter membrane with a pore size of 0.22 µm, and the sample was used for the next step of Ni-excel column purification.

Ni affinity chromatography purification was performed using the AKTA system;

Instrument system: AKTA Pure preparative liquid chromatograph;

Purification medium: Ni Sepharose excel affinity medium; Buffers: pump A buffer and pump B buffer, in which pump A buffer was 1×PBS buffer, and pump B buffer was 1×PBS+250 mmol/L imidazole buffer;

System loading flow rate: 8mL/min; Detection wavelength: UV@280 nm

System elution flow rate: 4ml/min; Detection wavelength: UV@280 nm

Elution conditions: The protein impurities were eluted with 20mM imidazole, and 250mM imidazole elution product was collected. The eluate was dialyzed against 1×PBS overnight, and the dialysate was replaced twice during the period. About 30ml of low-concentration target protein was harvested, and concentrated to 5 ml with Vivaspin20 ml, 100 KDa ultrafiltration tube for later use. The eluted samples collected were prepared into reduced and non-reduced samples, and subjected to SDS-PAGE according to the above method.

6. Experimental results

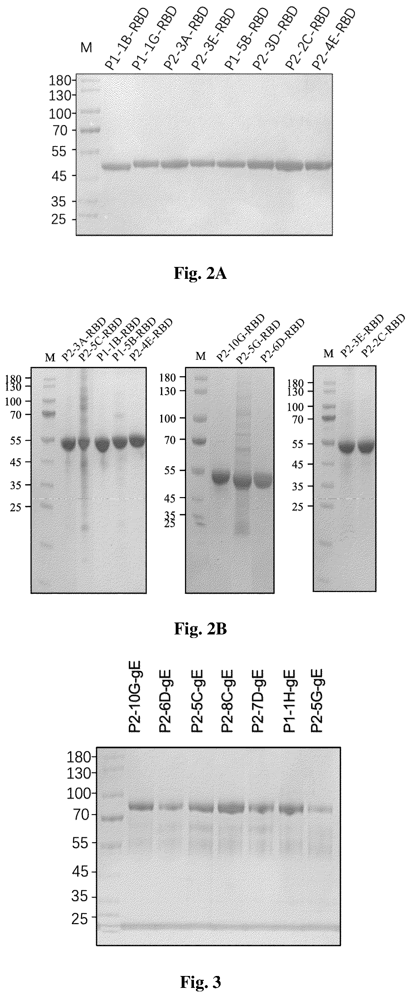

Fig. 2 showed the SDS-PAGE results of the 8 kinds of VHH-RBD proteins prepared by the present invention. Fig. 2A showed the electrophoresis results of various VHH-RBD fusion proteins produced and purified by the insect cell expression system. M represented molecular weight marker, and lanes 1 to 8 showed different VHH-RBD fusion proteins, respectively. The results showed that after one-step purification by affinity chromatography, the VHH-RBD proteins had a purity of about 90% and a molecular size of about 50 KDa. Fig. 2B showed the electrophoresis results of the 10 kinds of VHH-RBD fusion proteins produced and purified by the mammalian cell expression system. The results showed that the VHH-RBD proteins had a purity of about 90% and a molecular weight of about 50 KDa.
Fig. 3 showed the SDS-PAGE results of the 7 kinds of VHH-gE fusion proteins prepared by the present invention. M represented molecular weight marker, and lanes 1 to 7 showed different VHH-gE fusion proteins, respectively. The results showed that after one step of affinity chromatography purification, the VHH-gE proteins had a purity of about 80%, and a molecular weight of about 85KDa.

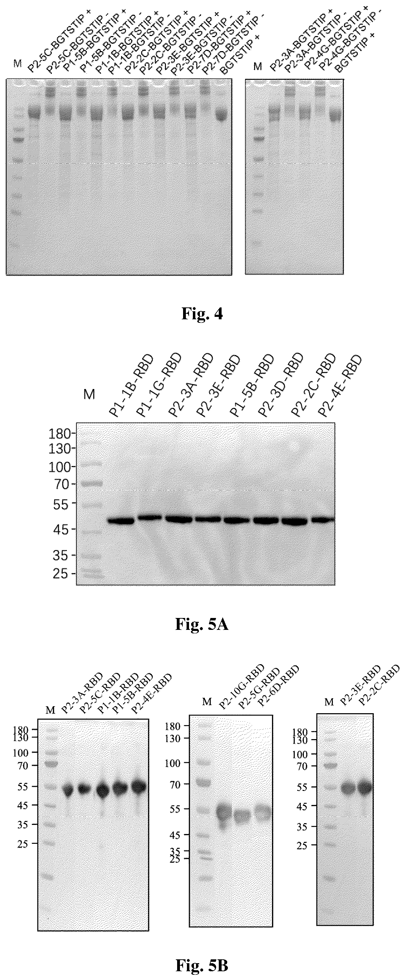

Fig. 4 showed the SDS-PAGE results of the 8 kinds of VHH-BGTSTIP proteins prepared by the present invention. "+" indicated reducing conditions, and "-" indicated non-reducing conditions. The results showed that after one step of affinity chromatography purification, the VHH-BGTSTIP proteins had a purity of about 90%, and a molecular weight of about 180KD.

Example 3: Immunoblotting experiment of VHH-RBD and VHH-gE proteins

Equal amounts of protein samples were mixed with loading buffer, boiled for 10 min, and loaded into SDS-PAGE gel for western blotting (BioRad) according to the laboratory standard protocol. The proteins were electrophoresed at 80 V for 70 min on the BioRad mini protean Tetra system, and the gel was stained with Coomassie Brilliant Blue R-250 (Bio-Rad) at room temperature for 30 min. The separated proteins were transferred to a nitrocellulose membrane using a trans-blot turbo transfer system (Bio-Rad), blocked, and incubated with anti-His-HRP (1:5000 dilution) for 1 hour. Washing was performed 5 times, 5 min each time, to remove unbound antibodies, and then detection was carried out using a chemiluminescent substrate kit.
Fig. 5 showed the results of immunoblotting experiments (Western Blot) of various VHH-RBD fusion proteins in the example of the present invention. Fig. 5A showed the results of the 8 kinds of VHH-RBD fusion proteins produced and purified by the insect cell expression system, in which M represented molecular weight marker, and lanes 1 to 8 showed the fusion proteins of different nanobodies and RBD, respectively. The results confirmed that the 50 KDa molecules were determined to be the target proteins of the present invention. Fig. 5B showed the results of the 10 kinds of VHH-RBD fusion proteins produced and purified by the mammalian cell expression system 293F cells, and the results showed that the VHH-RBD fusion proteins had a purity of about 90%, and a molecular weight of about 50 KDa. The results confirmed that the 50 KDa molecules were determined to be the target proteins of the present invention.

Fig. 6 showed the results of Western Blot of the 7 kinds of candidate molecules VHH-gE of the example of the present invention. M represented molecular weight marker, and lanes 1 to 7 showed the fusion proteins of different nanobodies and gE, respectively. The results confirmed that the 85KDa molecules were determined to be the target proteins of the present invention.

Example 4: Molecular activity analysis (ELISA) of fusion proteins

(1) A fusion protein to be tested was diluted to 1 $\mu$g/mL and coated on a 96-well plate, 100 $\mu$L per well, and allowed to stand at room temperature for 2 h;

(2) The plate was washed once and blocked with a bovine serum albumin diluent (ED, 200 $\mu$L/well) at room temperature for 2 h;

(3) The plate was washed once, and the corresponding specific monoclonal antibody was diluted to 1$\mu$g/mL, and added to the first well at a volume of 100$\mu$l; 2-fold serial dilution with 11 gradients, and two repeat wells for each gradient were adopted, and the plate was allowed to stand at room temperature for 1 h;

(4) The plate was washed 5 times, the secondary antibody GAH-HRP (1:5000) was added to the 96-well plate, 100 $\mu$L/well, and the plate was allowed to stand at room temperature for 1 h;

(5) The plate was washed 5 times, color development was carried out at room temperature for 10 min and then stopped, detection was performed at 450 nm wavelength using a microplate reader; and GraphPad Prism 5 (GraphPad, USA) software was used for data analysis.

Fig. 7 showed the results of ELISA of various VHH-RBDs of the examples and various reported specific monoclonal antibodies. Fig. 7A showed the results of activity identification of the 8 kinds of VHH-RBD proteins produced and purified by the insect cell expression system, in which the RBD antibodies included broad-spectrum neutralizing antibodies VacW-105 (corresponding to JSR-105 in Fig. 7A), JSR-551, VacW-209 (corresponding to JSR-209 in Fig. 7A), 6D6 (corresponding to m6D6 in Fig. 7A), and 7D6 (corresponding to m7D6 in Fig. 7A) (the specific information and sequences of these antibodies were obtained from the literature, Ju B, Zheng Q, Guo H, Fan Q, Li T, Song S, Sun H, Shen S, Zhou X, Xue W, Cui L, Zhou B, Li S, Xia N, Zhang Z. Immune escape by SARS-CoV-2 Omicron variant and structural basis of its effective neutralization by a broad neutralizing human antibody VacW-209. Cell Res. 2022 May;32(5):491-494. doi: 10.1038/s41422-022-00638-6. Epub 2022 Mar 8. PMID: 35260792; PMCID: PMC8902274.).

The results confirmed that the VHH-RBDs produced and purified by the insect cell expression system could still maintain a complete and correct molecular conformation and had good binding activity with RBD-specific antibodies. Fig. 7B showed the results of activity identification of the 10 kinds of VHH-RBD proteins produced and purified by the mammalian cell expression system 293F cells, in which the antibodies included 3G11, 8H12, 13F10, 8B8, 9D3, and 3F9 (these antibodies were prepared by conventional antibody preparation methods of the reference literature in this laboratory. In short, the extracellular segment of the SARS-CoV-2 surface spike protein S protein was expressed and purified in 293F cells. Then BALB/c mice were immunized, and blood was collected for serum detection after 0, 2, 3, and 5 weeks of immunization. After the immunization, two mice with higher mouse immune serum binding titers and neutralization titers were selected for spleen immunization. After 10 $\mu$g of mixed proteins was injected into the mouse spleen, the mice were sutured and observed regularly, and cell fusion experiments were performed three days later. After fusion and culture, the cell supernatant was taken and the reactions of the cell supernatant with SARS-CoV-2 S-2P trimer protein, RBD, S2 protein and SARS-CoV-1 S-2P protein were detected by indirect ELISA method. The wells with strong binding ability to the corresponding proteins were selected for picking and cloning experiments, and monoclonal antibodies were obtained after more than three rounds of cloning. Ascites mice were immunized, and ascites was extracted and purified with protein A column to obtain corresponding monoclonal antibodies.). The results showed that the VHH-RBDs produced and purified by the mammalian cell expression system could still maintain a complete and correct molecular conformation, and had good binding activity with RBD-specific antibodies. In addition, the binding activity of these fusion proteins with antibodies (e.g., 8H12, 13F10, 8B8) was better than that of RBD proteins.

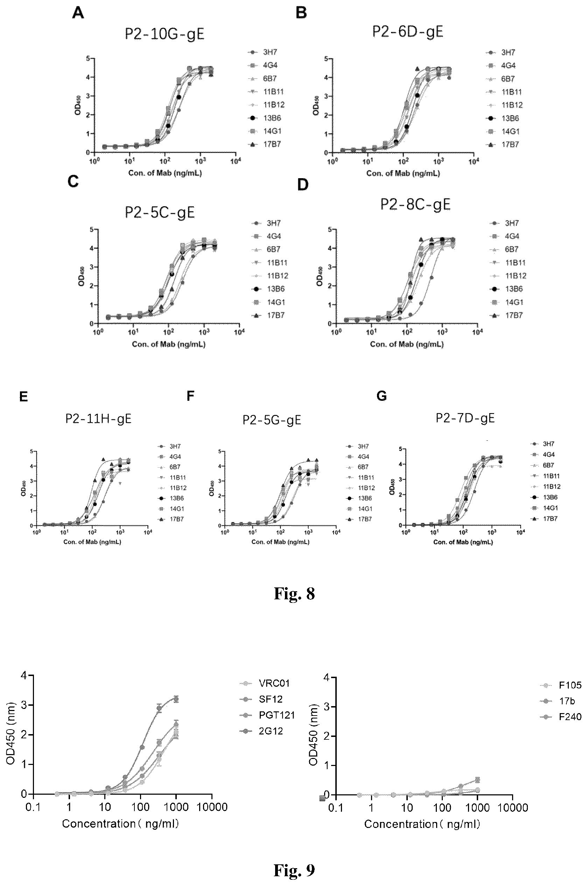

Fig. 8 showed the results of ELISA of the 7 kinds of VHH-gEs in the examples with various specific monoclonal antibodies. The gE-specific monoclonal antibodies included 3H7, 4G4, 6B7, 11B11, 11B12, 13B6, 14G1, and 17B7 (the specific information and sequences of these antibodies were obtained from the literature, Liu, J., Ye, X., Jia, J. et al. Serological Evaluation of Immunity to the Varicella-Zoster Virus Based on a Novel Competitive Enzyme-Linked Immunosorbent Assay. Sci Rep 6, 20577 (2016). https://doi.org/10.1038/srep20577). The results showed that the produced and purified VHH-gEs could still maintain a complete and correct molecular conformation and had good binding activity with gE-specific antibodies.

Fig. 9 showed the results of ELISA of various VHH-BGTSTIPs in the examples with various reported HIV-1 neutralizing antibodies or non-neutralizing antibodies. These antibodies were recombinantly expressed in 293F cells, and the sequences of these antibodies were obtained from NCBI (VRC01 (GeneBank: MK032237.1/ GU980703.1), SF12 (GeneBank: MK722171.1/ MK722164.1), 2G12 (GeneBank: OM484328.1/ AF029237.1), PGT121 (GeneBank: JN201911.1/ JN201894.1), or the article reported F105 (Wilkinson, R.A., C. Piscitelli, M. Teintze, et al. Structure of the Fab fragment of F105, a broadly reactive anti-human immunodeficiency virus (HIV) antibody that recognizes the CD4 binding site of HIV type 1 gp120. J Virol, 2005. 79(20): 13060-13069.), F240 (Gohain, N., W.D. Tolbert, C. Orlandi, et al. Molecular basis for epitope recognition by non-neutralizing anti-gp41 antibody F240. Sci Rep, 2016. 6: 36685.), 17b (Carlo D. Rizzuto, Richard Wyatt, Nivia Herna ndez-Ramos, et al. A Conserved HIV gp120 Glycoprotein Structure Involved in Chemokine Receptor Binding. Science, 1998. 280(19): 1949-1953.). The results showed that the various VHH-BGTSTIP fusion proteins of the present invention had good binding activity with various neutralizing antibodies, but weaker binding activity with non-neutralizing antibodies. This indicated that the VHH-BGTSTIPs expressed by 293F cells well presented some neutralizing antibody epitopes on Env, without exposing some non-neutralizing antibody epitopes.

Example 5: Affinity analysis (SPR) of fusion proteins with HEV-p239

Biacore 8K was loaded with CM5 chip, and the pipeline was flushed with PBS-P buffer (PBS+0.5% P20, cytiva).
Ligand HEV-p239 (10 $\mu$g/mL) was centrifuged at high speed for 10min.
HEV-p239 coupling program was set: chip channel was activated (EDC:NHS=1:1, flow rate: 10$\mu$l/min), and then the flushing program (PBS-P buffer, flow rate: 30$\mu$l/min) was performed. HEV-p239 (amino acid sequence was set forth in SEQ ID NO: 40) was loaded for coupling (time: 420 s, flow rate: 10 $\mu$l/min). After the amount of ligand for coupling was determined, ethanolamine was used for channel blocking procedure (time: 420 s, flow rate: 10 $\mu$l/min).
Affinity detection: The fusion protein was set to have concentration gradients of 125 nM, 62.5 nM, 31.2 nM, 15.6 nM, 7.8 nM, and 3.9 nM, and loaded for detection. The sample was set to have an association time of 120 s, a dissociation time of 200 s, and a flow rate of 30 $\mu$l/min.
Biacore 8K (Cytiva) software was used to fit the association (Ka)/dissociation (Kd) curve by the kinetic method, and the affinity (KD) was analyzed and calculated.

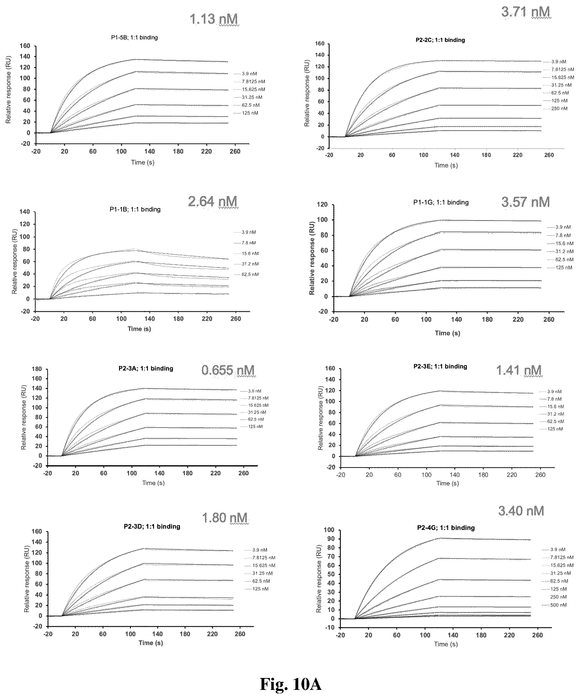

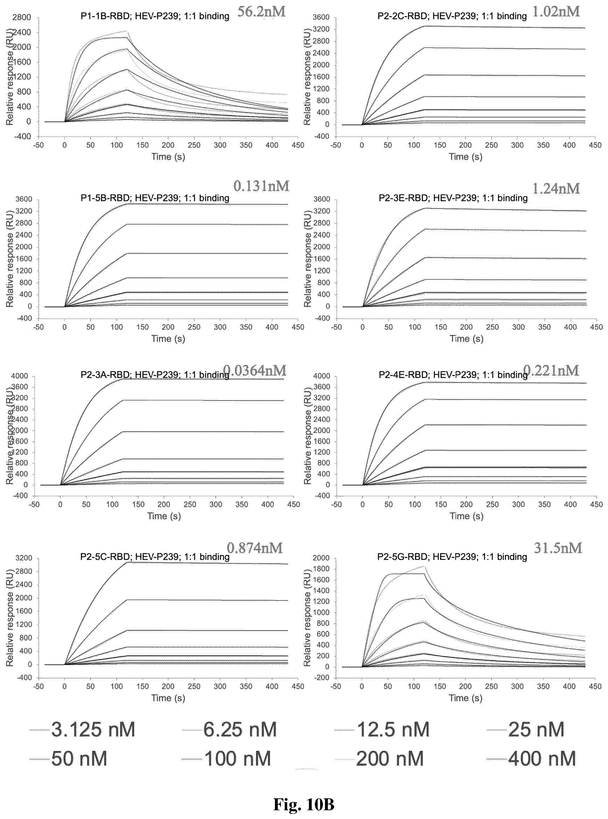

Fig. 10 showed the affinity analysis results of various VHH-RBD fusion proteins of the examples with HEV-p239 protein. Fig. 10A showed the affinity results of the VHH-RBDs produced and purified by the insect cell expression system with HEV-p239 protein, and the results showed that the various VHH-RBDs all had high affinity at nanomolar level with HEV-p239 particles. Fig. 10B showed the affinity results of the VHH-RBDs produced and purified by the mammalian cell expression system 293F cells with HEV-p239 protein, and the results showed that the VHH-RBDs had high affinity at nanomolar level with HEV-p239 particles.

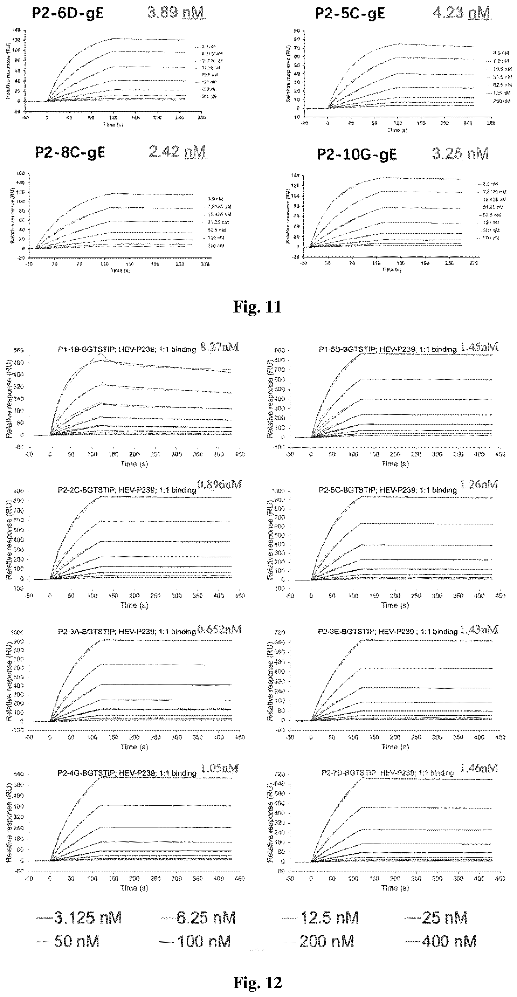

Fig. 11 showed the affinity analysis results of various VHH-gE fusion proteins of the examples with HEV-p239 protein. The results showed that the various VHH-gEs all had high affinity at nanomolar level with HEV-p239 particles.
Fig. 12 showed the affinity analysis results of various VHH-BGTSTIP fusion proteins of the examples with HEV-p239 protein. The results showed that the various VHH-BGTSTIPs all had high affinity at nanomolar level with HEV-p239 particles.

Example 6: Molecular sieve chromatography purification of fusion proteins and complexes thereof

Instrument system: AKTA explorer 100 type preparative liquid chromatography system, produced by GE Healthcare (formerly Amershan Pharmacia).

Chromatographic medium: Superdex 200 increase (cytiva).

Column volume: 20cm$\times$20mm.

Buffer: 20mM phosphate buffer, pH7.4.

Flow rate: 0.7 mL/min.

Detector wavelength: 280 nm.

The samples were the samples in Example 1 and Example 5.
The elution procedure comprised: fractionally collecting penetration peak.

The product penetrated Supedex 200 increase was collected to obtain 5 mL of purified sample. 50 $\mu$L of each elution product was taken, added with 10 $\mu$L of 6X Loading Buffer and mixed well. After 10 min in a water bath at 80°C, 10 $\mu$l of the mixture was taken and electrophoresed in a 10% SDS-polyacrylamide gel at 120V for 60 min. Then Coomassie Brilliant Blue staining was used to show the electrophoresis bands.

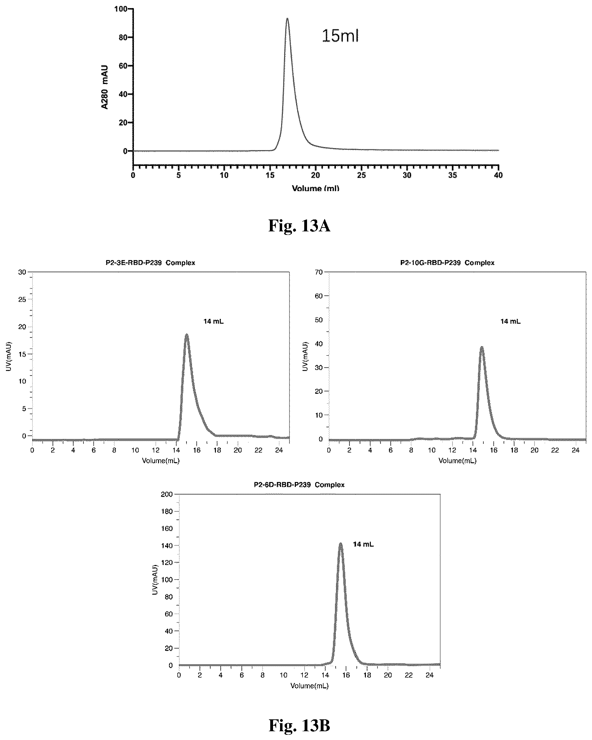

Fig. 13A showed the purification results of the fusion protein P1-5B-RBD produced and purified by the insect cell expression system in the example of the present invention by superdex 200 increase (high performance liquid chromatography molecular sieve). The results showed that the purity of P1-5B-RBD protein reached more than 95%. Fig. 13B showed the purification results of the P2-6D-RBD, P2-3E-RBD, and P2-10G-RBD produced and purified by the mammalian cell expression system in the example of the present invention by superdex 200 increase. The results showed that the three fusion proteins all presented a single elution peak, indicating that the proteins had high purity and uniformity.

Fig.s 14 to 16 showed the purification chromatograms and SDS-PAGE identification results of the complexes formed by various fusion proteins constructed in the present invention and HEV-p239, in which,

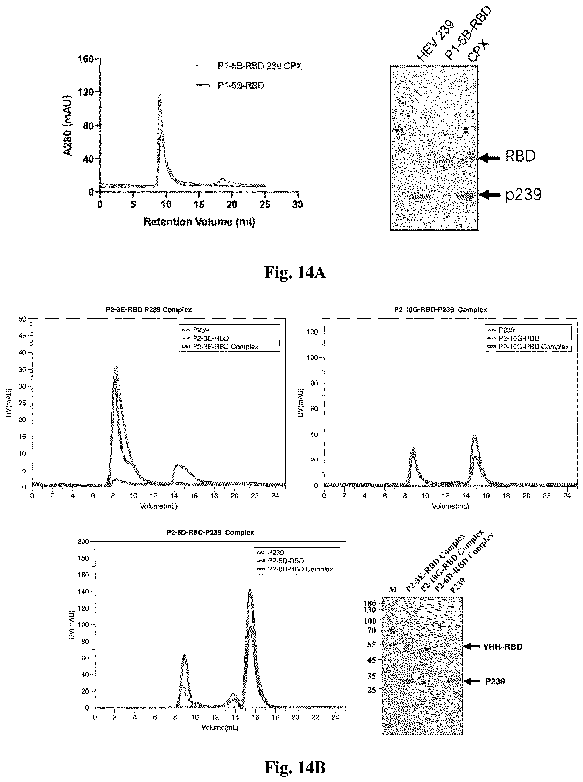

Fig. 14A showed the purification chromatograms and SDS-PAGE identification results of the complexes formed by the P1-5B-RBD fusion protein purified by the insect cell expression system and HEV-p239, in which the purple curve was significantly higher than the blue curve, proving that the fusion protein P1-5B-RBD specifically bound to the surface of the HEV particles to form the complexes.

Fig. 14B showed the purification chromatograms and SDS-PAGE identification results of the complexes formed by the P2-3E-RBD, P2-10G-RBD and P2-6D-RBD purified by the mammalian cell expression system for the candidate fusion proteins of the example of the present invention and HEV-p239, respectively. The results showed that the P2-3E-RBD, P2-10G-RBD and P2-6D-RBD formed complexes with HEV-p239, and could be purified by superdex 200 10/300 increase.

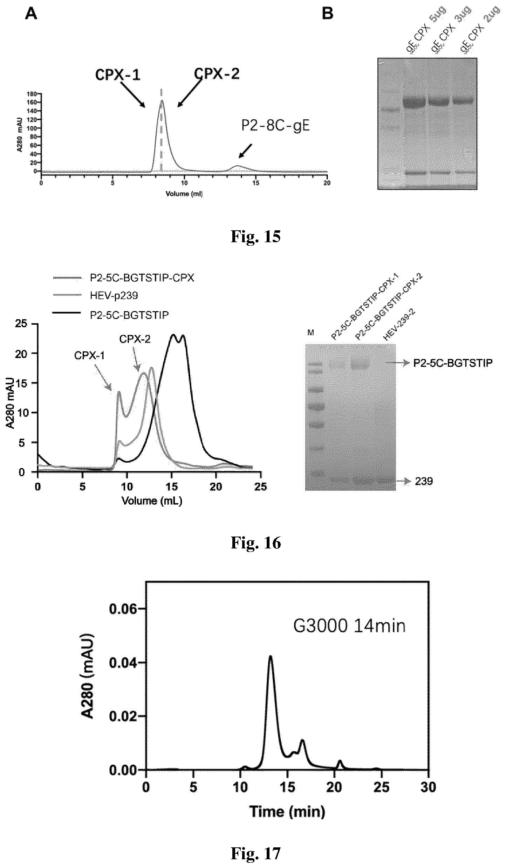

Fig. 15 showed the purification chromatogram and SDS-PAGE identification results of the complex formed by the candidate fusion protein P2-8C-gE and HEV-p239 in the example of the present invention. Fig. 15A showed the purification results of the complex. The results showed that the retention volume of the complex in the superdex 200 increase (Cytiva) chromatography column was 8 ml, which was displayed as the particle peak component, and the 14 ml peak was the unbound P2-8C-gE, indicating that the 8 ml particle peak component had been bound. Therefore, P2-8C-gE was saturatedly bound to the surface of the HEV particles to form the complex. The SDS-PAGE results confirmed that the complex lane showed typical two-component bands (HEV-p239 = 20 KDa, P2-8C-gE = 85 KDa), so the sample purified by superdex 200 increase was determined to be a gE complex sample.

Fig. 16 showed the purification chromatogram and SDS-PAGE identification results of the complex of the candidate fusion protein P2-5C-BGTSTIP in the example of the present invention and HEV-p239. The results showed that the retention volume of the complex in the superose 6 column was about 9 ml, the retention volume of P2-5C-BGTSTIP was about 16 ml, and different components were collected for SDS-PAGE identification. The sample with an elution volume of 9 mL showed two bands in SDS-PAGE, confirming that the complex sample was obtained at about 9 mL after the complex was purified by superose 6.

In summary, the above experimental results proved that the complex particles formed by various candidate fusion proteins and HEV-p239 were successfully purified and obtained in the present application.

Example 7: High-performance size exclusion chromatography (HPSEC) analysis of P2-8C-gE protein

Instrument: Waters. System flow rate: 0.5 mL/min for G3000PW$_{XL}$. Wavelength: 190 to 600 nm. Column wavelengths: 280 nm and 254 nm.

Buffer: PBS.

Operation process: The column was pre-equilibrated for 30 to 60 min until there was no obvious change in the absorption value at 280 nm. The absorption value of the detector was returned to zero. The chromatography operation method was edited, the sample was centrifuged first, the sample to be analyzed was injected into a 100 $\mu$L sample loop, automatic loading was set, and the instrument run for 30 min. It was observed that the retention time of the S trimer was about 14 min.

The results were shown in Fig. 17. The P2-8C-gE fusion protein of the example of the present invention presented a single main peak, without aggregation component, and had a retention time of about 14 min, and a purity of about 80%.

Example 8: Molecular particle size detection (DLS) of RBD complexes and gE complexes

Instrument: NanoBrook Series (Brookhaven instrument).

Functional module: DLS (Dynamic Light Scattering).

Buffer: PBS.

Operation process: The instrument was turned on and preheated for 5 min in advance. The sample to be tested was prepared (concentration: 0.5 mg/ml, centrifugation: 12000 rpm for 5 min, 50 μl was taken and added to the sample cup). Detection parameter settings: Detection time: 300 s each time, 3 repeats for each sample.

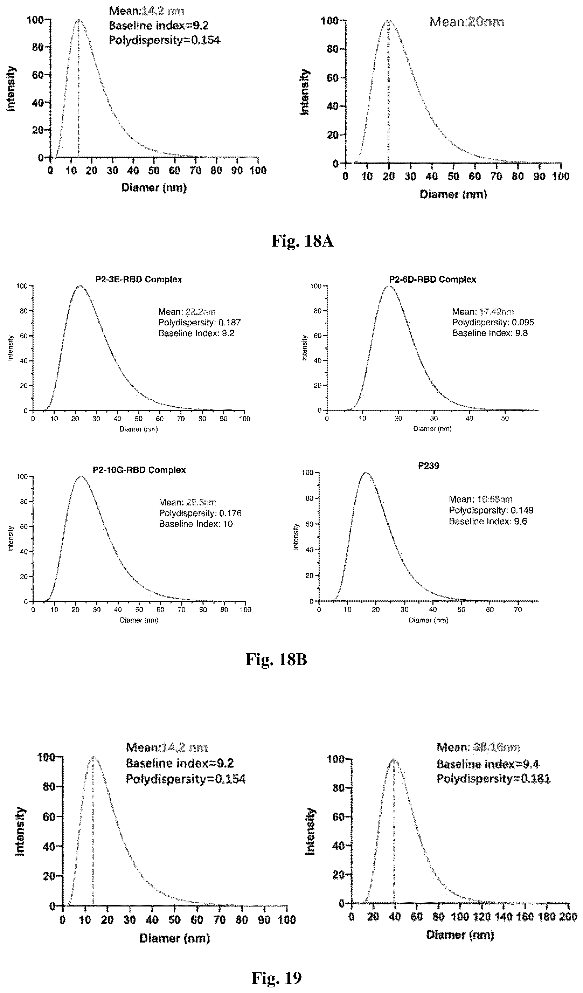

Fig. 18 showed the molecular particle size detection results of the HEV-RBD complex sample formed by the VHH-RBD produced and purified by the insect cell expression system and HEV-p239 protein in the example of the present invention. Fig. 18B showed the molecular particle size detection results of the HEV-RBD complex sample formed by the VHH-RBD produced and purified by the mammalian cell expression system and HEV-p239 protein. The results of Fig. 18A and Fig. 18B showed that the molecular particle size of the HEV-RBD was significantly larger than that of HEV-p239 particles. The particle size of the HEV-RBD complex was significantly larger than that of HEV-p239, which confirmed that VHH-RBD could bind to the surface of HEV particles and maintain a particle state.

Fig. 19 showed the molecular particle size detection results of the HEV-gE complex samples in the example of the present invention. The results showed that the molecular particle size of the HEV-p239 particles was 14.2 nm, and the molecular particle size of the HEV-gE was 38 nm. The results confirmed that the VHH-gE could bind to the surface of the HEV particles and maintain a particle state.

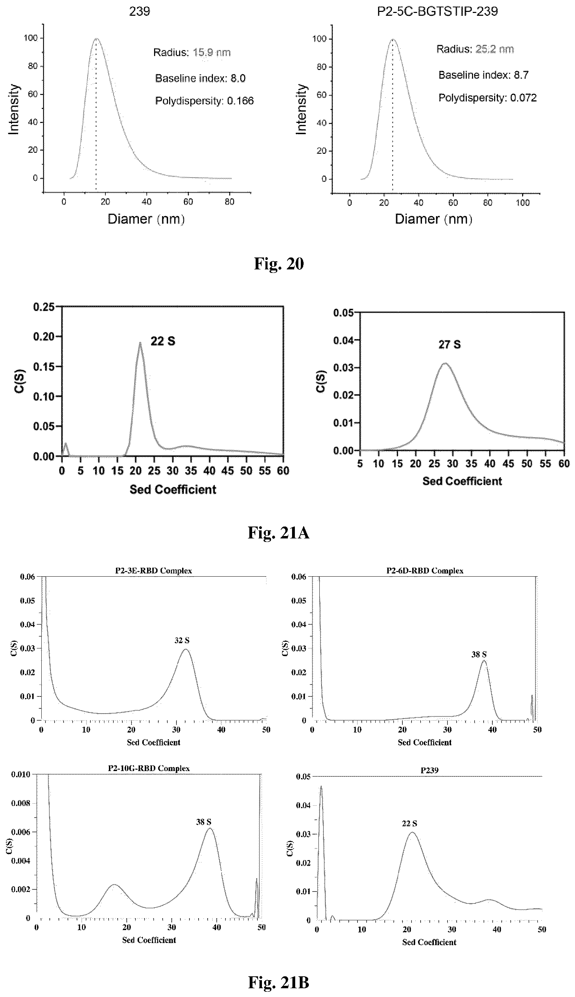

Fig. 20 showed the molecular particle size detection results of the HEV-BGTSTIP complex sample of the example of the present invention. The results showed that the molecular particle size of the HEV-p239 particle was 15.9 nm, and the particle size of the HEV-BGTSTIP complex was 25.2 nm. The results showed that VHH-BGTSTIP could bind to the surface of the HEV particles and maintain a particle state.

Example 9: Calculation of sample sedimentation coefficient by analytical ultracentrifugation method

The instrument used was a Beckman XL-A analytical ultracentrifuge equipped with an optical detection system and an An-60Ti rotor.

The sample pool was installed according to the operating instructions, 400 μL of sample buffer was added to the control pool (the same buffer as used for sample), 380 μL of sample (OD280 was about 0.8) was added to the sample pool, and the sample pool was balanced to reach a weight difference within 0.1 g.

The sample pool was placed into the An-60Ti rotor, the rotor was placed in the cavity of the Beckman XL-A analytical ultracentrifuge, and the optical path detector was installed.

Parameter settings: temperature (20°C), Rmin (6.0 cm), Rmax (7.2 cm), wavelength (280 nm), step speed (0.003 cm), scan mode (continuous), data interval (30 sec), and number of data (150 scans). The centrifuge speed was set to 30, 000 rpm.

After the experiment was completed, the density and viscosity of the buffer and the partial specific volume of the known protein were calculated using the SENDTERP software. The sedimentation coefficient was analyzed using the Origin version of the Nonlin and SEDFIT software, in which the globulin friction ratio f/f0 was preset to 1.2, the analysis range was set according to the molecular weight and basic properties of the sample protein, the calculation resolution was set to 100, and it was generally required that the RMSD value was not more than 0.01 and the residue map fluctuation was within 0.05.

Fig. 21 showed the analytical ultracentrifugation results of HEV-p239 and the HEV-RBD complex constructed based on HEV-p239 particles in the example of the present invention. Fig. 21A showed the analytical ultracentrifugation results of the complex particles prepared by the VHH-RBD produced and purified by the insect cell expression system and HEV-p239 (right figure), and HEV-p239 (left figure). The results showed that HEV-p239 presented a single component with a sedimentation coefficient of 22S, and the HEV-RBD complex presented a single component with a sedimentation coefficient of 27 S, which was significantly higher than the sedimentation coefficient (22S) of HEV-p239 particles. The results confirmed that the RBD complex could maintain a stable particle morphology in the aqueous solution state, and the RBD was firmly bound to the surface of the HEV particles to form RBD particle protein. Fig. 21B showed the analytical ultracentrifugation results of the complex particles prepared by the VHH-RBD produced and purified by the mammalian cell expression system 293F cells and HEV-p239, and the results showed that the HEV-RBD complex presented a single component, and its sedimentation coefficient was significantly higher than the sedimentation coefficient (22 S) of the HEV-p239 particles, which confirmed that it could maintain a stable particle morphology in the aqueous solution state, and RBD was firmly bound to the surface of the HEV particles to form RBD particle protein.

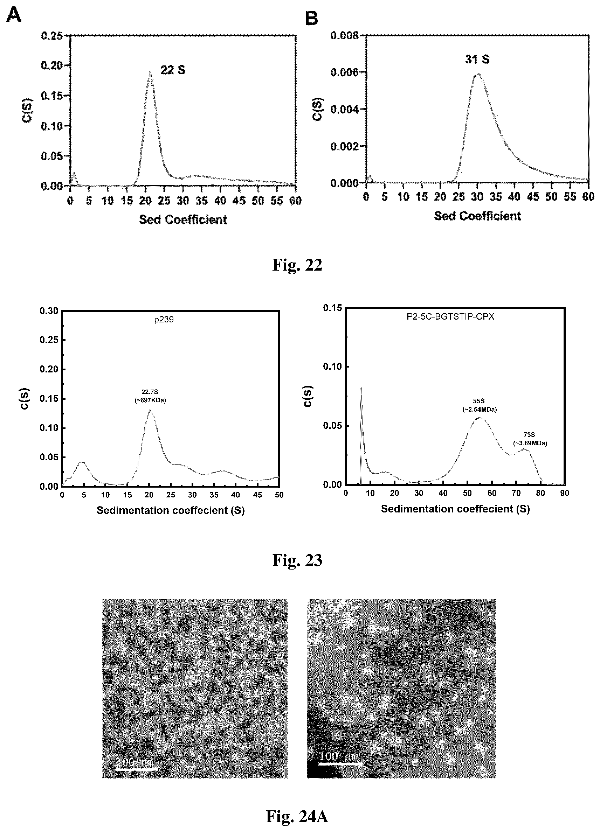

Fig. 22 showed the analytical ultracentrifugation results of HEV-p239 (Fig. 22A) and HEV-gE complex constructed based on HEV-p239 particles (Fig. 22B). The results showed that HEV-p239 presented a single component and had a sedimentation coefficient of 22S, while the HEV-gE complex presented a single component and had a sedimentation coefficient of 31 S, which was significantly higher than the sedimentation coefficient (22 S) of the HEV-p239 particles. The results confirmed that the HEV-gE complex could maintain a stable particle morphology in the aqueous solution state, and gE was firmly bound to the surface of the HEV particles to form gE particle protein.

Fig. 23 showed the analytical ultracentrifugation results of HEV-p239 and Env (BGTSTIP) complex constructed based on HEV-p239 particles in the example of the present invention.

Example 10: Observation of particle morphology by transmission electron microscope

The transmission electron microscope used was Tecnai G2 Spirit 120 kV (FEI). Phosphotungstic acid was used for negative staining.

Sample preparation: First, the copper mesh (R2/2, 200 mesh, ThermoFisher Scientific) was subjected to glow discharge hydrophilic treatment. Then 5 μl of a sample with a concentration of 0.5mg/ml was dripped onto the copper mesh, and after standing at room temperature for 60s, the liquid droplet was absorbed from the edge of the copper mesh with absorbent paper. After being placed at room temperature to dry, the copper mesh was observed on the machine.

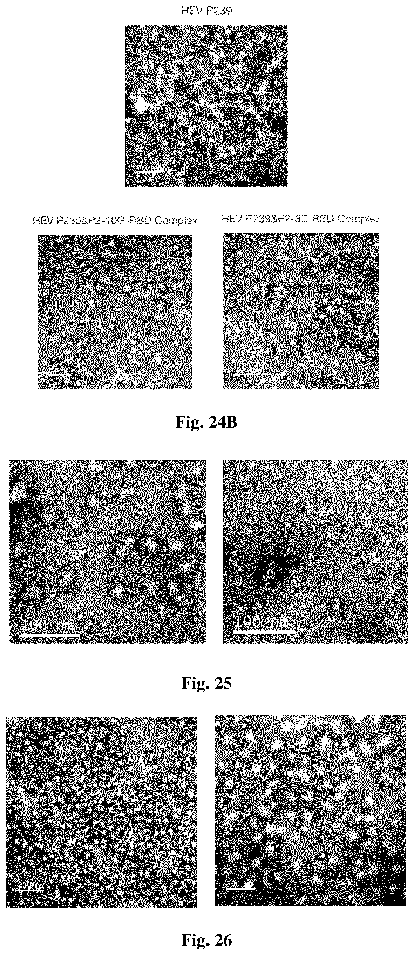

Fig. 24A showed the transmission electron microscope negative staining results of the complex sample prepared by HEV-p239 and the VHH-RBD produced and purified by the insect cell expression system in the example of the present invention. Fig. 24B showed the transmission electron microscope negative staining results of the complex sample prepared by HEV-p239 and the VHH-RBD produced and purified by the mammalian expression system 293F cells in the example of the present invention. The results all showed that the HEV-RBD complexes presented a typical virus-like particle morphology. The results confirmed that the HEV-RBD complex samples constructed in the present application were RBD particulate antigens.

Fig. 25 showed the transmission electron microscopy negative staining results of the HEV-gE complex in the example of the present invention. The results showed that the HEV-gE complex presented a typical virus-like particle morphology. The results confirmed that the HEV-gE complex sample constructed in the present application was a particulate antigen.

Fig. 26 showed the transmission electron microscopy negative staining results of the HEV-Env (BGTSTIP) complex in the example of the present invention. The results showed that the HEV-Env complex presented a typical virus-like particle morphology, which confirmed that the HEV-Env complex sample constructed in the present application was a particulate antigen.

Example 11: Evaluation of immunogenicity of HEV-RBD particles

This experimental protocol was approved by the Experimental Animal Management Ethics Committee of Xiamen University. All operations were carried out in strict accordance with the animal ethics guidelines and the approved procedures.

Evaluation of immunogenicity of RBD particles expressed in insect cells

Six-week-old Balb/C mice were selected and divided into four groups, with five mice in each group. Each group was immunized with HEV-RBD particles (immunization dose was 0.5 μg), HEV-RBD particles (immunization dose was 5 μg), P1-5B-RBD monomer (immunization dose was 0.5 μg), and P1-5B-RBD monomer (immunization dose was 5 μg) combined with aluminum adjuvant, by intramuscular injection (50 μL) at the left or right hind limb of mice at 0, 2, and 6 weeks. Ocular venous blood was collected at 0, 1, 2, 3, 4, 5, 6, 7, and 8 weeks, in which blood was collected before injection at 0, 1, and 4 weeks. Blood samples were centrifuged at 13, 000 g for 10 min, and serum samples obtained were stored at -20°C. The antigen-specific IgG and neutralizing antibody titer were determined by the end-point enzyme-linked immunosorbent assay and the neutralization method based on wild-type SARS-CoV-2 pseudovirus (the construction method referred to the literature, Xiong HL, Wu YT, Cao JL, Yang R, Liu YX, Ma J, Qiao XY, Yao XY, Zhang BH, Zhang YL, Hou WH, Shi Y, Xu JJ, Zhang L, Wang SJ, Fu BR, Yang T, Ge SX, Zhang J, Yuan Q, Huang BY, Li ZY, Zhang TY, Xia NS. Robust neutralization assay based on SARS-CoV-2 S-protein-bearing vesicular stomatitis virus (VSV) pseudovirus and ACE2-overexpressing BHK21 cells. Emerg Microbes Infect. 2020 Dec;9(1):2105-2113. doi: 10.1080/22221751.2020.1815589. PMID: 32893735; PMCID: PMC7534347.), respectively.

Evaluation of immunogenicity of RBD particles of mammalian expression system

Mice: female, 6 weeks old, purchased from Beijing Weitong Lihua Experimental Animal Technology Co., Ltd. Twelve groups of immunized mice were set up, with 5 mice in each group. P2-3E-RBD, P2-10G-RBD, P2-6D-RBD and p239 were used to prepare particulate complex antigens, which were diluted with physiological saline to the required concentrations, mixed with aluminum adjuvant in a volume ratio of 1:1 and adsorbed on the adjuvant overnight at 4°C, and mice were immunized intramuscularly at 0, 2, and 5 weeks (100 μl in total; 50 μL each for the left and right hind legs). The immunization schemes in mice were shown in Table 4.

Table 4: Immunization schemes in mice

| | |
|---|---|
| P2-3E-RBD | 0.5 μg/5 μg |
| P2-3E-RBD-CPX | |
| P2-10G-RBD | 0.5 μg/5 μg |
| P2-10G-RBD-CPX | |
| P2-6D-RBD | 0.5 μg/5 μg |
| P2-6D-RBD-CPX | |

Blood was collected from the eyes of mice weekly, and the mice were euthanized by carbon dioxide after 9 weeks. After the blood sample was placed at 37°C for 30 min, it was centrifuged at 13300 rpm for 10 min, and the serum was collected and stored at -20°C, and subjected to the wild-type SARS-CoV-2 pseudovirus neutralization and binding antibody titer determination.

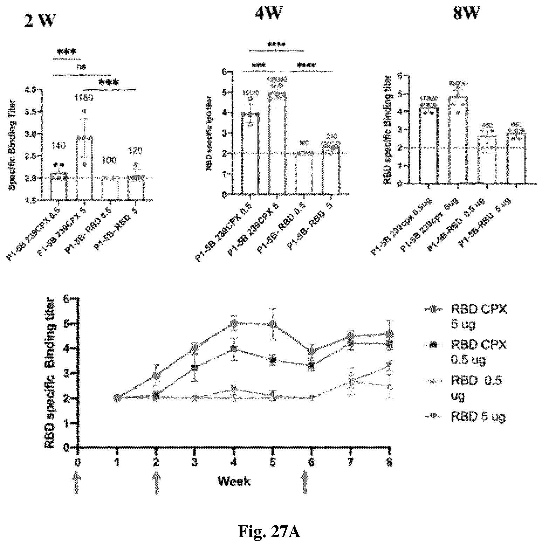

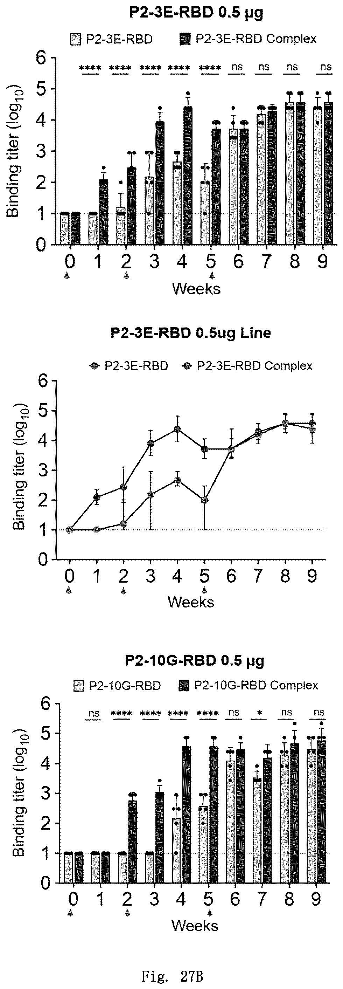

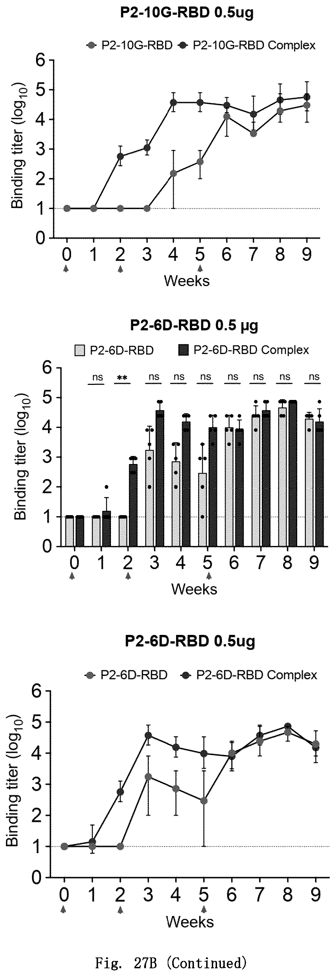

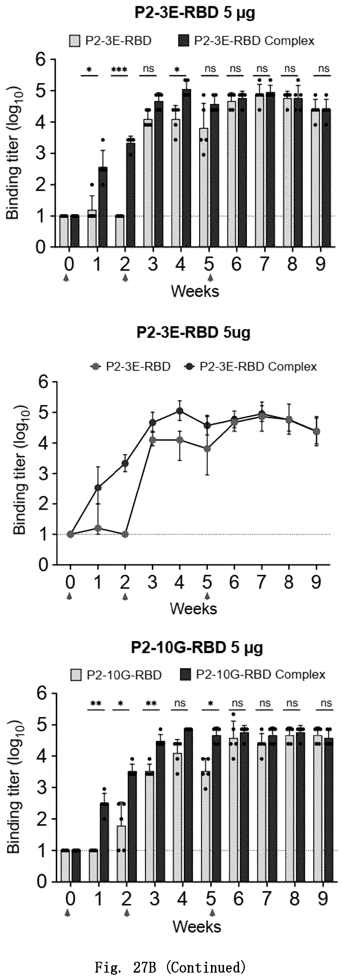

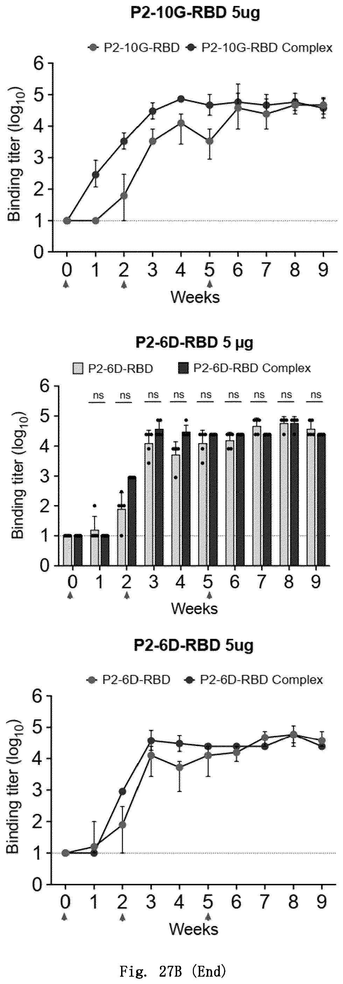

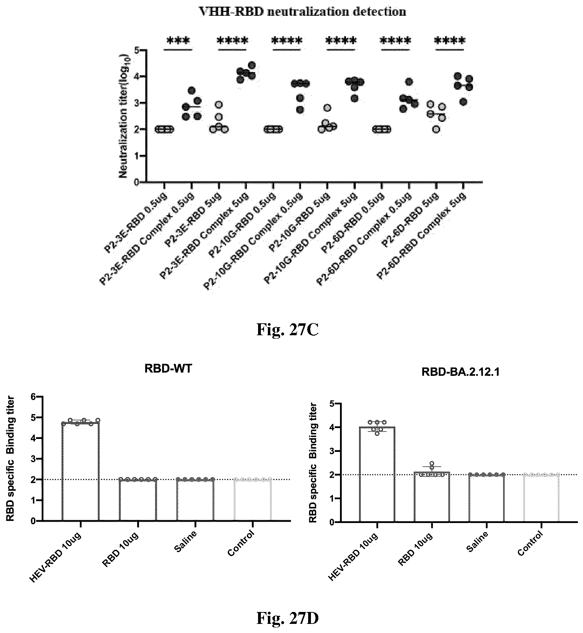

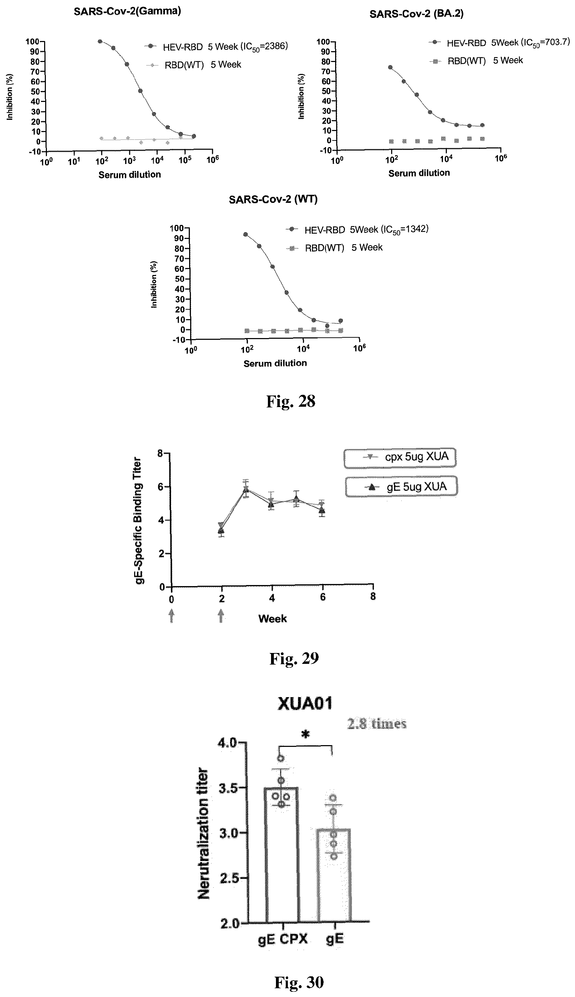

The results were shown in Fig. 27 and Fig. 28. Fig. 27 showed the immunogenicity detection results of the particulate antigen HEV-RBD in the example of the present invention. Fig. 27A showed the results of serum binding and neutralization activity detection after the mice were immunized with the particulate antigen HEV-RBD obtained by the insect cell expression system. Fig. 27B and Fig. 27C showed the results of serum binding activity and wild-type SARS-CoV-2 pseudovirus neutralization activity detection after the mice were immunized with the particulate antigen HEV-RBD obtained by the mammalian cell expression system, and Fig. 27D showed the serum antibody binding titer after the hamsters were immunized with the particulate antigen HEV-RBD obtained by the mammalian cell expression system.

Fig. 28 showed the neutralization detection results of the antibodies induced by the particulate antigen HEV-RBD of the example of the present invention combined with aluminum adjuvant for the wild-type (WT) strain, Gamma strain, and BA.2 strain of SARS-CoV-2.

The results showed that the display of VHH-RBD expressed by insect cells or VHH-RBD expressed by mammalian cells on the surface of p239 particles could significantly improve the immunogenicity of RBD antigens.

Specifically, in the 5μg of HEV-RBD particles (shown as RBD CPX in the figure) immunization group, an immune barrier could be quickly established after the first immunization, and the binding antibody titer reached about 3log. After booster immunization, the binding antibody titer reached 5log, which was 500 times higher than the binding antibody titer produced by the P1-5B-RBD monomer, and did not decrease significantly in 8 weeks. In the mouse immunization experiment, the binding antibody titer induced by HEV-RBD was significantly higher than that of the monomeric RBD protein. At an immunization dose of 0.5μg/mouse, P2-3E-RBD-p239, P2-10G-RBD-p239 and P2-6D-RBD-p239 complex particles could induce relatively higher binding antibody titers (compared to the corresponding VHH-RBD proteins), in which the binding antibody titers of the P2-3E-RBD-p239 and P2-10G-RBD-p239 complex immunization groups before the fifth injection were significantly higher than those of the corresponding VHH-RBD immunization groups (Fig. 27B). The pseudovirus neutralization results of the sixth week showed that the complex particles formed by the three VHH-RBDs and p239 could induce more significant neutralizing antibody responses (Fig. 27C).

Moreover, the neutralizing antibodies of the HEV-RBD particles had a broad-spectrum neutralizing ability, and could effectively neutralize pseudoviruses (VSV-Spike) including the wild strain, Gamma strain, and BA.2 strain of SARS-CoV-2.

Example 12: Evaluation of immunogenicity of gE particles

This experimental protocol was approved by the Experimental Animal Management Ethics Committee of Xiamen University. All operations were carried out in strict accordance with the animal ethics guidelines and the approved procedures.

Six-week-old Balb/C mice were selected and divided into 8 groups, with 5 mice in each group. Each group was immunized with HEV-gE particles (immunization dose was 0.1 μg), HEV-gE particles (immunization dose was 0.5 μg), HEV-gE particles (immunization dose was 5 μg), P2-8C-gE monomer (immunization dose was 0.1 μg), P2-8C-gE monomer (immunization dose was 0.5 μg), P2-8C-gE monomer (immunization dose was 5 μg) in combination with aluminum adjuvant, by intramuscular injection (50 μL) at the left or right hind limbs of mice at 0 and 2 weeks, respectively. Ocular venous blood was collected at 0, 1, 2, 3, 4, 5, and 6 weeks, respectively. Blood samples were centrifuged at 13000 g for 10 min, and the resultant serum samples were stored at - 20°C. The antigen-specific IgG and neutralizing antibody titers were determined by endpoint enzyme-linked immunosorbent assay and attenuated virus (v-Oka strain) neutralization method, respectively.

Process of neutralization operation (ELISPOT method):

1. Guinea pig serum (purchased from Beijing Boerxi Technology Co., Ltd., Cat. No.: BM361Y) and v-Oka virus (ATCC, Cat. No.:VR-795) dry powder were redissolved with virus protection solution, and the complement was filtered with a 0.22 μm small filter for later use;

2. The serum was diluted 50 times with virus protection solution, added to the first well of a 24-well plate, subjected to 2-fold serial dilution with 4 gradients, and incubated with vOka virus at 37°C for 1h;

3. The serum virus mixture was transferred to a 24-well plate pre-coated with ARPE-19 cells, incubated at 37°C for 1h, the liquid was discarded after 1h, and F12 medium was supplemented, and cultured at 37°C for 3 days;

4. After 3 days, the medium was discarded, the plate was washed once with PBS, fixed with a fixation solution at room temperature for 5 min, the fixation solution was discarded, and permeabilization was performed with permeabilization solution at room temperature for 10min;

5. Primary antibody 1B11-HRP (1:2000) was added to the 24-well plate, and the plate was allowed to stand at 37°C for 1 h;

6. The plate was washed 5 times, and subjected to color development at room temperature for 5 min, an enzyme-linked spot image analysis system was used for reading and counting spots; and GraphPad Prism 5 (GraphPad, USA) software was used for data analysis.

Fig. 29 showed the immunogenicity detection results of the gE particulate antigens in the example of the present invention, which showed that the gE particulate antigen combined with AS01B-like adjuvant could induce binding antibodies at high titer level (shown as XUA in the figure, up to about 6 log).

Fig. 30 showed the results of the live virus neutralization detection of mouse immune serum for the HEV-gE particulate antigen in the example of the present invention. The neutralizing antibody induced by the HEV-gE particulate antigen was 2.8 times that of the P2-8c-gE monomer. The results confirmed that the expression on the surface of p239 particles could significantly improve the immunogenicity of gE antigen.

Example 13: Flow cytometry detection of cytokines in immune group of gE particulate antigen

Mice were immunized according to the method described in Example 6 (using the same adjuvant), and the subsequent experimental operations were as follows:

a) Spleen removal: The mice were killed by cervical dislocation, and soaked in 75% ethanol for 3 to 5 min. The mice were placed on their right sides and the spleen was removed aseptically (fat was removed as much as possible);

b) Grinding: A 6-well cell plate was placed in a mesh, 1640 culture medium containing 10% FBS was added, and the spleen was placed in the mesh (completely immersed in the culture medium), and the red tissue was ground with a 2 mL syringe head until no red tissue was visible. The ground cells were transferred to a 50 mL tube and placed on ice;

c) Centrifugation was performed at 400 g, 4°C for 5 min, the supernatant was discarded, and the cell pellet at the bottom of the tube was loosen by knocking with a hemostatic forceps;

d) The cell pellet was added with 10 mL of pre-cooled RBC solution, resuspended, and placed on ice for 5 min (inverted and mixed during the period);

e) Centrifugation was performed at 400g, 4°C for 5 min, the supernatant was discarded, and the cell pellet at the bottom of the tube was loosen by knocking with a hemostatic forceps;

f) The cell pellet was added with 10 mL of pre-cooled culture medium, resuspended, and washed to remove red blood cell pellet or fat, after being pipetted evenly, 50 μl of the cells was taken for counting;

g) Centrifugation was performed at 400 g, 4°C for 5 min, the supernatant was discarded, the cell pellet at the bottom of the tube was loosen by knocking with a hemostatic forceps, added with a certain amount of culture medium, and diluted to reach a cell count of $2 \times 10^7$/mL;

h) A 96-well U-bottom plate was coated at 200 μL ($2 \sim 4 \times 10^6$ cells) per well, centrifuged at 400 g, 4°C for 5 min, and the supernatant was discarded;

i) The cells were resuspended by adding 100 µL of culture medium containing a polypeptide ((gE/gI) overlapping polypeptide, which was diluted with FACS solution to reach a final polypeptide concentration of 2 µg/mL), and stimulated for 18 h;

j) 20 µL of Golgi inhibitor (1:1000, diluted with culture medium) was added and incubated for 6h;

k) Centrifugation was performed at 400 g, 4°C for 2 min, the supernatant was discarded, and the cells were added with 200 µL of FACS solution (1×PBS+10% FBS) and resuspended, centrifuged, and the supernatant was discarded;

(All the following operations were formed in the dark and kept at 4°C)

l) Cell surface staining: The cells were stained using FITC-conjugated anti-mouse CD4 antibody (purchased from Biolegend, Cat. No.: 100510), PE-Cy7-conjugated anti-mouse CD8α antibody (purchased from Biolegend, Cat. No.: 100722), and LIVE/DEAD™ fixable Aqua dead cell staining reagent (purchased from Invitrogen, Cat. No.: L34966), resuspended by adding surface antibody (AQUA/CD4/CD8 antibody, diluted with FACS solution) at 40 µL per well, treated at 4°C in the dark for 30 to 60 min, added with 200µL of FACS solution, and pipetted 7 to 8 times;

m) Fixation/Permeabilization: Centrifugation was performed at 2000 rpm, 4°C for 2 min, the supernatant was discarded, and the cells were resuspended by adding Fixation/Permeabilization solution at 75 µL per well, and treated at 4°C in the dark for 60 min;

n) Centrifugation was performed at 2000rpm, 4°C for 2 min, and the supernatant was discarded;

o) The cells were resuspended by adding 1×BD Perm/Wash solution at 200 µL per well, and centrifuged at 2000 rpm and 4°C for 2 min, and the supernatant was discarded;

p) Intracellular staining: The cells were stained using PE-conjugated anti-mouse IL-2 antibody (purchased from BD, Cat. No.: 554428) and APC-conjugated anti-mouse IFN-γ antibody (purchased from BD, Cat. No.: 554413), resuspended by adding fluorescent antibody (IL-2/IFN-γ antibody, diluted with 1×BD Perm/Wash solution) at 50 µL per well, and treated at 4°C in the dark for 60 min, and the cells were resuspended by adding 200 µL of Perm Buffer;

q) Centrifugation was performed at 2000rpm, 4°C for 2 min, the supernatant was discarded, and 200 µL of 1×BD Perm/Wash solution was added to each well to resuspend the cells;

r) The treated cells were filtered with a 200-mesh screen (the screen strips were laid on the surface of the wells, and the cell suspension was pipetted vertically and slowly added by a pipette), and then transferred into a flow cytometer;

s) The samples were measured using a BD LSRFortessa X-20 flow cytometer, and the data were analyzed using FlowJo V10.

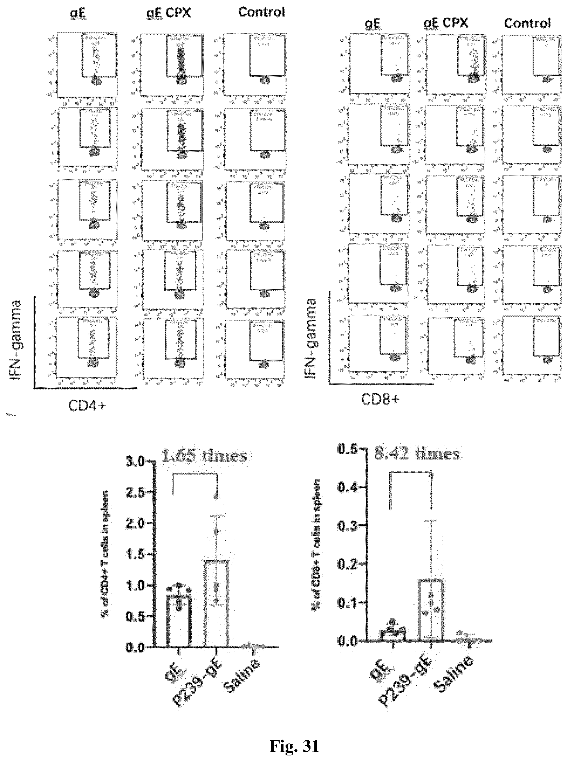

The experimental results were shown in Fig. 31, which showed that the number of IFN-gamma-positive CD4 cells activated by the gE particulate antigen was 1.65 times that of P2-8C-gE monomer, and the number of the activated IFN-gamma-positive CD8 cells was 8.42 times that of P2-8C-gE monomer. The results confirmed that the gE particulate antigen had more advantages in inducing cellular immune response.

Example 14: Enzyme-linked immunospot assay of cytokines in immune group of gE particulate antigen

At week 8, spleen was harvested and underwent flow cytometry and Elispot detection of T cell response, wherein the flow cytometry method was the same as Example 6, and the process of ELISPOT detection of cytokines (the kit used was purchased from MABTECH, Cat. No.: 3321-4HPW-10, 3441-4HPW-10) was as follows:

(1) Splenocytes were separated, and then plated at 500, 000 cells per well, centrifuged to remove the supernatant, and added with 100 µL of gE polypeptide (0.15 µg/100 µl) culture medium to resuspend the cells, and stimulated at 37°C for 20 h;

(2) The culture medium was discarded, and the cells were washed 5 times with sterile PBS, and the detection antibodies R4-6A2-biotin (IFN-$\gamma$) and 5H4-biotin (IL-2) were diluted to 1 $\mu$g/mL, added at 100 $\mu$L per well, and incubated at room temperature for 2 h;

(3) The cells were washed with sterile PBS for 5 times, added with Streptavidin-ALP (1:1000) at 100$\mu$L per well, and incubated at room temperature for 1 h;

(4) The cells were washed with sterile PBS for 5 times, added with substrate solution (BCIP/NBT-plus) at 100 $\mu$L per well for color development until spots appeared, and then washing plate was stopped;

(5) The plate was reversed and dried, and an enzyme-linked spot image analysis system was used for reading and counting spots; and the GraphPad Prism 5 (GraphPad, USA) software was used for data analysis.

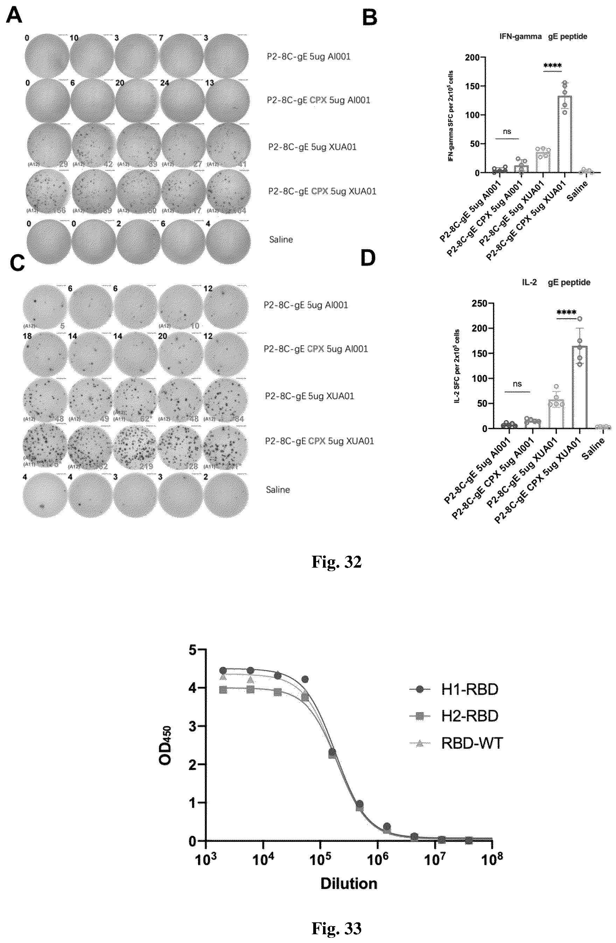

The results were shown in Fig. 32, which confirmed that the T cell immune response activated by the gE particulate antigen was significantly higher than that of the P2-8C-gE monomer protein.

Example 15: Humanization of P1-5B nanobody

The P1-5B nanobody (the full-length amino acid sequence of the P1-5B nanobody was set forth in SEQ ID NO: 12, and the sequences of CDR1 to 3 were set forth in SEQ ID NOs: 57-59) was subjected to CDR alignment using the online software (IMGT/DomainGapAlign; http://www.bioinf.org.uk/abs/abnum/). Homologous sequence alignment retrieval was performed using the online software (https://www.imgt.org/IMGT_vquest/input), and the humanized sequence with the highest score was selected for CDR transplantation. The selected sequence was synthesized by Sangon Biotech and constructed into the PCDNA3.1 vector. The plasmid was prepared in large quantity using an endotoxin-free plasmid large-scale extraction kit (Tiangen Company). Then 293FT cells were transfected by PEI transient transfection to express the humanized nanobody. A total of two humanized nanobodies were obtained, named H1 and H2, respectively. The full-length amino acid sequences of the humanized nanobodies were set forth in SEQ ID NOs: 71 and 72, and the sequences of CDR1 to 3 were set forth in SEQ ID NOs: 65-67.

PEI transient transfection of HEK239FT cells

PCDNA3.1-H1-RBD and PCDNA3.1-H2-RBD plasmids (the plasmids contained nucleotide sequences encoding fusion proteins H1-RBD and H1-RBD, respectively, wherein the amino acid sequences of H1-RBD and H1-RBD were set forth in SEQ ID NOs: 63 and 64) PEI were diluted in 5 ml of serum-free CD05 medium, respectively, and the two were fully mixed and allowed to stand for 18 min to form a plasmid PEI complex, which was used at a ratio of PEI: plasmid = 2:1 to transfect HEK293FT cells. Fresh CD05 medium was used for replacement 6 hours after the transfection. After culture at a constant temperature of 37°C, and 5% $CO_2$ for 48 hours, 100 ml of CD05 medium was supplemented. After 96 hours, the supernatant was harvested for purification, and then the affinity of the humanized nanobodies was detected by enzyme-linked immunosorbent assay (ELISA, see Example 4).

The results were shown in Fig. 33: The modified humanized nanobodies had the same molecular activity as their parents, in which H1-RBD and H2-RBD were RBD fusion proteins formed by modified humanized nanobodies, and RBD-WT was a wild RBD protein.

Example 16: Evaluation of immunogenicity of particulate antigen HEV-Env prepared by humanized nanobodies

White mice: female, 6 weeks old, purchased from Shanghai Silaike Experimental Animal Co., Ltd. Six groups of immunized mice were set up, with 5 mice in each group. In this example, P2-5C nanobody (the amino acid sequence of P2-5C nanobody was set forth in SEQ ID NO: 21, and the sequences of CDR1 to 3 were set forth in SEQ ID NOs: 60-62). The prepared complex particles and P2-5C-BGTSTIP antigen were diluted with physiological saline, mixed with aluminum adjuvant at a volume ratio of 1:1, so that the protein was adsorbed on the adjuvant, and the mice were immunized intramuscularly. The mouse immunization schemes were shown in Table 5.

Table 5: Immunization schemes in mice

| Immunogen | Dose |
|---|---|
| | 0.5$\mu$g |
| P2-5C-BGTSTIP | 5$\mu$g |
| | 50$\mu$g |

(continued)

| Immunogen | Dose |
|---|---|
| P2-5C-BGTSTIP CPX | 0.5μg |
| | 5μg |
| | 50μg |

Blood was collected from eyes of mice before immunization, and the immunization was performed according to the above immunization schemes. Blood was collected from eyes of mice before each immunization. After blood was collected from eyes after the sixth injection, the mice were killed by cervical dislocation. The blood samples were placed at 37°C for 30 min, centrifuged at 13300 rpm for 10 min, and the serum was collected for HIV-1 pseudovirus neutralization and antibody titer determination.

The immune sera of 0 to 6 injections were collected for virus neutralization experiment.

Example 17: Expression and identification of P1F8-BGTSTIP fusion protein

Design and expression of P1F8-BGTSTIP fusion protein

By screening nanobodies against HPV L1 protein (SEQ ID NO: 71), nanobody P1F8 (SEQ ID NO: 88) was obtained. A $(GGGGS)_3$ flexible linker was added after the antibody sequence, and then coupled to the front of the BGTSTIP sequence. The amino acid sequence of the constructed P1F8-BGTSTIP fusion protein was set forth in SEQ ID NO: 89. P1F8-BGTSTIP plasmid was extracted. Frozen 293F cells were taken from -80°C refrigerator, thawed at 37°C, and centrifuged at 1300rpm for 4min, the supernatant was discarded in a clean bench, the cells were flicked and resuspended with 293 freestyle medium incubated at 37°C in advance, transferred to a flask containing 50mL of incubation medium, and suspension cultured at 37°C, 5% $CO_2$ and 120 rpm. When the cell density reached $2.0\times10^6$, the cells were subcultured to gradually expand the culture system. When the cells were sufficient, PEI (MW 25, 000) was used to transiently transfect the 293F cells, the cells were collected in a sterile 50 mL tube, centrifuged at 1300 rpm for 4 min, then the cells were flicked and resuspended with 37°C incubation medium, transferred to a flask containing 450 mL of culture medium incubated at 37°C, and placed on a 37°C shaker for later use.

The extracted P1F8-BGTSTIP plasmid and PEI (MW 25, 000) were placed at a ratio of 1:2 into 50 ml of culture medium, mixed thoroughly, and then allowed to stand for 18 min. The mixture was then transferred to the above 450 ml of culture medium, suspended and cultured at 37°C, 5% $CO_2$ and 120 rpm for 6 days to express the P1F8-BGTSTIP protein. During the transfection process, attention was paid to the light-proof operation of PEI.

Purification of P1F8-BGTSTIP fusion protein

Six days after transient transfection, the cell culture medium was collected, centrifuged at 7000 g for 10 min with a JA-14 rotor, and the cell supernatant was taken. After centrifugation at 20000 g for 10 min, the supernatant was taken and filtered twice with a 0.22 μm pore size filter membrane, and the sample was used for the next step of Ni-excel column purification.

Ni affinity chromatography purification was performed using the AKTA system;

Instrument system: AKTA Pure type preparative liquid chromatograph;

Purification medium: Ni Sepharose excel affinity medium; Buffers: pump A buffer and pump B buffer, in which pump A buffer was 1×PBS buffer, and pump B buffer was 1×PBS + 250 mmol/L imidazole buffer;

System loading flow rate: 8 mL/min; Detection wavelength: UV@280 nm

System elution flow rate: 4 ml/min; Detection wavelength: UV@280 nm

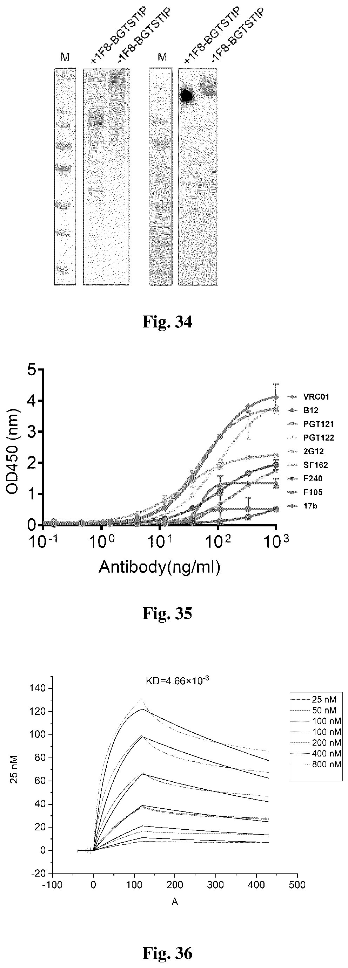

Elution conditions: Protein impurities were eluted with 20 mM imidazole, and 250 mM imidazole elution product was collected. The eluate was dialyzed against 1×PBS overnight, and the dialysate was changed twice during the period. About 30 ml of low-concentration target protein was harvested, concentrated to 5 ml by a Vivaspin 20 ml, 100 KD ultrafiltration concentration tube for later use. The collected eluted samples were prepared into reduced and non-reduced samples, and SDS-PAGE gel electrophoresis was performed according to the above method. Fig. 34 showed the SDS-PAGE results of the P1F8-BGTSTIP protein; M represented molecular weight marker; "+" indicated reducing SDS-PAGE; "-" indicated non-reducing SDS-PAGE. The results showed that the P1F8-BGTSTIP molecule had a molecular weight of about 160 KDa under reducing conditions, and was a polymer under non-reducing conditions, which was consistent with the theoretical molecular weight.

Activity analysis (ELISA) of P1F8-BGTSTIP protein molecule

The fusion protein was diluted to 1 μg/mL and coated on a 96-well plate, 100 μL per well, and allowed to stand at room temperature for 2 h;

(2) The plate was washed once and blocked with bovine serum albumin diluent (ED, 200 μL/well) at room temperature for 2 h;

(3) The plate was washed once, the corresponding specific monoclonal antibody was diluted to 1 μg/mL, 100μl was added to the first well, 3-fold serial dilution was performed, double wells repeat was set, and the plate was allowed to stand at room temperature for 1 h;

(4) The plate was washed 5 times, the secondary antibody GAH-HRP (1:5000) was added to the 96-well plate, 100 μL/well, and the plate was allowed to stand at room temperature for 1 h;

(5) The plate was washed 5 times, color development was performed at room temperature for 10 min, and then terminated, and a microplate reader was used for detection at a wavelength of 450 nm; data analysis was performed using GraphPad Prism 5 (GraphPad, USA) software.

Fig. 35 showed the results of the enzyme-linked immunosorbent assay of P1F8-BGTSTIP. The results showed that P1F8 had good binding activity with neutralizing antibodies VRC01, PGT121, PGT122 (JN201912.1/JN201895.1), 2G12, SF162, and B12, but had very weak binding activity with 17b, F105, and F240, indicating that P1F8-BGTSTIP had well exposed the broad-spectrum neutralizing antibody epitopes, but did not expose the non-neutralizing epitopes.

Affinity analysis (SPR) of P1F8-BGTSTIP and HPV 58VLP

CM5 chip was installed, and the pipeline was rinsed with PBS-P buffer PBS.

Ligand 58VLP (10 μg/mL) was centrifuged at high speed for 10min.

58VLP coupling program was set: The chip channel was activated (EDC:NHS = 1:1, flow rate: 10 μl/min), and then the flushing program (PBS buffer, flow rate: 30 μl/min) was carried out. 58VLP was loaded for coupling (time: 420 s, flow rate: 10 μl/min). After the coupling amount of ligand was determined, ethanolamine was used to perform the channel blocking program (time: 420 s, flow rate: 10 μl/min).

Affinity detection: The fusion protein was set to have concentration gradients of 800 nM, 400 nM, 200 nM, 100 nM, 50 nM, and 25 nM, and loaded for detection. The sample was set to have an association time of 120 s, a dissociation time of 300 s, and a flow rate of 30 μl/min.

Biacore 8K (Cytiva) software was used to fit the association (Ka)/dissociation (Kd) curve by the kinetic method, and the affinity (KD) was analyzed and calculated.
Fig. 36 showed the affinity determination results of P1F8-BGTSTIP with 58VLP. The results showed that the affinity of P1F8-BGTSTIP with the fusion protein was $4.66 \times 10^{-8}$, indicating that the two could bind, and the binding was relatively strong.
Example 18: Preparation and identification of HPV-Env fusion protein (P1F8-BGTSTIP) and HPV 58VLP particle complex

Preparation of P1F8-BGTSTIP complex

P1F8-BGTSTIP and 58VLP were incubated at a mass ratio of 5:1 in a 37°C water bath for 30 min, centrifuged at 13300 rpm for 10 min, and then analyzed by high performance size exclusion chromatography (HPSEC).
Instrument: Waters. The system flow rate for G5000PW$_{XL}$ was 0.5 mL/min. Wavelength was 190 to 600 nm, and column wavelengths were 280 nm and 254 nm.

Buffer: termination buffer.

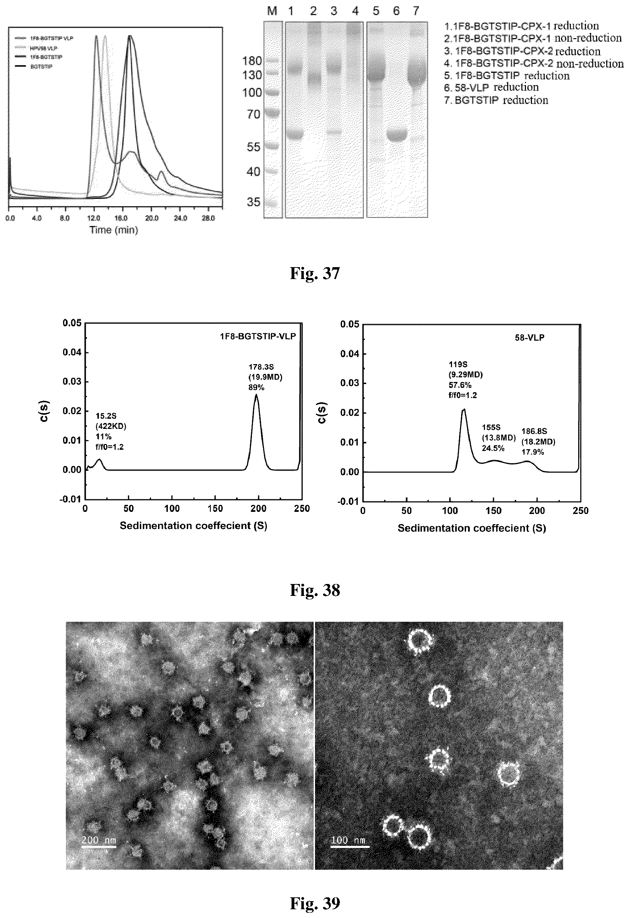

Operation process: The column was pre-equilibrated for 60 min until there was no significant change in the absorption value at 280 nm, and then the absorption value of the detector was returned to zero. The chromatography operation method was edited, the sample to be analyzed was injected into a 100 μL sample loop, automatic loading was set, and the instrument was run for 30 min. Fig. 37 (left) showed the HPSEC chromatograms of individual HPV 58VLP, P1F8-BGTSTIP, BGTSTIP, and P1F8-BGTSTIP-58VLP complexes. The results showed that the peak time of the complexes was 12 min, which was earlier as compared to 58VLP. The complex components were collected and subjected to SDS-PAGE gel electrophoresis. The results showed that the sample collected at 12 min showed a band corresponding to the sum of molecular weights of P1F8-BGTSTIP and 58VLP indicating that a complex was successfully formed by P1F8-BGTSTIP with 58VLP, and its peak time was about 12 min.

Analytical ultracentrifugation (AUC) of particulate antigen P1F8-BGTSTIP-58VLP

The P1F8-BGTSTIP-58VLP complex collected through the above purification process was subjected to AUC analysis.

The instrument used was a Beckman XL-A analytical ultracentrifuge equipped with an optical detection system and an An-60Ti rotor.

The sample pool was installed according to the operating instructions, 400 μL of sample buffer (the same buffer as the sample) was added to the control pool, 380 μL of sample (OD280 was about 0.8) was added to the sample pool, and the sample pool was balance to have a weight difference within 0.1g.

The sample pool was placed into the An-60Ti rotor, the rotor was placed in the cavity of Beckman XL-A analytical ultracentrifuge, and the optical path detector was installed.

Parameter settings: temperature (20°C), Rmin (6.0 cm), Rmax (7.2 cm), wavelength (280 nm), step speed (0.003 cm), scan mode (continuous), data interval (30 sec) and number of data (150 scans). The centrifugal speed for the complex was 7000 rpm and the centrifugal speed for the fusion protein was 30000 rpm.

After the experiment was completed, the density and viscosity of the buffer and the partial specific volume of the known protein were calculated using SENDTERP software. The sedimentation coefficient was analyzed using the Origin version of Nonlin and SEDFIT software, in which the friction ratio of globulin f/f0 was preset to 1.2. The analysis range was set according to the molecular weight and basic properties of the sample protein. The calculation resolution was set to 100. It is generally required that the RMSD value was not greater than 0.01 and the residue map fluctuated within 0.05.

Fig. 38 showed the analytical ultracentrifugation results of the complex of P1F8-BGTSTIP and 58VLP of the present invention. The results showed that 58VLP presented a single component with a sedimentation coefficient of 119 S (Fig. 38, right panel), and the P1F8-BGTSTIP-58VLP complex presented a single component with a sedimentation coefficient of 176.3S (Fig. 38, left panel), which was significantly higher than the sedimentation coefficient of 58VLP particles. The results showed that 1F8-BGTSTIP could form a particle complex with 58VLP and maintain a stable particle morphology in an aqueous solution state.

Observation of morphology of particulate antigen P1F8-BGTSTIP-58VLP by transmission electron microscopy

The transmission electron microscope used was Tecnai G2 Spirit 120 kV (FEI). Negative staining was performed with phosphotungstic acid.

Sample preparation: First, the copper mesh (R2/2, 200 mesh, ThermoFisher Scientific) was subjected to a glow discharge hydrophilic treatment. Then 5 μl of a sample with a concentration of 0.5 mg/ml was dropped on the copper mesh, and the droplet was absorbed from the edge of the copper mesh with absorbent paper after being placed at room temperature for 60 s. After drying at room temperature, the copper mesh was observed on the machine.

Fig. 39 showed the morphology of the complex particles under the transmission electron microscope. It could be clearly seen that the surface of the particles was covered with a layer of protein, indicating that P1F8-BGTSTIP was successfully displayed on the surface of HPV 58VLP particles.

Evaluation of the immunogenicity of particulate antigen P1F8-BGTSTIP-58VLP

This experimental protocol was approved by the Experimental Animal Management Ethics Committee of Xiamen University. All operations were carried out in strict accordance with the animal ethics guidelines and the approved procedures.

Six-week-old Balb/C mice were selected and divided into 6 groups, i.e., 3 high-dose groups and 3 low-dose groups, with 5 mice in each group. P1F8-BGTSTIP-58VLP particles, P1F8-BGTSTIP and BGTSTIP immunogens combined with aluminum adjuvant were used for immunization, and two immunization doses of 0.5μg and 5μg were set for each antigen. The mice were immunized intramuscularly (100μl/mouse) at 0, 2, 4, and 6 weeks.

The eyeball blood of mice was collected before immunization. The eyeball blood of mice was collected before each immunization. After the sixth injection, the mice were killed by cervical dislocation. The blood samples were placed at 37°C for 30 min, centrifuged at 13300rpm for 10 min, and the sera were collected for specific binding antibody titer determination.

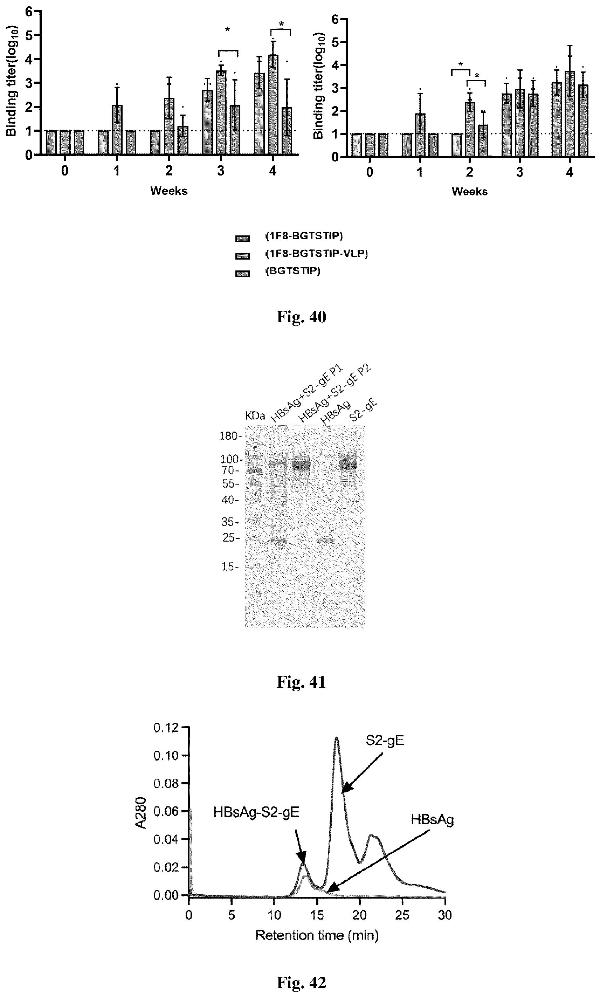

Fig. 40 showed the immunogenicity detection results of particulate antigen P1F8-BGTSTIP-VLP in the example of the present invention. Fig. 40 showed the BGTSTIP-specific binding antibody titer detection results (the left figure represented the 0.5 µg group, and the right figure represented the 5 µg group). The results showed that in weeks 1 to 4, the binding titer of the complex particles was significantly higher than that of the single BGTSTIP and the single P1F8-BGTSTIP protein.

Example 19: Design and evaluation of nanobody fusion protein based on particulate antigen HBsAg VLP of hepatitis B

In this example, VLP carriers were prepared based on the granulatedparticulate antigen SHBs protein (SEQ ID NO: 73) of the HBV vaccine that has been marketed, and gE fusion protein was prepared according to the method of the previous examples, the particulate antigen HBV-gE was further prepared, and the immunogenicity of the particulate antigen was detected. The specific experimental process was as follows:

Design and expression of gE fusion protein

The reported HBsAg nanobody (1.Serruys, B., Houtte, F. V., Verbrugghe, P., Leroux-Roels, G. & Vanlandschoot, P. Llama-derived single-domain intrabodies inhibit secretion of hepatitis B virions in mice. Hepatology 49, 39-49 (2009)) was selected for the experiment. A $(GGGGS)_3$ flexible linker was added after the antibody sequence, and then coupled to the RBD and gE sequences to construct the RBD and gE fusion proteins. The plasmid was extracted, the frozen 293F cells were taken from a -80°C refrigerator, thawed at 37°C, centrifuged at 1300rpm for 4min, the supernatant was discarded in a clean bench, the cells were flicked and resuspended with 293freestyle culture medium incubated at 37°C in advance, transferred to a flask containing 50mL of incubated culture medium, suspended and cultured at 37°C, 5% $CO_2$ and 120 rpm, and subcultured when cell density reached $2.0\times10^6$ to gradually expand the culture system. When the cells were sufficient, PEI (MW 25, 000) was used to transiently transfect 293F cells, the cells were collected in a sterile 50 mL tube, centrifuged at 1300 rpm for 4 min, the cells were flicked and resuspended with culture medium incubated at 37°C, transferred to a flask containing 450 mL of culture medium incubated at 37°C, and placed on a 37°C shaker for later use.

The plasmid and PEI (MW 25, 000) were placed at a ratio of 1:2 in 50 ml of culture medium, mixed thoroughly, then allowed to stand for 18 min, and transferred to the above 450 ml of culture medium, suspended and cultured at 37°C, 5% $CO_2$ and 120 rpm for 6 days to express the gE fusion protein. During the transfection process, attention was paid to the light-proof operation of PEI.

Purification of gE fusion protein

After 6 days of transient transfection, the cell culture medium was collected, centrifuged at 7000g for 10 min in a JA-14 rotor, and the cell supernatant was taken. After centrifugation at 20000g for 10 min, the supernatant was taken and filtered twice with a 0.22 µm pore size filter membrane, and this sample was used for the next step of Ni-excel column purification.

Ni affinity chromatography purification was performed using the AKTA system;

Instrument system: AKTA Pure type preparative liquid chromatograph;

Purification medium: Ni Sepharose excel affinity medium; Buffers: pump A buffer and pump B buffer, in which pump A buffer was 1×PBS buffer, and pump B buffer was 1×PBS+250 mmol/L imidazole buffer;

System loading flow rate: 8 mL/min; Detection wavelength: UV@280 nm

System elution flow rate: 4 ml/min; Detection wavelength: UV@280 nm

Elution conditions: Protein impurities were eluted with 20 mM imidazole, and 250 mM imidazole elution product was collected. The eluate was dialyzed against 1×PBS overnight, and the dialysate was replaced twice during the period. About 30 ml of low-concentration target protein was harvested, and concentrated to 5 ml by a Vivaspin 20 ml, 100 KDa ultrafiltration concentration tube for later use. The collected eluted samples were prepared and subjected to SDS-PAGE gel electrophoresis according to the above method.

The experimental results showed that a fusion protein of a nanobody with a high expression level was obtained, which was named S2-gE. As shown in Fig. 41, the results of SDS-PAGE showed that S2-gE was consistent with the theoretical molecular weight.

Purification of fusion protein and complex thereof by molecular sieve chromatography

Instrument system: AKTA explorer 100 type preparative liquid chromatography system produced by GE Healthcare (formerly Amershan Pharmacia).

Chromatographic medium: Superdex 200 increase (Cytiva).

Column volume: 20 cm×20 mm.

Buffer: 20mM phosphate buffer, pH7.4.

Flow rate: 0.7 mL/min.

Detector wavelength: 280 nm.

The samples were the samples in Example 1 and Example 5.

The elution procedure was: fractionally collecting penetration peak.

The product penetrated Supedex 200 increase was collected to obtain 5 mL of purified sample. 50 μL of each elution product was taken, added with 10 μL of 6X Loading Buffer and mixed well. After being placed in 80°C water bath for 10 min, 10 μl thereof was taken and electrophoresed in 10% SDS-polyacrylamide gel at 120 V voltage for 60 min. Then Coomassie Brilliant Blue staining was performed to show the electrophoresis bands.

As shown in Fig. 42, the results of SDS-PAGE showed that HBsAg and HBsAg-S2-gE (peak of complex of HBsAg and S2-gE) absorbed different peaks, and the particle peak of HBsAg-S2-gE became larger and had a shorter retention time. It showed that S2-gE could form a particulate antigen with HBsAg.

Particle size detection (DLS) of HBV-gE complex molecule

Instrument: NanoBrook Series (Brookhaven instrument).

Functional module: DLS (Dynamic Light Scattering).

Buffer: PBS.

Operation process: The instrument was turned on and preheated for 5 min in advance. The sample to be tested was prepared (concentration: 0.5 mg/ml, centrifugation: 12000 rpm for 5 min, 50 μl was taken and added to the sample cup). Detection parameter settings: Detection time: 300 s each time, 3 repeats for each sample.

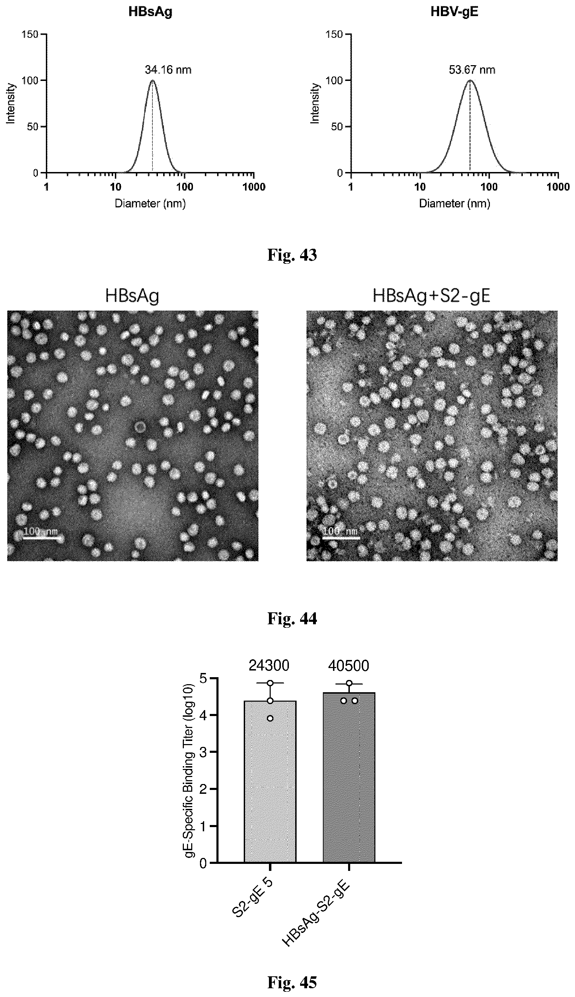

As shown in Fig. 43, the DLS results showed that the particle size of single HBsAg was different from that of the HBsAg-S2-gE complex. The particle size of HBsAg-S2-gE (HBV-gE) was significantly larger than that of HBsAg, indicating the formation of a gE particulate antigen.

Observation of particle morphology by transmission electron microscopy

The transmission electron microscope used was Tecnai G2 Spirit 120 kV (FEI). Negative staining was performed with phosphotungstic acid.

Sample preparation: First, the copper mesh (R2/2, 200 mesh, ThermoFisher Scientific) was subjected to glow discharge hydrophilic treatment. Then, 5 μl of the sample with a concentration of 0.5 mg/ml was dropped on the copper mesh. After standing at room temperature for 60 s, the droplet was absorbed from the edge of the copper mesh with absorbent paper. After drying at room temperature, the copper mesh was observed on the machine.

As shown in Fig. 44, the negative staining results showed that the size and morphology of single HBsAg were different from those of the HBsAg-S2-gE complex, indicating the formation of a gE particulate antigen.

Evaluation of immunogenicity of gE particle

This experimental protocol was approved by the Experimental Animal Management Ethics Committee of Xiamen University. All operations were carried out in strict accordance with the animal ethics guidelines and the approved procedures.

Six-week-old Balb/C mice were selected and divided into two groups, with 3 mice for each group. Each group was immunized with the HBsAg-gE complex prepared above and single S2-gE, in which the gE dose of both was 5 μg, and intramuscular injection (50 μL) was carried out in the left or right hind limb of mice at 0 and 2 weeks, respectively. Ocular venous blood was collected at 0, 1, 2, 3, and 4 weeks, respectively. The blood samples were centrifuged at 13000 g for 10 min, and the serum samples were stored at -20°C. The antigen-specific IgG titer was determined by end-point enzyme-linked immunosorbent assay.

As shown in Fig. 45, the gE-specific titer results after two injections for immunization showed that the particulate antigen HBsAg-S2-gE could induce a significantly higher antibody level than single S2-gE, and had an immune enhancement effect.

**Claims**

1. A fusion protein, comprising an immunogenic polypeptide and a nanobody capable of specifically binding to a virus-like particle (VLP);

   preferably, the VLP is a VLP assembled from an assembly polypeptide.

2. The fusion protein according to claim 1, wherein the assembly polypeptide is a polypeptide capable of being assembled into a VLP;

   preferably, the assembly polypeptide is a capsid protein of a natural virus or a viroid, or is an artificially prepared and/or screened polypeptide.
   preferably, the assembly polypeptide is selected from the group consisting of protein of hepatitis E virus (HEV) or fragment thereof or variant thereof, protein of hepatitis B virus (HBV) or fragment thereof or variant thereof, protein of human papillomavirus (HPV) or fragment thereof or variant thereof, and any combination thereof; wherein the fragment or variant retains the ability to assemble into VLP;
   preferably, the assembly polypeptide is an ORF2 protein of HEV or fragment thereof or variant thereof; preferably, the fragment of ORF2 protein is selected from the group consisting of p239 protein, and p495 protein;
   preferably, the assembly polypeptide is selected from the group consisting of p239 protein or fragment thereof or variant thereof, and p495 protein or fragment thereof or variant thereof;
   preferably, the variant has a substitution, deletion or addition of one or several amino acids (e.g., a substitution, deletion or addition of 1, 2 or 3 amino acids) as compared to the sequence of the protein from which it is derived;
   preferably, the substitution is a conservative substitution;
   preferably, the ORF2 protein has an amino acid sequence as set forth in SEQ ID NO: 9; preferably, the p239 protein has an amino acid sequence as set forth in SEQ ID NO: 40; preferably, the p495 protein has an amino acid sequence as set forth in SEQ ID NO: 41;
   preferably, the assembly polypeptide is a capsid protein L1 of HPV or fragment thereof or variant thereof;
   preferably, the assembly polypeptide has an amino acid sequence as set forth in SEQ ID NO: 74;
   preferably, the assembly polypeptide is a surface protein of HBV (e.g., hepatitis B virus surface antigen) or fragment thereof or variant thereof;
   preferably, the assembly polypeptide is selected from the group of LHBs protein or fragment thereof or variant thereof, MHBs protein or fragment thereof or variant thereof, and SHBs protein or fragment thereof or variant thereof of hepatitis B virus surface antigen (HBsAg);
   preferably, the assembly polypeptide is a SHBs protein or fragment thereof or variant thereof of hepatitis B virus surface antigen (HBsAg);
   preferably, the assembly polypeptide has an amino acid sequence as set forth in SEQ ID NO: 73.

3. The fusion protein according to claim 1 or 2, wherein the immunogenic polypeptide is a polypeptide or immunogenic variant thereof derived from an organism or a non-organism (e.g., artificially synthesized);

   preferably, the organism is a pathogen (e.g., virus, bacteria, fungus, parasite) or a non-pathogen;
   preferably, the immunogenic polypeptide is obtained from a tumor cell of a mammal (e.g., human); preferably, the immunogenic polypeptide is selected from the group consisting of carcinoembryonic antigen (CEA), α-fetoprotein (AFP), and cancer antigen 125 (CA125);
   preferably, the immunogenic polypeptide is obtained from a virus, a bacterium (e.g., *Mycobacterium tuberculosis*), a fungus (e.g., Nostoc), or a parasite (e.g., *Plasmodium falciparum*);
   preferably, the virus is selected from the group consisting of varicella zoster virus (VZV), Severe Acute Respiratory Syndrome Coronavirus 2 (SARS-CoV-2), coronavirus (SARS-COV-1), human immunodeficiency virus type I (HIV-1), human papillomavirus, hepatitis B virus, hepatitis A virus, hepatitis C virus, hepatitis E virus, measles virus, mumps virus, influenza virus, and encephalitis B virus;

preferably, the immunogenic polypeptide is selected from the group consisting of RBD protein of SARS-CoV-2 or immunogenic fragment thereof or variant thereof, Env protein of HIV-1 or immunogenic fragment thereof (e.g., gp140, gp160) or variant thereof, and gE protein of VZV or immunogenic fragment thereof or variant thereof;

preferably, the variant has a substitution, deletion or addition of one or several amino acids (e.g., a substitution, deletion or addition of 1, 2 or 3 amino acids) as compared to the sequence of the protein from which it is derived;

preferably, the substitution is a conservative substitution;

preferably, the RBD protein has an amino acid sequence as set forth in any one of SEQ ID NOs: 1-8;

preferably, the Env protein has an amino acid sequence as set forth in SEQ ID NO: 34 or 35;

preferably, the gE protein has an amino acid sequence as set forth in SEQ ID NO: 30.

4. The fusion protein according to any one of claims 1 to 3, wherein the nanobody is a camel-derived (e.g., alpaca-derived) antibody or a fish-derived (e.g., shark-derived) antibody;

preferably, the nanobody is a chimeric antibody, a humanized antibody or a fully human antibody;

preferably, the fusion protein comprises 2, 3, or more kinds of immunogenic polypeptides;

preferably, each kind of immunogenic polypeptide is independently obtained from the same or different pathogens (e.g., viruses);

preferably, each kind of immunogenic polypeptide is a different polypeptide obtained from the same pathogen (e.g., virus);

preferably, the fusion protein comprises one kind of immunogenic polypeptide;

preferably, the nanobody is a nanobody that specifically binds to a polypeptide of HEV, HBV and/or HPV;

more preferably, the nanobody comprises CDR-H1, CDR-H2 and CDR-H3 as contained in a heavy chain variable region (VHH) as set forth in any one of SEQ ID NOs: 10-29, 68, 69; preferably, the CDRs are defined according to the IMGT, Kabat or Chothia numbering system;

preferably, the nanobody comprises:

(a) a heavy chain variable region (VHH) comprising the following 3 complementary determining regions (CDRs): a VH CDR1 with a sequence as set forth in SEQ ID NO: 90, a VH CDR2 with a sequence as set forth in SEQ ID NO: 91, and a VH CDR3 with a sequence as set forth in SEQ ID NO: 92;

(b) a heavy chain variable region (VHH) comprising the following 3 complementary determining regions (CDRs): a VH CDR1 with a sequence as set forth in SEQ ID NO: 93, a VH CDR2 with a sequence as set forth in SEQ ID NO: 94, and a VH CDR3 with a sequence as set forth in SEQ ID NO: 95;

(c) a heavy chain variable region (VH) comprising the following 3 complementary determining regions (CDRs): a VH CDR1 as set forth in SEQ ID NO: 96, a VH CDR2 as set forth in SEQ ID NO: 97, and a VH CDR3 as set forth in SEQ ID NO: 98;

(d) a heavy chain variable region (VH) comprising the following 3 complementary determining regions (CDRs): a VH CDR1 as set forth in SEQ ID NO: 99, a VH CDR2 as set forth in SEQ ID NO: 100, and a VH CDR3 as set forth in SEQ ID NO: 101;

(e) a heavy chain variable region (VH) comprising the following 3 complementary determining regions (CDRs): a VH CDR1 as set forth in SEQ ID NO: 102, a VH CDR2 as set forth in SEQ ID NO: 103, and a VH CDR3 as set forth in SEQ ID NO: 104;

(f) a heavy chain variable region (VH) comprising the following 3 complementary determining regions (CDRs): a VH CDR1 as set forth in SEQ ID NO: 105, a VH CDR2 as set forth in SEQ ID NO: 106, and a VH CDR3 as set forth in SEQ ID NO: 107;

(g) a heavy chain variable region (VH) comprising the following 3 complementary determining regions (CDRs): a VH CDR1 as set forth in SEQ ID NO: 108, a VH CDR2 as set forth in SEQ ID NO: 109, and a VH CDR3 as set forth in SEQ ID NO: 110;

(h) a heavy chain variable region (VH) comprising the following 3 complementary determining regions (CDRs): a VH CDR1 as set forth in SEQ ID NO: 111, a VH CDR2 as set forth in SEQ ID NO: 112, and a VH CDR3 as set forth in SEQ ID NO: 113;

(i) a heavy chain variable region (VH) comprising the following 3 complementary determining regions (CDRs): a VH CDR1 as set forth in SEQ ID NO: 114, a VH CDR2 as set forth in SEQ ID NO: 115, and a VH CDR3 as set forth in SEQ ID NO: 116;

(j) a heavy chain variable region (VH) comprising the following 3 complementary determining regions (CDRs): a VH CDR1 as set forth in SEQ ID NO: 117, a VH CDR2 as set forth in SEQ ID NO: 118, a VH CDR3 as set forth in SEQ ID NO: 119;

(k) a heavy chain variable region (VH) comprising the following 3 complementary determining regions (CDRs): a VH CDR1 as set forth in SEQ ID NO: 120, a VH CDR2 as set forth in SEQ ID NO: 121, a VH CDR3 as set forth in SEQ ID NO: 122;

(l) a heavy chain variable region (VH) comprising the following 3 complementary determining regions (CDRs): a VH CDR1 as set forth in SEQ ID NO: 123, a VH CDR2 as set forth in SEQ ID NO: 124, a VH CDR3 as set forth in SEQ ID NO: 125;

(m) a heavy chain variable region (VH) comprising the following 3 complementary determining regions (CDRs): a VH CDR1 as set forth in SEQ ID NO: 126, a VH CDR2 as set forth in SEQ ID NO: 127 CDR2, a VH CDR3 as set forth in SEQ ID NO: 128;

(n) a heavy chain variable region (VH) comprising the following 3 complementary determining regions (CDRs): a VH CDR1 as set forth in SEQ ID NO: 129, a VH CDR2 as set forth in SEQ ID NO: 130, and a VH CDR3 as set forth in SEQ ID NO: 131;

(o) a heavy chain variable region (VH) comprising the following 3 complementary determining regions (CDRs): a VH CDR1 as set forth in SEQ ID NO: 132, a VH CDR2 as set forth in SEQ ID NO: 133, and a VH CDR3 as set forth in SEQ ID NO: 134;

(p) a heavy chain variable region (VH) comprising the following 3 complementary determining regions (CDRs): a VH CDR1 as set forth in SEQ ID NO: 135, a VH CDR2 as set forth in SEQ ID NO: 136, and a VH CDR3 as set forth in SEQ ID NO: 137;

(q) a heavy chain variable region (VH) comprising the following 3 complementary determining regions (CDRs): a VH CDR1 as set forth in SEQ ID NO: 138, a VH CDR2 as set forth in SEQ ID NO: 139, and a VH CDR3 as set forth in SEQ ID NO: 140;

(r) a heavy chain variable region (VH) comprising the following 3 complementary determining regions (CDRs): a VH CDR1 as set forth in SEQ ID NO: 141, a VH CDR2 as set forth in SEQ ID NO: 142, and a VH CDR3 as set forth in SEQ ID NO: 143;

(s) a heavy chain variable region (VH) comprising the following 3 complementary determining regions (CDRs): a VH CDR1 as set forth in SEQ ID NO: 57, a VH CDR2 as set forth in SEQ ID NO: 58, and a VH CDR3 as set forth in SEQ ID NO: 59;

(t) a heavy chain variable region (VH) comprising the following 3 complementary determining regions (CDRs): a VH CDR1 as set forth in SEQ ID NO: 60, a VH CDR2 as set forth in SEQ ID NO: 61, and a VH CDR3 as set forth in SEQ ID NO: 62; or

(u) a heavy chain variable region (VH) comprising the following 3 complementary determining regions (CDRs): a VH CDR1 as set forth in SEQ ID NO: 65, a VH CDR2 as set forth in SEQ ID NO: 66, and a VH CDR3 as set forth in SEQ ID NO: 67;

more preferably, the nanobody comprises a sequence as set forth in any one of SEQ ID NOs: 10-29, 68, 69 or a variant thereof; wherein the variant is capable of specifically binding to the assembly polypeptide and has a substitution, deletion or addition of one or more amino acids (e.g., a substitution, deletion or addition of 1, 2 or 3 amino acids) as compared to the sequence from which it is derived; preferably, the substitution is a conservative substitution.

5. The fusion protein according to any one of claims 1 to 4, wherein the fusion protein further comprises a linker;

preferably, the linker is a polypeptide, such as a flexible peptide or a rigid peptide;
preferably, the linker comprises one or several (e.g., 1, 2 or 3) sequences represented by $(G_mS)_n$, wherein m is an integer selected from 1 to 6, and n is integer selected from 1 to 6; preferably, m is 3, 4, or 5; preferably, n is 2, 3 or 4;
more preferably, the linker has an amino acid sequence as set forth in SEQ ID NO: 39;
preferably, the immunogenic polypeptide and nanobody of the fusion protein are directly connected or connected through the linker;
preferably, the immunogenic polypeptide is located at the N-terminal or C-terminal of the fusion protein;
preferably, the fusion protein comprises, from the N-terminal to the C-terminal, the immunogenic polypeptide and the nanobody; or, the nanobody and the immunogenic polypeptide; or, the immunogenic polypeptide, the linker and the nanobody; or, the nanobody, the linker and the immunogenic polypeptide.

6. A fusion protein according to any one of claims 1 to 5, wherein the fusion protein further comprises a signal peptide and/or a tag;

preferably, the signal peptide has an amino acid sequence as set forth in SEQ ID NO: 31, 37 or 38;
preferably, the tag is a tag for purification, such as a His tag or a GST tag;
preferably, the signal peptide is located at the N-terminal of the fusion protein;

preferably, the tag is located at the C-terminal of the fusion protein;

preferably, the fusion protein has an amino acid sequence as set forth in any one of SEQ ID NOs: 42-49, 50-56, 72-79, 80-87, 63, 64, 89.

7. An isolated nucleic acid molecule, comprising a nucleotide sequence encoding the fusion protein according to any one of claims 1 to 6;

preferably, the nucleotide sequence is codon-optimized or not optimized according to the codon preference of a host cell.

8. A vector, comprising the isolated nucleic acid molecule according to claim 7; preferably, the vector is used to express (e.g., in a cell in vitro) a protein encoded by the isolated nucleic acid molecule.

9. A host cell, comprising the nucleic acid molecule according to claim 7 or the vector according to claim 8;

preferably, the host cell is selected from the group consisting of prokaryotic cell and eukaryotic cell;

preferably, the prokaryotic cell is selected from the group consisting of *Escherichia coli* cell and *Bacillus subtilis* cell;

preferably, the eukaryotic cell is selected from the group consisting of yeast cell, insect cell, plant cell and animal cell;

preferably, the animal cell is a mammalian cell (e.g., a mouse cell, a human cell).

10. A method for expressing or producing the fusion protein according to any one of claims 1 to 6, the method comprising culturing the host cell according to claim 9 under a condition that allows protein expression, and optionally, recovering or purifying the expressed fusion protein.

11. A composition, comprising at least one kind of the fusion protein according to any one of claims 1 to 6;

preferably, the composition further comprises an assembly polypeptide;

preferably, the assembly polypeptide is present in the form of VLP;

preferably, the fusion protein is attached to the VLP.

12. A kit, comprising: the fusion protein according to any one of claims 1 to 6 or a first nucleic acid molecule containing a nucleotide sequence encoding the fusion protein, and an assembly polypeptide or a second nucleic acid molecule containing a nucleotide sequence encoding the assembly polypeptide;

preferably, the nucleotide sequence is codon-optimized or not optimized according to the codon preference of a host cell;

preferably, the fusion protein or the first nucleic acid molecule and the assembly polypeptide or the second nucleic acid molecule are provided separately or in the form of a composition;

preferably, the kit further comprises a vector (e.g., an expression vector); preferably, the first nucleic acid molecule and the second nucleic acid molecule are contained in the same or different vectors;

optionally, the kit further comprises a buffer;

preferably, the buffer is selected from the group consisting of phosphate buffer, citrate buffer, carbonate buffer, acetate buffer, barbituric acid buffer, Tris buffer, and any combination thereof;

preferably, the buffer is a PBS buffer;

preferably, the buffer further comprises a salt;

preferably, the salt is selected from the group consisting of $NaCl$, $(NH_4)SO_4$, $NaSO_4$, $NH_4Cl$, and any combination thereof.

13. A particulate antigen, comprising an assembly polypeptide in the form of a VLP, and the fusion protein according to any one of claims 1 to 6 attached to the assembly polypeptide;

preferably, the fusion protein is attached to the VLP through the interaction of between the nanobody and the assembly polypeptide;

preferably, the VLP is attached to at least one kind of the fusion protein according to any one of claims 1 to 6;

preferably, the VLP is also attached to additional polypeptide or fusion protein (e.g., a T cell epitope).

14. A method for preparing the particulate antigen according to claim 13, the method comprising: using the kit according to claim 12;
preferably, the method comprises: contacting the assembly polypeptide with the fusion protein under a condition that allows VLP assembly; optionally, recovering or purifying the particulate antigen in a buffer.

15. A vaccine, comprising the fusion protein according to any one of claims 1 to 6, or the composition according to claim 11, or the particulate antigen according to claim 13, and an adjuvant;
preferably, the adjuvant is selected from the group consisting of aluminum salt adjuvant, zinc-aluminum mixed adjuvant (e.g., FH002C), Freund's adjuvant, oil emulsion adjuvant, cytokine, TLR agonist, CpG adjuvant, liposome, AS01B adjuvant, and any combination thereof.

16. A pharmaceutical composition, comprising any one or more of (1) to (6):

(1) the fusion protein according to any one of claims 1 to 6;
(2) the nucleic acid molecule according to claim 7;
(3) the vector according to claim 8;
(4) the host cell according to claim 9;
(5) the composition according to claim 11;
(6) the particulate antigen according to claim 13;

optionally, the pharmaceutical composition further comprises a pharmaceutically acceptable carrier and/or excipient.

17. Use of the fusion protein according to any one of claims 1 to 6, or the nucleic acid molecule according to claim 7, or the vector according to claim 8, or the host cell according to claim 9, or the composition according to claim 11, or the kit according to claim 12, or the particulate antigen according to claim 13, in the manufacture of a pharmaceutical composition or vaccine, wherein the pharmaceutical composition or vaccine is used to induce an immune response in a subject;

preferably, the immune response is a response to an immunogenic polypeptide; preferably, the immune response is a T cell response (e.g., a CD4+ response or a CD8+ response); preferably, the immune response is a B cell response;
preferably, the subject is a mammal, such as a human, a monkey or a mouse.

18. Use of the fusion protein according to any one of claims 1 to 6, or the nucleic acid molecule according to claim 7, or the vector according to claim 8, or the host cell according to claim 9, or the composition according to claim 11, or the kit according to claim 12, or the particulate antigen according to claim 13, in the manufacture of a pharmaceutical composition or vaccine, wherein the pharmaceutical composition or vaccine is used for preventing and/or treating a disease and/or symptom that benefits or is prevented by an immune response to an immunogenic polypeptide in a subject;

preferably, the disease and/or symptom is caused by a tumor cell or pathogen (e.g., virus, bacteria, fungus, parasite) from which the immunogenic polypeptide is derived;
preferably, the disease and/or symptom is caused by a virus from which the immunogenic polypeptide is derived, for example, chickenpox, COVID-19, AIDS, condyloma acuminatum, viral hepatitis (e.g., viral hepatitis B, viral hepatitis A, viral hepatitis C, viral hepatitis E), measles, mumps;
preferably, the subject is a mammal, such as a human, a monkey or a mouse.

19. A method for inducing an immune response in a subject, comprising administering to the subject an effective amount of the fusion protein according to any one of claims 1 to 6, or the nucleic acid molecule according to claim 7, or the vector according to claim 8, or the host cell according to claim 9, or the composition according to claim 11, or the kit according to claim 12, or the particulate antigen according to claim 13, or the vaccine according to claim 15, or the pharmaceutical composition according to claim 16;

preferably, the immune response is a response to an immunogenic polypeptide; preferably, the immune response is a T cell response (e.g., a CD4+ response or a CD8+ response); preferably, the immune response is a B cell response;
preferably, the subject is a mammal, such as a human, a monkey or a mouse.

20. A method for preventing and/or treating a disease and/or symptom in a subject that benefits or is prevented by an immune response to an immunogenic polypeptide, comprising administering to the subject an effective amount of the fusion protein according to any one of claims 1 to 6, or the nucleic acid molecule according to claim 7, or the vector according to claim 8, or the host cell according to claim 9, or the composition according to claim 11, or the kit according to claim 12, or the particulate antigen according to claim 13, or the vaccine according to claim 15, or the pharmaceutical composition according to claim 16;

preferably, the disease and/or symptom is caused by a tumor cell or pathogen (e.g., virus, bacteria, fungus, parasite) from which the immunogenic polypeptide is derived;

preferably, the disease and/or symptom is caused by a virus from which the immunogenic polypeptide is derived, for example, chickenpox, COVID-19, AIDS, condyloma acuminatum, viral hepatitis (e.g., viral hepatitis B, viral hepatitis A, viral hepatitis C, viral hepatitis E), measles, mumps;

preferably, the subject is a mammal, such as a human, a monkey or a mouse.

21. A system for preparing a particulate immunogenic polypeptide, comprising a first vector and a second vector, wherein the first vector comprises a nucleotide sequence encoding a fusion protein, the fusion protein comprises the immunogenic polypeptide and an assembly polypeptide, and the second vector comprises a nucleotide sequence encoding a nanobody; and the nanobody is capable of specifically binding to the assembly polypeptide, and the assembly polypeptide is capable of being assembled into a VLP;

preferably, the nucleotide sequence is codon-optimized or not optimized according to the codon preference of a host cell;

preferably, the assembly polypeptide is selected from protein of hepatitis E virus (HEV) or fragment thereof or variant thereof; preferably, the assembly polypeptide is as defined in claim 2;

preferably, the fusion protein is as defined in any one of claims 1 to 6.

22. A method for enhancing an immunogenicity of an immunogenic polypeptide, comprising: preparing or obtaining a fusion protein comprising the immunogenic polypeptide and a nanobody capable of specifically binding to an assembly polypeptide; and, contacting the fusion protein with a VLP comprising the assembly polypeptide, thereby obtaining a particulate antigen comprising the immunogenic polypeptide attached to the VLP;

preferably, the method comprising: using the system according to claim 21; preferably, the method comprising: (1) expressing or producing the fusion protein by a first vector, and expressing or producing the assembly polypeptide by a second vector; (2) contacting the fusion protein and the assembly polypeptide under a condition that allows VLP assembly;

preferably, the nanobody is as defined in claim 4;

preferably, the fusion protein is as defined in any one of claims 1 to 6;

preferably, the assembly polypeptide is assembled into the VLP; preferably, the fusion protein is attached to the VLP through the interaction between the nanobody and the assembly polypeptide.

Fig. 1A

Fig. 1B

Fig. 2A

Fig. 2B

Fig. 3

**Fig. 4**

**Fig. 5A**

**Fig. 5B**

**Fig. 6**

**Fig. 7A**

Fig. 7B

Fig. 8

Fig. 9

**Fig. 10A**

Fig. 10B

**Fig. 11**

──3.125 nM    ──6.25 nM    ──12.5 nM    ──25 nM
──50 nM      ──100 nM    ──200 nM    ──400 nM

**Fig. 12**

Fig. 13A

Fig. 13B

**Fig. 14A**

**Fig. 14B**

Fig. 15

Fig. 16

Fig. 17

**Fig. 18A**

**Fig. 18B**

**Fig. 19**

239

P2-5C-BGTSTIP-239

Radius: 15.9 nm

Baseline index: 8.0

Polydispersity: 0.166

Radius: 25.2 nm

Baseline index: 8.7

Polydispersity: 0.072

**Fig. 20**

22 S

27 S

**Fig. 21A**

P2-3E-RBD Complex

32 S

P2-6D-RBD Complex

38 S

P2-10G-RBD Complex

38 S

P239

22 S

**Fig. 21B**

**Fig. 22**

**Fig. 23**

**Fig. 24A**

HEV P239

HEV P239&P2-10G-RBD Complex      HEV P239&P2-3E-RBD Complex

**Fig. 24B**

**Fig. 25**

**Fig. 26**

Fig. 27A

**P2-3E-RBD 0.5 µg**

**P2-3E-RBD 0.5ug Line**

**P2-10G-RBD 0.5 µg**

Fig. 27B

**P2-10G-RBD 0.5ug**

**P2-6D-RBD 0.5 µg**

**P2-6D-RBD 0.5ug**

Fig. 27B (Continued)

## P2-3E-RBD 5 µg

## P2-3E-RBD 5ug

## P2-10G-RBD 5 µg

Fig. 27B (Continued)

**P2-10G-RBD 5ug**

**P2-6D-RBD 5 µg**

**P2-6D-RBD 5ug**

Fig. 27B (End)

**VHH-RBD neutralization detection**

Fig. 27C

Fig. 27D

SARS-Cov-2(Gamma)

SARS-Cov-2 (BA.2)

SARS-Cov-2 (WT)

Fig. 28

Fig. 29

XUA01

Fig. 30

Fig. 31

Fig. 32

Fig. 33

**Fig. 34**

**Fig. 35**

**Fig. 36**

1. 1F8-BGTSTIP-CPX-1 reduction
2. 1F8-BGTSTIP-CPX-1 non-reduction
3. 1F8-BGTSTIP-CPX-2 reduction
4. 1F8-BGTSTIP-CPX-2 non-reduction
5. 1F8-BGTSTIP reduction
6. 58-VLP reduction
7. BGTSTIP reduction

**Fig. 37**

**Fig. 38**

**Fig. 39**

**Fig. 40**

**Fig. 41**

**Fig. 42**

**HBsAg**

**HBV-gE**

**Fig. 43**

HBsAg

HBsAg+S2-gE

**Fig. 44**

**Fig. 45**

## INTERNATIONAL SEARCH REPORT

| International application No. |
|---|
| **PCT/CN2023/129973** |

### A. CLASSIFICATION OF SUBJECT MATTER

A61K39/12(2006.01)i; A61K39/29(2006.01)i; A61P31/00(2006.01)i; C12N15/86(2006.01)i

According to International Patent Classification (IPC) or to both national classification and IPC

### B. FIELDS SEARCHED

Minimum documentation searched (classification system followed by classification symbols)

IPC:A61K,A61P,C12N

Documentation searched other than minimum documentation to the extent that such documents are included in the fields searched

Electronic data base consulted during the international search (name of data base and, where practicable, search terms used)

EPTXT; VEN; USTXT; WOTXT; CNABS; GOOGLE SCHOLAR; PUBMED; ISI WEB OF SCIENCE; CNKI; 万方, WANFANG; GENBANK; STN: 表面抗原, 病毒样颗粒, 抗体, 抗原, 免疫原, 纳米抗体, 融合蛋白, 衣壳蛋白, 展示, 组装, 组装多肽, antibody, fusion, surface antigen, ORF2, nanobody, display, particle, HEV, HBV, HPV, LHBs, MHBs, p239, p495, SHBs, VLP, SEQ ID NOs: 10-29, 57-68, 69, 90-143

### C. DOCUMENTS CONSIDERED TO BE RELEVANT

| Category* | Citation of document, with indication, where appropriate, of the relevant passages | Relevant to claim No. |
|---|---|---|
| Y | WO 2022207645 A1 (VIRAVAXX AG) 06 October 2022 (2022-10-06) claim 1, and description, page 4, line 20 to page 6, line 8, and page 19, lines 5-10 | 1-22 |
| Y | CA 3149320 A1 (THE HOSPITAL FOR SICK CHILDREN et al.) 04 February 2021 (2021-02-04) description, page 1, line 20 to page 2, line 30 | 1-22 |
| Y | US 2013302371 A1 (STC.UNM) 14 November 2013 (2013-11-14) description, paragraphs 10-11 | 1-22 |
| A | CN 104031144 A (XIAMEN UNIVERSITY et al.) 10 September 2014 (2014-09-10) entire document | 1-22 |
| A | CN 113557431 A (EUROIMMUN MEDIZINISCHE LABORDIAGNOSTIKA AG et al.) 26 October 2021 (2021-10-26) entire document | 1-22 |
| A | US 2012015000 A1 (LANAR, D. et al.) 19 January 2012 (2012-01-19) entire document | 1-22 |

☑ Further documents are listed in the continuation of Box C.    ☑ See patent family annex.

| | |
|---|---|
| * Special categories of cited documents: <br> "A" document defining the general state of the art which is not considered to be of particular relevance <br> "D" document cited by the applicant in the international application <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
|---|---|
| **08 December 2023** | **14 December 2023** |

| Name and mailing address of the ISA/CN | Authorized officer |
|---|---|
| **China National Intellectual Property Administration (ISA/ CN)** <br> **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
| --- |
| **PCT/CN2023/129973** |

| C. | DOCUMENTS CONSIDERED TO BE RELEVANT | |
| --- | --- | --- |
| Category* | Citation of document, with indication, where appropriate, of the relevant passages | Relevant to claim No. |
| A | US 2017056493 A1 (YALE UNIVERSITY) 02 March 2017 (2017-03-02)<br>    entire document | 1-22 |
| A | WO 2014205491 A1 (MONASH UNIVERSITY et al.) 31 December 2014 (2014-12-31)<br>    entire document | 1-22 |
| A | 杨珂 等 (YANG, Ke et al.). "纳米抗体及其应用 (Non-official translation: Nanobody and Application thereof)"<br>细胞与分子免疫学杂志 *(Chinese Journal of Cellular and Molecular Immunology),*<br>Vol. 24, No. (4), 31 December 2008 (2008-12-31),<br>    pages 425-427 | 1-22 |
| A | PEYRET, H. et al. "Tandem Fusion of Hepatitis B Core Antigen Allows Assembly of Virus-Like Particles in Bacteria and Plants with Enhanced Capacity to Accommodate Foreign Proteins"<br>*PLoS One,* Vol. 10, No. (4), 01 April 2015 (2015-04-01),<br>    Article number: e0120751 | 1-22 |

Form PCT/ISA/210 (second sheet) (July 2022)

## INTERNATIONAL SEARCH REPORT

| International application No. |
| --- |
| **PCT/CN2023/129973** |

**Box No. I      Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

   a.  ☑  forming part of the international application as filed.

   b.  ☐  furnished subsequent to the international filing date for the purposes of international search (Rule 13*ter*.1(a)),

   ☐  accompanied by a statement to the effect that the sequence listing does not go beyond the disclosure in the international application as filed.

2.  ☐  With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

## EP 4 628 095 A1

### INTERNATIONAL SEARCH REPORT

| International application No. |
|---|
| **PCT/CN2023/129973** |

**Box No. II**      **Observations where certain claims were found unsearchable (Continuation of item 2 of first sheet)**

This international search report has not been established in respect of certain claims under Article 17(2)(a) for the following reasons:

1. ☑ Claims Nos.: **19-20**
    because they relate to subject matter not required to be searched by this Authority, namely:

    Claims 19-20 relate to a method for inducing an immune response in a subject, which falls within methods for treatment of diseases as defined in PCT Rule 39.1(iv); however, a search is still performed on the basis of the pharmaceutical use thereof.

2. ☐ Claims Nos.:
    because they relate to parts of the international application that do not comply with the prescribed requirements to such an extent that no meaningful international search can be carried out, specifically:

3. ☐ Claims Nos.:
    because they are dependent claims and are not drafted in accordance with the second and third sentences of Rule 6.4(a).

Form PCT/ISA/210 (continuation of first sheet) (July 2022)

**INTERNATIONAL SEARCH REPORT**
Information on patent family members

| International application No. |
| --- |
| **PCT/CN2023/129973** |

| Patent document cited in search report | | | Publication date (day/month/year) | Patent family member(s) | | | Publication date (day/month/year) |
| --- | --- | --- | --- | --- | --- | --- | --- |
| WO | 2022207645 | A1 | 06 October 2022 | None | | | |
| CA | 3149320 | A1 | 04 February 2021 | BR | 112022001800 | A2 | 12 April 2022 |
| | | | | US | 2023145060 | A1 | 11 May 2023 |
| | | | | WO | 2021016724 | A1 | 04 February 2021 |
| | | | | MX | 2022001387 | A | 08 June 2022 |
| | | | | JP | 2022543070 | A | 07 October 2022 |
| | | | | KR | 20220107151 | A | 02 August 2022 |
| | | | | AU | 2020320459 | A1 | 03 March 2022 |
| | | | | EP | 4007779 | A1 | 08 June 2022 |
| | | | | EP | 4007779 | A4 | 06 September 2023 |
| US | 2013302371 | A1 | 14 November 2013 | US | 2017095550 | A1 | 06 April 2017 |
| | | | | US | 9884107 | B2 | 06 February 2018 |
| | | | | WO | 2012037078 | A2 | 22 March 2012 |
| | | | | WO | 2012037078 | A3 | 21 June 2012 |
| | | | | US | 9511135 | B2 | 06 December 2016 |
| CN | 104031144 | A | 10 September 2014 | None | | | |
| CN | 113557431 | A | 26 October 2021 | AU | 2021223701 | A1 | 06 January 2022 |
| | | | | AU | 2021223701 | B2 | 19 May 2022 |
| | | | | RU | 2021134819 | A | 17 February 2022 |
| | | | | RU | 2021134819 | A3 | 29 April 2022 |
| | | | | CA | 3109607 | A1 | 20 May 2021 |
| | | | | CA | 3109607 | C | 04 April 2023 |
| | | | | ES | 2822295 | T1 | 30 April 2021 |
| | | | | EP | 3809137 | A1 | 21 April 2021 |
| | | | | KR | 20220006125 | A | 14 January 2022 |
| | | | | KR | 102570713 | B1 | 25 August 2023 |
| | | | | JP | 2021167805 | A | 21 October 2021 |
| | | | | JP | 7022969 | B2 | 21 February 2022 |
| | | | | WO | 2021165448 | A1 | 26 August 2021 |
| | | | | SG | 11202112544 | XA | 30 December 2021 |
| | | | | EP | 3978927 | A2 | 06 April 2022 |
| | | | | EP | 3978927 | A3 | 22 June 2022 |
| | | | | US | 2021190797 | A1 | 24 June 2021 |
| | | | | DE | 202021100842 | U1 | 17 August 2021 |
| | | | | DE | 21158065 | T1 | 10 June 2021 |
| US | 2012015000 | A1 | 19 January 2012 | EP | 2313108 | A2 | 27 April 2011 |
| | | | | EP | 2313108 | A4 | 12 June 2013 |
| | | | | WO | 2010002818 | A2 | 07 January 2010 |
| | | | | WO | 2010002818 | A3 | 27 May 2010 |
| US | 2017056493 | A1 | 02 March 2017 | CA | 2948181 | A1 | 19 November 2015 |
| | | | | US | 9987353 | B2 | 05 June 2018 |
| | | | | EP | 3143144 | A1 | 22 March 2017 |
| | | | | EP | 3143144 | A4 | 20 December 2017 |
| | | | | BR | 112016026721 | A2 | 31 October 2017 |
| | | | | AU | 2015259470 | A1 | 10 November 2016 |
| | | | | JP | 2017515508 | A | 15 June 2017 |
| | | | | WO | 2015175380 | A1 | 19 November 2015 |
| WO | 2014205491 | A1 | 31 December 2014 | None | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **BAKER et al.** *Biophys. J.*, 1991, vol. 60, 1445-1456
- **HAGENSEE et al.** *J. Virol.*, 1994, vol. 68, 4503-4505
- **BRUMMELL et al.** *Biochem.*, 1993, vol. 32, 1180-1187
- **KOBAYASHI et al.** *Protein Eng.*, 1999, vol. 12 (10), 879-884
- **BURKS et al.** *Proc. Natl Acad. Set USA*, 1997, vol. 94, 412-417
- **KABAT**. Sequences of Proteins of Immunological Interest. National Institutes of Health, 1987
- **CHOTHIA** ; **LESK**. *J. Mol. Biol.*, 1987, vol. 196, 901-917
- **CHOTHIA et al.** *Nature*, 1989, vol. 342, 878-883
- **MARTIN ACR** ; **CHEETHAM JC** ; **REES AR**. Modelling antibody hypervariable loops: A combined algorithm.. *Proc Natl Acad Sci USA*, 1989, vol. 86, 9268-9272
- **KABAT et al.** Sequences of Proteins of Immunological Interest. Public Health Service, National Institutes of Health, 1991
- **LEFRANC et al.** *Dev. Comparat. Immunol.*, 2003, vol. 27, 55-77
- **HOLT, L. et al.** *Trends in Biotechnology*, 2003, vol. 21 (11), 484-490
- Remington's Pharmaceutical Sciences. Pennsylvania: Mack Publishing Company, 1995
- **J. SAMBROOK et al.** Molecular Cloning: A Laboratory Manual. Cold Spring Harbor Laboratory Press, 1989
- **F. M. AUSUBEL et al.** Compiled Molecular Biology Laboratory Manual. John Wiley & Sons, Inc., 1995

- **JU B** ; **ZHENG Q** ; **GUO H** ; **FAN Q** ; **LI T** ; **SONG S** ; **SUN H** ; **SHEN S** ; **ZHOU X** ; **XUE W**. Immune escape by SARS-CoV-2 Omicron variant and structural basis of its effective neutralization by a broad neutralizing human antibody VacW-209. *Cell Res*, 08 March 2022, vol. 32 (5), 491-494
- **LIU, J.** ; **YE, X** ; **JIA, J. et al.** Serological Evaluation of Immunity to the Varicella-Zoster Virus Based on a Novel Competitive Enzyme-Linked Immunosorbent Assay. *Sci Rep*, 2016, vol. 6, 20577, https://doi.org/10.1038/srep20577
- **WILKINSON, R.A.** ; **C. PISCITELLI** ; **M. TEINTZE et al.** Structure of the Fab fragment of F105, a broadly reactive anti-human immunodeficiency virus (HIV) antibody that recognizes the CD4 binding site of HIV type 1 gp120. *J Virol*, 2005, vol. 79 (20), 13060-13069
- **GOHAIN, N** ; **W.D. TOLBERT** ; **C. ORLANDI et al.** Molecular basis for epitope recognition by non-neutralizing anti-gp41 antibody F240. *Sci Rep*, 2016, vol. 6, 36685
- **CARLO D. RIZZUTO** ; **RICHARD WYATT** ; **NIVIA HERNA NDEZ-RAMOS et al.** A Conserved HIV gp120 Glycoprotein Structure Involved in Chemokine Receptor Binding. *Science*, 1998, vol. 280 (19), 1949-1953
- **XIONG HL** ; **WU YT** ; **CAO JL** ; **YANG R** ; **LIU YX** ; **MA J** ; **QIAO XY** ; **YAO XY** ; **ZHANG BH** ; **ZHANG YL**. Robust neutralization assay based on SARS-CoV-2 S-protein-bearing vesicular stomatitis virus (VSV) pseudovirus and ACE2-overexpressing BHK21 cells. *Emerg Microbes Infect*, December 2020, vol. 9 (1), 2105-2113
- **SERRUYS, B** ; **HOUTTE, F. V** ; **VERBRUGGHE, P** ; **LEROUX-ROELS, G.** ; **VANLANDSCHOOT, P**. Llama-derived single-domain intrabodies inhibit secretion of hepatitis B virions in mice. *Hepatology*, 2009, vol. 49, 39-49